# EUROPEAN PATENT APPLICATION

(11) **EP 3 974 410 A1**
(43) Date of publication of application: **30.03.2022**
(21) Application number: 20831790.9
(22) Date of filing: 19.06.2020
(51) Int. Cl.: C07C 43/225, C07C 43/23, C07C 211/30, C07C 229/08, C07C 269/06, C07C 271/22, C07K 1/06, C07B 61/00

(54) **METHOD FOR PRODUCING PEPTIDE COMPOUND, REAGENT FOR FORMING PROTECTING GROUP, AND CONDENSED POLYCYCLIC AROMATIC HYDROCARBON COMPOUND**

(30) Priority: 28.06.2019 JP 2019122431
(71) Applicant: FUJIFILM Corporation, Tokyo 106-8620 (JP)
(72) Inventor: YAMAMOTO, Yosuke, Ashigarakami-gun, Kanagawa 258-8577 (JP); KANEKO, Kazuhei, Ashigarakami-gun, Kanagawa 258-8577 (JP); OMURA, Hirofumi, Ashigarakami-gun, Kanagawa 258-8577 (JP); TAKAHASHI, Motomasa, Ashigarakami-gun, Kanagawa 258-8577 (JP); TAKAHASHI, Makoto, Ashigarakami-gun, Kanagawa 258-8577 (JP)
(74) Representative: Hoffmann Eitle
(86) International application number: PCT/JP2020/024231
(87) International publication number: WO 2020/262258

(57) **Abstract**

Provided are a method for producing a peptide compound including a step of using a condensed polycyclic aromatic hydrocarbon compound represented by Formula (1); a protective group-forming reagent including the compound; and the compound. In Formula (1), a ring A represents a condensed polycyclic aromatic hydrocarbon ring, Y^{A}'s each independently represent -OH, -NHR, -SH, or -X⁰, where X⁰ represents Cl, Br, or I, R^{A} and R^{C} each independently represent an aliphatic hydrocarbon group or an organic group having an aliphatic hydrocarbon group, R^{B}s' each independently represent a monovalent aliphatic hydrocarbon group, a (1+c)-valent aromatic group, or a (1+c)-valent heteroaromatic group, where, in a case where both a and c is 0, R^{B} is a monovalent aliphatic hydrocarbon group, and the number of carbon atoms in at least one aliphatic hydrocarbon group is 12 or more.

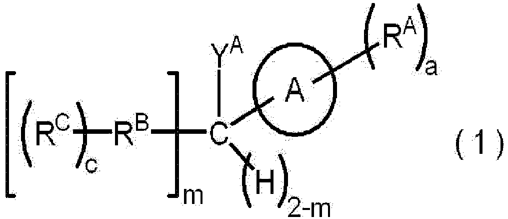

## Description

The present disclosure relates to a method for producing a peptide compound, a protective group-forming reagent, and a condensed polycyclic aromatic hydrocarbon compound.

Examples of a method for producing peptide include a solid phase method and a liquid phase method.

The solid phase method is advantageous in that an isolation and purification after a reaction can be performed only by washing the resin. However, the solid phase method is associated with problems in that the reaction is essentially a heterogeneous phase reaction, a reaction reagent need to be used in excess to compensate for the low reactivity, and tracing of the reaction and analysis of a reaction product supported by a carrier are difficult.

On the other hand, the liquid phase method is advantageous in that good reactivity is exhibited, and intermediate peptide can be purified by extraction and washing, isolation, and the like after a condensation reaction. However, the liquid phase method still has problems in each step of coupling reaction and deprotection.

As a protective group-forming reagent in the related art, a compound having a fluorene structure, which is disclosed in WO2010/104169A, and a compound having a diphenylmethane structure, which is disclosed in WO2010/113939A, have been known.

An object to be achieved by an embodiment of the present invention is to provide a method for producing a peptide compound having an excellent yield.

An object to be achieved by another embodiment of the present invention is to provide a protective group-forming reagent having an excellent yield.

An object to be achieved by still another embodiment of the present invention is to provide a novel condensed polycyclic aromatic hydrocarbon compound.

The methods for achieving the above-described objects include the following aspects.
<1> A method for producing a peptide compound, comprising:
   a step of using a condensed polycyclic aromatic hydrocarbon compound represented by Formula (1).

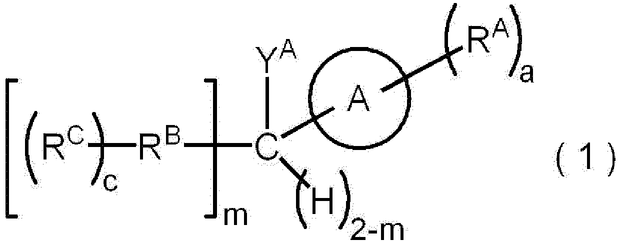

In Formula (1),
a ring A represents a condensed polycyclic aromatic hydrocarbon ring,
Y^{A} represents -OH, -NHR, SH, or -X⁰, where R represents a hydrogen atom, an alkyl group, an aromatic group-substituted alkyl group, a heteroaromatic group-substituted alkyl group, or a 9-fluorenylmethoxycarbonyl group, and X⁰ represents Cl, Br, or I,
R^{A}'s each independently represent an aliphatic hydrocarbon group or an organic group having an aliphatic hydrocarbon group,
R^{B}'s each independently represent a monovalent aliphatic hydrocarbon group, a (1+c)-valent aromatic group, or a (1+c)-valent heteroaromatic group,
R^{C}'s each independently represent an aliphatic hydrocarbon group or an organic group having an aliphatic hydrocarbon group,
m represents 1 or 2, a represents an integer of 0 to 5, and c represents an integer of 0 to 5,
in a case where both a and c are 0, R^{B} is a monovalent aliphatic hydrocarbon group, and
the number of carbon atoms in at least one of the aliphatic hydrocarbon group (of R^{A}, R^{B}, and R^{C}) is 12 or more.

<2> The method for producing a peptide compound according to <1>,
   in which the step of using the condensed polycyclic aromatic hydrocarbon compound represented by Formula (1) is a C-terminal protecting step of protecting a carboxy group or an amide group of an amino acid compound or a peptide compound with the condensed polycyclic aromatic hydrocarbon compound represented by Formula (1).
<3> The method for producing a peptide compound according to <2>,
   in which the amino acid compound or the peptide compound in the C-terminal protecting step is an N-terminal protected amino acid compound or an N-terminal protected peptide compound.
<4> The method for producing a peptide compound according to <3>, further comprising:
   an N-terminal deprotecting step of deprotecting an N-terminal end of an N-terminal and C-terminal protected amino acid compound or an N-terminal and C-terminal protected peptide compound, which is obtained in the C-terminal protecting step; and
   a peptide chain extending step of condensing the N-terminal end of a C-terminal protected amino acid compound or a C-terminal protected peptide compound, which is obtained in the N-terminal deprotecting step, with an N-terminal protected amino acid compound or an N-terminal protected peptide compound.
<5> The method for producing a peptide compound according to <4>, further comprising:
   a precipitating step of precipitating the N-terminal and C-terminal protected peptide compound obtained in the peptide chain extending step.
<6> The method for producing a peptide compound according to <5>, further comprising, one or more times in the following order after the precipitating step:
   a step of deprotecting the N-terminal end of the obtained N-terminal and C-terminal protected peptide compound;
   a step of condensing the N-terminal end of the obtained C-terminal protected peptide compound with an N-terminal protected amino acid compound or an N-terminal protected peptide compound; and
   a step of precipitating the obtained N-terminal and C-terminal protected peptide compound.
<7> The method for producing a peptide compound according to any one of <1> to <6>, further comprising:
   a C-terminal deprotecting step of deprotecting a C-terminal protective group.
<8> The method for producing a peptide compound according to any one of <1> to <7>,
   in which the ring A is a naphthalene ring.
<9> The method for producing a peptide compound according to any one of <1> to <8>,
   in which a total number of carbon atoms in all aliphatic hydrocarbon groups included in all R^{A}, R^{B}, and R^{C} is 36 to 80.
<10> The method for producing a peptide compound according to any one of <1> to <9>,
   in which the condensed polycyclic aromatic hydrocarbon compound represented by Formula (1) is a compound represented by any of Formula (10) or Formula (20).

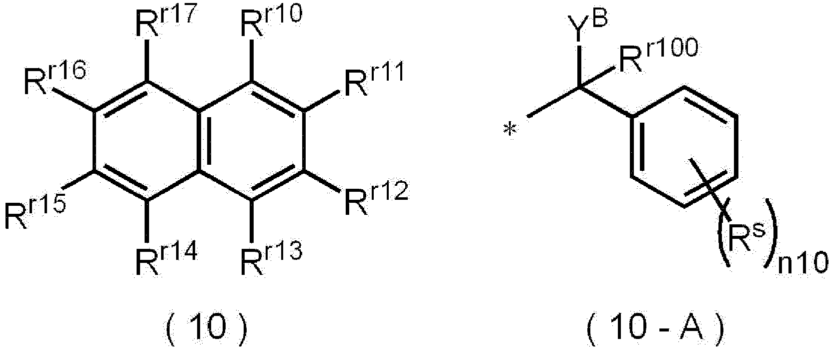

In Formula (10) and Formula (10-A),
Y^{B} represents -OH, -NHR, -SH, or -X⁰, where R represents a hydrogen atom, an alkyl group, an aromatic group-substituted alkyl group, a heteroaromatic group-substituted alkyl group, or a 9-fluorenylmethoxycarbonyl group (Fmoc group), and X⁰ represents Cl, Br, or I,
R^{r110} represents a hydrogen atom, an aryl group, or a heteroaryl group,
R^{S}'s each independently represent a substituent or R^{A},
n10 represents an integer of 0 to 5,
adjacent R^{S}'s may be linked to each other through a substituent to form a ring,
R^{r10} and R^{r11} each independently represent a hydrogen atom, a substituent, a group represented by Formula (10-A), or R^{A},
any one of R^{r10} or R^{r11} is a group represented by Formula (10-A),
^{∗} represents a linking position with R^{r10} or R^{r11},
R^{r12} to R^{r17} each independently represent a hydrogen atom, a substituent, or R^{A},
at least one of R^{s}, R^{r1O} , ... , or R^{r17} is R^{A},
R^{A}'s each independently represent an aliphatic hydrocarbon group or an organic group having an aliphatic hydrocarbon group, and
the number of carbon atoms in at least one of the aliphatic hydrocarbon group (included in at least one R^{A}) is 12 or more.

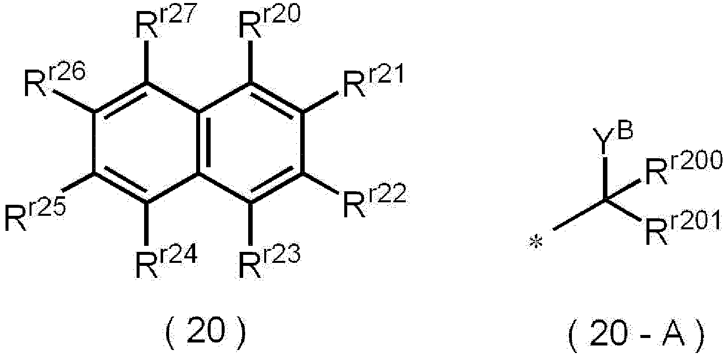

In Formula (20) and Formula (20-A),
Y^{B} represents -OH, -NHR, -SH, or -X⁰, where R represents a hydrogen atom, an alkyl group, an aromatic group-substituted alkyl group, a heteroaromatic group-substituted alkyl group, or a 9-fluorenylmethoxycarbonyl group (Fmoc group), and X⁰ represents Cl, Br, or I,
R^{r200} represents a hydrogen atom, an alkyl group, an aryl group, or a heteroaryl group,
R^{r201} represents an alkyl group,
R^{r20} and R^{r21} each independently represent a hydrogen atom, a substituent, a group represented by Formula (20-A), or R^{A},
any one of R^{r20} or R^{r21} is a group represented by Formula (20-A),
^{∗} represents a linking position with R^{r20} or R^{r21},
R^{r22} to R^{r27} each independently represent a hydrogen atom, a substituent, or R^{A},
the number of carbon atoms in R^{r201} is 12 or more, or at least one of R^{r20}, ... , or R^{r27} is R^{A},
R^{A}'s each independently represent an aliphatic hydrocarbon group or an organic group having an aliphatic hydrocarbon group, and
the number of carbon atoms in at least one of the aliphatic hydrocarbon group (included in at least one R^{A}) is 12 or more.

<11> The method for producing a peptide compound according to <10>,
in which R^{A}'s in Formula (10) or Formula (20) are each independently a group represented by Formula (f1) or Formula (a1).

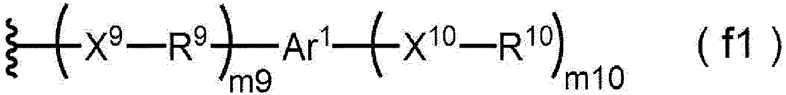

In Formula (f1), a wavy line portion represents a bonding position to other structures, m9 represents an integer of 1 to 3, X⁹'s each independently represent a single bond, -O-, -S-, -COO-, -OCO-, -OCONH-, -NHCONH-, -NHCO-, or -CONH-, R⁹'s each independently represent a divalent aliphatic hydrocarbon group, Ar¹ represents an (m10+1)-valent aromatic group or an (m10+1)-valent heteroaromatic group, m10 represents an integer of 1 to 3, X¹⁰'s each independently represent a single bond, -O-, -S-, -COO-, -OCO-, -OCONH-, -NHCONH-, -NHCO-, or -CONH-, R¹⁰'s each independently represent a monovalent aliphatic hydrocarbon group, and at least one of R¹⁰'s is a monovalent aliphatic hydrocarbon group having 5 or more carbon atoms.

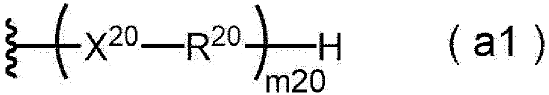

In Formula (a1), a wavy line portion represents a bonding position to other structures, m20 represents an integer of 1 to 10, X²⁰'s each independently represent a single bond, -O-, -S-, -COO-, -OCO-, -OCONH-, -NHCONH-, -NHCO-, or -CONH-, R²⁰'s each independently represent a divalent aliphatic hydrocarbon group, and at least one of R²⁰'s is a divalent aliphatic hydrocarbon group having 5 or more carbon atoms.

<12> The method for producing a peptide compound according to <11>,
in which the group represented by Formula (f1) is a group represented by Formula (f2).

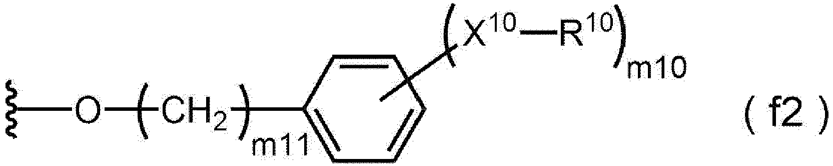

In Formula (f2), a wavy line portion represents a bonding position to other structures, m10 represents an integer of 1 to 3, m11 represents an integer of 1 to 3, X¹⁰'s each independently represent a single bond, -O-, -S-, -COO-, -OCO-, -OCONH-, -NHCONH-, -NHCO-, or -CONH-, and R¹⁰'s each independently represent a monovalent aliphatic hydrocarbon group having 5 or more carbon atoms.
<13> The method for producing a peptide compound according to <11>,
   in which X²⁰ in Formula (a1) is -O-.
<14> A protective group-forming reagent comprising:
   a condensed polycyclic aromatic hydrocarbon compound represented by Formula (1).

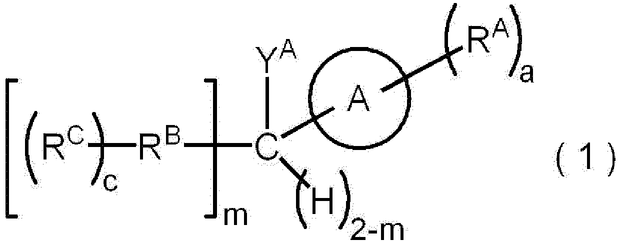

In Formula (1),
a ring A represents a condensed polycyclic aromatic hydrocarbon ring,
Y^{A} represents -OH, -NHR, SH, or -X⁰, where R represents a hydrogen atom, an alkyl group, an aromatic group-substituted alkyl group, a heteroaromatic group-substituted alkyl group, or a 9-fluorenylmethoxycarbonyl group (Fmoc group), and X⁰ represents Cl, Br, or I,
R^{A}'s each independently represent an aliphatic hydrocarbon group or an organic group having an aliphatic hydrocarbon group,
R^{B}'s each independently represent a monovalent aliphatic hydrocarbon group, a (1+c)-valent aromatic group, or a (1+c)-valent heteroaromatic group,
R^{C}'s each independently represent an aliphatic hydrocarbon group or an organic group having an aliphatic hydrocarbon group,
m represents 1 or 2, a represents an integer of 0 to 5, and c represents an integer of 0 to 5,
in a case where both a and c are 0, R^{B} is a monovalent aliphatic hydrocarbon group, and
the number of carbon atoms in at least one of the aliphatic hydrocarbon group (of R^{A}, R^{B}, and R^{C}) is 12 or more.

<15> The protective group-forming reagent according to <14>,
   in which the protective group-forming reagent is a protective group-forming reagent of a carboxy group or an amide group.
<16> The protective group-forming reagent according to <14> or <15>,
   in which the protective group-forming reagent is a C-terminal protective group-forming reagent of an amino acid compound or a peptide compound.
<17> A condensed polycyclic aromatic hydrocarbon compound represented by Formula (1a).

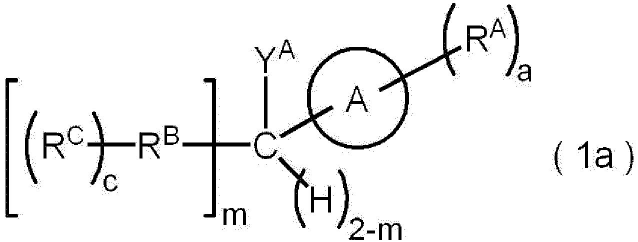

In Formula (1a),
a ring A represents a condensed polycyclic aromatic hydrocarbon ring,
Y^{A} represents -OH, -NHR, -SH, or -X⁰, where R represents a hydrogen atom, an alkyl group having 10 or less carbon atoms, an aromatic group-substituted alkyl group, a heteroaromatic group-substituted alkyl group, or a 9-fluorenylmethoxycarbonyl group (Fmoc group), and X⁰ represents Cl, Br, or I,
R^{A}'s each independently represent an aliphatic hydrocarbon group or an organic group having an aliphatic hydrocarbon group,
R^{B}'s each independently represent a monovalent aliphatic hydrocarbon group, a (1+c)-valent aromatic group, or a (1+c)-valent heteroaromatic group,
R^{C}'s each independently represent an aliphatic hydrocarbon group or an organic group having an aliphatic hydrocarbon group,
m represents 1 or 2, a represents an integer of 0 to 5, and c represents an integer of 0 to 5,
in a case where both a and c are 0, R^{B} is a monovalent aliphatic hydrocarbon group,
the number of carbon atoms in at least one of the aliphatic hydrocarbon group (of R^{A}, R^{B}, and R^{C}) is 18 or more, and
in a case where R^{B} is a monovalent aliphatic hydrocarbon group, R^{B} includes a linear saturated aliphatic hydrocarbon group having 18 or more carbon atoms.

<18> The condensed polycyclic aromatic hydrocarbon compound according to <17>, in which the ring A is a naphthalene ring.
<19> The condensed polycyclic aromatic hydrocarbon compound according to <17> or <18>,
   in which the condensed polycyclic aromatic hydrocarbon compound represented by Formula (1a) is a compound represented by any of Formula (10a) or Formula (20a).

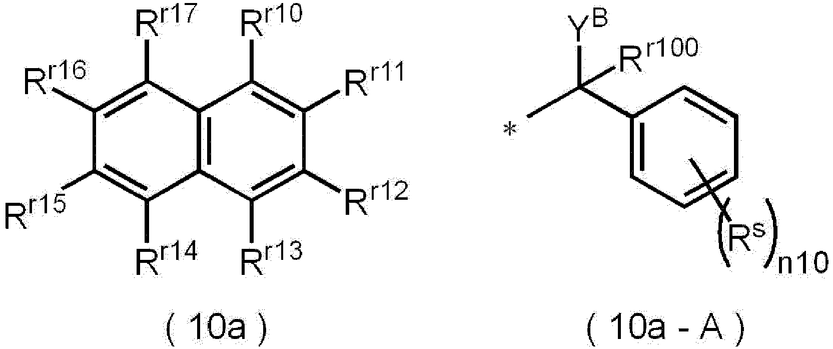

In Formula (10a) and Formula (10a-A),
Y^{B} represents -OH, -NHR, -SH, or -X⁰, where R represents a hydrogen atom, an alkyl group having 10 or less carbon atoms, an aromatic group-substituted alkyl group, a heteroaromatic group-substituted alkyl group, or a 9-fluorenylmethoxycarbonyl group (Fmoc group), and X⁰ represents Cl, Br, or I,
R^{r100} represents a hydrogen atom, an aryl group, or a heteroaryl group,
R^{S}'s each independently represent a substituent or R^{A},
n10 represents an integer of 0 to 5,
adjacent R^{S}'s may be linked to each other through a substituent to form a ring,
R^{r10} and R^{r11} each independently represent a hydrogen atom, a substituent, a group represented by Formula (10a-A), or R^{A},
any one of R^{r10} or R^{r11} is a group represented by Formula (10a-A),
^{∗} represents a linking position with R^{r10} or R^{r11},
R^{r12} to R^{r17} each independently represent a hydrogen atom, a substituent, or R^{A},
at least one of R^{s}, R^{r1O} , ... , or R^{r17} is R^{A},
R^{A}'s each independently represent an aliphatic hydrocarbon group or an organic group having an aliphatic hydrocarbon group, and
the number of carbon atoms in at least one of the aliphatic hydrocarbon group (included in at least one R^{A}) is 18 or more.

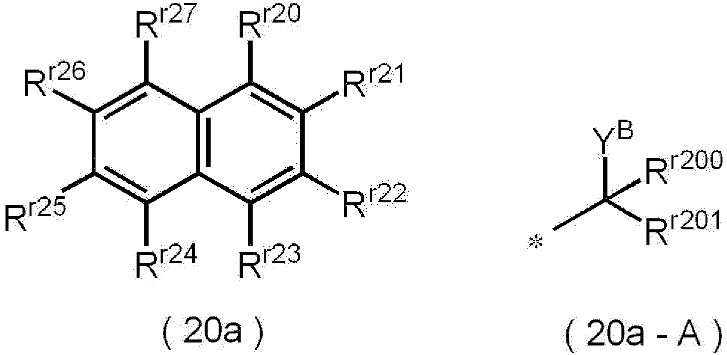

In Formula (20a) and Formula (20a-A),
Y^{B} represents -OH, -NHR, -SH, or -X⁰, where R represents a hydrogen atom, an alkyl group having 10 or less carbon atoms, an aromatic group-substituted alkyl group, a heteroaromatic group-substituted alkyl group, or a 9-fluorenylmethoxycarbonyl group (Fmoc group), and X⁰ represents Cl, Br, or I,
R^{r200} represents a hydrogen atom, an alkyl group, an aryl group, or a heteroaryl group, R^{r201} represents an aliphatic hydrocarbon group,
R^{r20} and R^{r21} each independently represent a hydrogen atom, a substituent, a group represented by Formula (20a-A), or R^{A},
any one of R^{r20} or R^{r21} is a group represented by Formula (20a-A),
^{∗} represents a linking position with R^{r20} or R^{r21},
R^{r22} to R^{r27} each independently represent a hydrogen atom, a substituent, or R^{A}, and
in a case where R^{B} is an aliphatic hydrocarbon group, R^{B} includes a linear saturated aliphatic hydrocarbon group having 18 or more carbon atoms, or at least one of R^{R20}, ... , or R^{r27} is R^{A}, where R^{A}'s each independently represent an aliphatic hydrocarbon group or an organic group having an aliphatic hydrocarbon group, and the number of carbon atoms in at least one of the aliphatic hydrocarbon group included in at least one R^{A} is 18 or more.

<20> The condensed polycyclic aromatic hydrocarbon compound according to <19>,
in which R^{A}'s in Formula (10a) or Formula (20a) are each independently a group represented by Formula (f1) or Formula (a1). 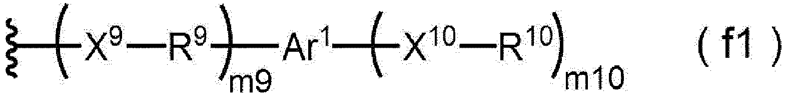 In Formula (f1), a wavy line portion represents a bonding position to other structures, m9 represents an integer of 1 to 3, X⁹'s each independently represent a single bond, -O-, -S-, -COO-, -OCO-, -OCONH-, -NHCONH-, -NHCO-, or -CONH-, R⁹'s each independently represent a divalent aliphatic hydrocarbon group, Ar¹ represents an (m10+1)-valent aromatic group or an (m10+1)-valent heteroaromatic group, m10 represents an integer of 1 to 3, X¹⁰'s each independently represent a single bond, -O-, -S-, -COO-, -OCO-, -OCONH-, -NHCONH-, -NHCO-, or -CONH-, R¹⁰'s each independently represent a monovalent aliphatic hydrocarbon group, and at least one of R¹⁰'s is a monovalent aliphatic hydrocarbon group having 5 or more carbon atoms.

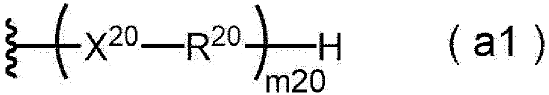

In Formula (a1), a wavy line portion represents a bonding position to other structures, m20 represents an integer of 1 to 10, X²⁰'s each independently represent a single bond, -O-, -S-, -COO-, -OCO-, -OCONH-, -NHCONH-, -NHCO-, or -CONH-, R²⁰'s each independently represent a divalent aliphatic hydrocarbon group, and at least one of R²⁰'s is a divalent aliphatic hydrocarbon group having 5 or more carbon atoms.

<21> The condensed polycyclic aromatic hydrocarbon compound according to <20>,
in which the group represented by Formula (f1) is a group represented by Formula (f2).

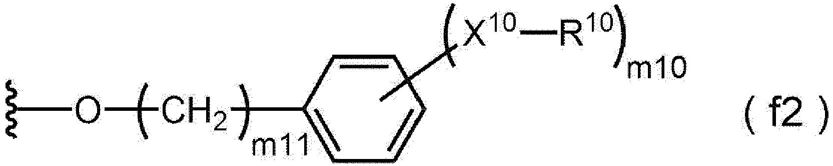

In Formula (f2), a wavy line portion represents a bonding position to other structures, m10 represents an integer of 1 to 3, m11 represents an integer of 1 to 3, X¹⁰'s each independently represent a single bond, -O-, -S-, -COO-, -OCO-, -OCONH-, -NHCONH-, -NHCO-, or -CONH-, and R¹⁰'s each independently represent a monovalent aliphatic hydrocarbon group having 5 or more carbon atoms.

<22> The condensed polycyclic aromatic hydrocarbon compound according to <20>, in which X²⁰ in Formula (a1) is -O-.

According to an embodiment of the present invention, it is possible to provide a method for producing a peptide compound having an excellent yield.

In addition, according to another embodiment of the present invention, it is possible to provide a protective group-forming reagent having an excellent yield.

In addition, according to still another embodiment of the present invention, it is possible to provide a novel condensed polycyclic aromatic hydrocarbon compound.

Hereinafter, the contents of the present disclosure will be described in detail. The description of configuration requirements below is made based on representative embodiments of the present disclosure in some cases, but the present disclosure is not limited to such embodiments.

In the present specification, unless otherwise specified, each term has the following meaning.

A numerical range represented using "to" means a range including numerical values described before and after the preposition "to" as a lower limit value and an upper limit value.

In numerical ranges described in stages in the present specification, an upper limit value or a lower limit value described in one numerical range may be replaced with an upper limit value or a lower limit value of a numerical range described in another stage. In addition, in the numerical ranges described in the present specification, the upper limit value or the lower limit value of the numerical ranges may be replaced with the values shown in examples.

The term "step" includes not only the independent step but also a step in which intended purposes are achieved even in a case where the step cannot be precisely distinguished from other steps.

In a case where substitution or unsubstitution is not noted in regard to the notation of a "group" (atomic group) in the present specification, the "group" includes not only a group not having a substituent but also a group having a substituent. For example, the concept of an "alkyl group" includes not only an alkyl group not having a substituent (unsubstituted alkyl group) but also an alkyl group having a substituent (substituted alkyl group).

A chemical structural formula may be described by a simplified structural formula in which hydrogen atoms are omitted.
"% by mass" has the same definition as that for "% by weight", and "part by mass" has the same definition as that for "part by weight".

A combination of two or more preferred aspects is a more preferred aspect.

The alkyl group may be chain-like or branched, and may be substituted with a halogen atom or the like. Examples of an alkyl group having 1 to 6 carbon atoms (also referred to as "the number of carbon atoms") include methyl, ethyl, propyl, isopropyl, butyl, and tert-butyl.

Examples of an alkenyl group having 2 to 6 carbon atoms include 1-propenyl.

As an aryl group, an aryl group having 6 to 14 carbon atoms is preferable, and examples thereof include a phenyl group, a 1-naphthyl group, a 2-naphthyl group, a biphenylyl group, and a 2-anthryl group. Among these, an aryl group having 6 to 10 carbon atoms is more preferable, and a phenyl group is particularly preferable.

Examples of a silyl group include trimethylsilyl, triethylsilyl, dimethylphenylsilyl, tert-butyldimethylsilyl, and tert-butyldiethylsilyl.

Examples of a halogen atom include a fluorine atom, a chlorine atom, a bromine atom, and an iodine atom.

Examples of an alkoxy group having 1 to 6 carbon atoms include methoxy, ethoxy, and propoxy.

Examples of an aralkyl group having 7 to 10 carbon atoms include benzyl.

Examples of an acyl group having 1 to 6 carbon atoms include acetyl and propionyl.

Examples of an aralkyl-carbonyl group having 7 to 10 carbon atoms include benzylcarbonyl.

Examples of an alkoxycarbonyl group having 1 to 6 carbon atoms include methoxycarbonyl, ethoxycarbonyl, and a Boc group. The Boc group means a tert-butoxycarbonyl group.

Examples of an aralkyloxycarbonyl group having 7 to 14 carbon atoms include benzyloxycarbonyl and an Fmoc group. The Fmoc group means a 9-fluorenylmethoxycarbonyl group.

### (Method for producing peptide compound)

A method for producing a peptide compound according to an embodiment of the present disclosure includes a step of using a condensed polycyclic aromatic hydrocarbon compound represented by Formula (1).

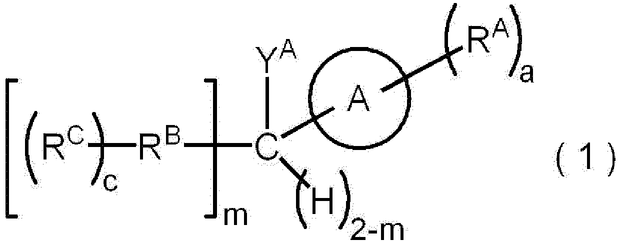

In Formula (1),
a ring A represents a condensed polycyclic aromatic hydrocarbon ring,
Y^{A} represents -OH, -NHR, SH, or -X⁰, where R represents a hydrogen atom, an alkyl group, an aromatic group-substituted alkyl group, a heteroaromatic group-substituted alkyl group, or an Fmoc group, and X⁰ represents Cl, Br, or I,
R^{A}'s each independently represent an aliphatic hydrocarbon group or an organic group having an aliphatic hydrocarbon group,
R^{B}'s each independently represent a monovalent aliphatic hydrocarbon group, a (1+c)-valent aromatic group, or a (1+c)-valent heteroaromatic group,
R^{C}'s each independently represent an aliphatic hydrocarbon group or an organic group having an aliphatic hydrocarbon group,
m represents 1 or 2, a represents an integer of 0 to 5, and c represents an integer of 0 to 5,
in a case where both a and c are 0, R^{B} is a monovalent aliphatic hydrocarbon group, and
the number of carbon atoms in at least one of the aliphatic hydrocarbon group (of R^{A}, R^{B}, and R^{C}) is 12 or more.

Since the compound represented by Formula (1) according to the present disclosure has at least one aliphatic hydrocarbon group having 12 or more carbon atoms, a compound to be protected has excellent solubility in a hydrophobic solvent. Furthermore, with regard to the hydrophilic solvent, since the aliphatic hydrocarbon groups in each R^{A}, R^{B}, and R^{C} aggregate with each other and the compound represented by Formula (1) has a condensed polycyclic aromatic hydrocarbon ring as the ring A, in the obtained peptide compound, due to the π-π interaction (π-π stacking) between the condensed polycyclic aromatic hydrocarbon ring, crystallization property is excellent, and purification and separability are also excellent. In other words, in a case where the protected compound is subjected to a reaction, since the protected compound has excellent solubility in a hydrophobic solvent as a reaction solvent, it is presumed that the reaction proceeds rapidly, and since a target product is efficiently crystallized and purified by adding a polar solvent which is a poor solvent during purification, it is presumed that yield of the obtained compound (peptide compound and the like) is excellent.

In addition, by having Y^{A} bonded to the condensed polycyclic aromatic hydrocarbon ring through a mono- or di-substituted methylene group, a deprotection rate is excellent. This is because the condensed polycyclic aromatic hydrocarbon ring contributes to electron donation or cation stabilization.

In addition, in the compound represented by Formula (1) according to the present disclosure, with the above-described structure, peptide can be synthesized with high purity while suppressing side reactions, and it is stable and easy to deprotect (remove) during the peptide synthesis reaction.

Furthermore, even poorly synthesized peptides such as unnatural peptide including unnatural amino acid, in which a side reaction is likely to occur, the peptide can be synthesized with high purity due to suppression of the side reaction.

In the production method according to the embodiment of the present disclosure, a C-terminal protective group can be deprotected even under weak acid conditions, and a side reaction of the obtained peptide can be suppressed. Therefore, the production method according to the embodiment of the present disclosure is suitable for the synthesis of acid-sensitive peptides, for example, peptides having an N-alkylamide structure.

Hereinafter, the method for producing a peptide compound according to the embodiment of the present disclosure will be described in detail.

In the method for producing a peptide compound according to the embodiment of the present disclosure, the condensed polycyclic aromatic hydrocarbon compound represented by Formula (1) can be used not only for formation of a protective group, but also for denaturation of a peptide compound, adjustment of solubility in water or an organic solvent, improvement of crystallinity, multimerization, and the like.

Among these, the condensed polycyclic aromatic hydrocarbon compound represented by Formula (1) is preferably used for formation of a protective group, and more preferably used for forming a C-terminal protective group in an amino acid compound or a peptide compound.

### <Condensed polycyclic aromatic hydrocarbon compound represented by Formula (1)>

The condensed polycyclic aromatic hydrocarbon compound represented by Formula (1) according to the present disclosure (hereinafter, also simply referred to as a "compound represented by Formula (1)") is shown below.

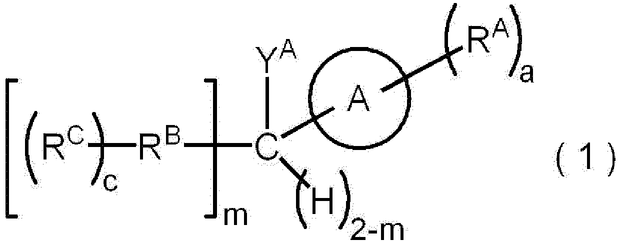

In Formula (1),
a ring A represents a condensed polycyclic aromatic hydrocarbon ring,
Y^{A} represents -OH, -NHR, SH, or -X⁰, where R represents a hydrogen atom, an alkyl group, an aromatic group-substituted alkyl group, a heteroaromatic group-substituted alkyl group, or an Fmoc group, and X⁰ represents Cl, Br, or I,
R^{A}'s each independently represent an aliphatic hydrocarbon group or an organic group having an aliphatic hydrocarbon group,
R^{B}'s each independently represent a monovalent aliphatic hydrocarbon group, a (1+c)-valent aromatic group, or a (1+c)-valent heteroaromatic group,
R^{C}'s each independently represent an aliphatic hydrocarbon group or an organic group having an aliphatic hydrocarbon group,
m represents 1 or 2, a represents an integer of 0 to 5, and c represents an integer of 0 to 5,
in a case where both a and c are 0, R^{B} is a monovalent aliphatic hydrocarbon group, and
the number of carbon atoms in at least one of the aliphatic hydrocarbon group (of R^{A}, R^{B}, and R^{C}) is 12 or more.

It is sufficient that at least one aliphatic hydrocarbon group having 12 or more carbon atoms is used in the compound. For example, in a case where a plurality of R^{A}'s are present in the compound, at least one of these may have an aliphatic hydrocarbon group having 12 or more carbon atoms.

The ring A in Formula (1) represents a condensed polycyclic aromatic hydrocarbon ring in which two or more aromatic hydrocarbon rings are condensed, and the ring A may further have a methylene group in which Y^{A} and R^{B} are linked, and a substituent in addition to R^{A}.

From the viewpoint of deprotection rate, crystallization property, and yield, the ring A is preferably a condensed polycyclic aromatic hydrocarbon ring having 2 to 4 rings, more preferably a condensed polycyclic aromatic hydrocarbon ring having 2 or 3 rings, and particularly preferably a condensed polycyclic aromatic hydrocarbon ring having 2 rings.

Among these, from the viewpoint of deprotection rate, crystallization property, and yield, the ring A is preferably a naphthalene ring, an anthracene ring, a phenanthrene ring, a tetracene ring, a triphenylene ring, a pyrene ring, or a chrysene ring, more preferably a naphthalene ring, an anthracene ring, or a phenanthrene ring, and particularly preferably a naphthalene ring.

In addition, from the viewpoint of yield, the ring A is preferably a having at least a naphthalene ring structure.

Furthermore, the ring A may have a substituent, and as described later, may form a ring structure in which two or more substituents are bonded to each other, and the ring A may have a structure in which an aliphatic hydrocarbon ring, an aliphatic hetero ring, a heteroaromatic ring, or the like is further fused.

From the viewpoint of deprotection rate, solubility in a solvent, and yield, Y^{A} in Formula (1) is preferably -OH, -NHR, or -SH, and more preferably -OH or -NHR

Examples of the alkyl group in R include an alkyl group having 1 to 30 carbon atoms (also referred to as "the number of carbon atoms"), and an alkyl group having 1 to 10 carbon atoms is preferable and an alkyl group having 1 to 6 carbon atoms is more preferable. Among these, more preferred examples thereof include a methyl group and an ethyl group.

Examples of the aromatic group-substituted alkyl group in R include an aromatic group-substituted alkyl group having 7 to 30 carbon atoms, and an aromatic group-substituted alkyl group having 7 to 20 carbon atoms is preferable and an aralkyl group having 7 to 16 carbon atoms (for example, a group in which an alkylene group having 1 to 6 carbon atoms is bonded to an aromatic group (aryl group) having 6 to 10 carbon atoms) is more preferable. Suitable specific examples thereof include a benzyl group, a 1-phenylethyl group, a 2-phenylethyl group, a 1-phenylpropyl group, a naphthylmethyl group, a 1-naphthylethyl group, and a 1-naphthylpropyl group, and a benzyl group is more preferable.

Examples of the heteroaromatic group-substituted alkyl group in R include a heteroaromatic group-substituted alkyl group having 5 to 30 carbon atoms, and a heteroaromatic group-substituted alkyl group having 5 to 20 carbon atoms is preferable and a heteroaromatic group-substituted alkyl group having 5 to 16 carbon atoms (for example, a group in which an alkylene group having 1 to 6 carbon atoms is bonded to a heteroaromatic group (heteroaryl group) having 4 to 10 carbon atoms) is more preferable. Suitable specific examples thereof include an indolylmethyl group, a furfuryl group, a benzofuranylmethyl group, a thiophenylmethyl group, and a benzothiophenylmethyl group.

Among these, R is preferably a hydrogen atom, an alkyl group having 1 to 6 carbon atoms, an aromatic group-substituted alkyl group having 7 to 16 carbon atoms, or an Fmoc group, more preferably a hydrogen atom, a methyl group, an ethyl group, a benzyl group, or an Fmoc group, and still more preferably a hydrogen atom or an Fmoc group. In a case where R is an Fmoc group, since Y^{A} becomes -NH₂ by deprotecting the Fmoc group with a base such as 1,8-diazabicyclo[5.4.0]-7-undecene (DBU) described later, it can be considered that the case where R is an Fmoc group and the case where R is a hydrogen atom are equivalent.

In addition, as the substituent on the ring A, the substituent on R^{B}, or R^{A}, the compound represented by Formula (1) may have a group which has the ring A having the methylene group in which Y^{A} and R^{B} are linked, or a group which has the ring A having the methylene group in which Y^{A} and R^{B} are linked, and Y^{A}. That is, the compound represented by Formula (1) may be a multimer such as a dimer. From the viewpoint of ease of synthesis, the multimer is preferably a dimer to a hexamer, more preferably a dimer to a tetramer, and particularly preferably a dimer.

From the viewpoint of deprotection rate, solubility in a solvent, and yield, a, which is the number of substitutions of R^{A} on the ring A in Formula (1), is preferably an integer of 1 to 4, more preferably an integer of 1 to 3, and particularly preferably 1 or 2.

In addition, from the viewpoint of deprotection rate, solubility in a solvent, and yield, c, which is the number of substitutions of R^{C} on R^{B} in Formula (1), is preferably an integer of 0 to 4, more preferably an integer of 0 to 2, still more preferably 0 or 1, and particularly preferably 0.

Furthermore, the value of a+c in Formula (1) is 0 or more, and from the viewpoint of deprotection rate, solubility in a solvent, and yield, preferably an integer of 1 to 6, more preferably an integer of 1 to 4, still more preferably 1 to 3, and particularly preferably 1.

R^{A}'s each independently represent an aliphatic hydrocarbon group or an organic group having an aliphatic hydrocarbon group.

In addition, R^{C}'s each independently represent an aliphatic hydrocarbon group or an organic group having the aliphatic hydrocarbon group.

In the present specification, the "organic group having an aliphatic hydrocarbon group" is a monovalent (one bonding site) organic group having an aliphatic hydrocarbon group in its molecular structure.

The "aliphatic hydrocarbon group" is a linear, branched, or cyclic saturated or unsaturated aliphatic hydrocarbon group having 5 or more carbon atoms, and from the viewpoint of solubility in a solvent, crystallization property, and yield, an aliphatic hydrocarbon group having 5 to 60 carbon atoms is preferable, an aliphatic hydrocarbon group having 5 to 30 carbon atoms is more preferable, and an aliphatic hydrocarbon group having 10 to 30 carbon atoms is particularly preferable.

In addition, from the viewpoint of solubility in a solvent, crystallization property, and yield, the number of carbon atoms in the "aliphatic hydrocarbon group" is preferably 12 or more, more preferably 14 or more, still more preferably 16 or more, and particularly preferably 18 or more.

The moiety of the aliphatic hydrocarbon group in the organic group having an aliphatic hydrocarbon group is not particularly limited, and may be present at the terminal (a monovalent group) or may be present at any other site (for example, a divalent group).

Examples of the "aliphatic hydrocarbon group" include an alkyl group, a cycloalkyl group, an alkenyl group, and an alkynyl group, and specific examples thereof include monovalent groups such as a pentyl group, a hexyl group, an octyl group, a decyl group, a hexadecyl group, an octadecyl group, an icosyl group, a docosyl group, a tetracosyl group, a lauryl group, a tridecyl group, a myristyl group, an oleyl group, and an isostearyl group; divalent groups derived from these (divalent groups obtained by removing one hydrogen atom from the monovalent groups); and groups removing a hydroxy group or the like from various steroid groups.

In addition, as the "alkyl group", for example, an alkyl group having 5 to 30 carbon atoms or the like is preferable, and examples thereof include a pentyl group, a hexyl group, an octyl group, a 2-ethylhexyl group, a decyl group, a hexadecyl group, an octadecyl group, an icosyl group, a docosyl group, a tetracosyl group, a lauryl group, a tridecyl group, a myristyl group, and an isostearyl group. Among these, an octadecyl group, an icosyl group, a docosyl group, or a tetracosyl group is preferable, and an icosyl group, a docosyl group, or a tetracosyl group is more preferable.

As the "cycloalkyl group", for example, a cycloalkyl group having 5 to 30 carbon atoms or the like is preferable, and examples thereof include a cyclopentyl group, a cyclohexyl group, an isobornyl group, and a tricyclodecanyl group. In addition, these may be linked repeatedly, or may be a fused-ring structure of two or more rings.

As the "alkenyl group", for example, an alkenyl group having 5 to 30 carbon atoms or the like is preferable, and examples thereof include a pentenyl group, a hexenyl group, and an oleyl group.

As the "alkynyl group", for example, an alkynyl group having 5 to 30 carbon atoms or the like is preferable, and examples thereof include a 4-pentynyl group and a 5-hexenyl group.

As the "steroid group", for example, a group having a cholesterol structure, a group having an estradiol structure, or the like is preferable.

The above-described organic group may be further substituted with a silyl group, a hydrocarbon group having a silyloxy structure, or an organic group having a perfluoroalkyl structure.

As the above-described silyl group, a trialkylsilyl group is preferable, and a silyl group having three alkyl groups having 1 to 3 carbon atoms is more preferable.

As the silyloxy structure in the above-described hydrocarbon group having a silyloxy structure, a trialkylsilyloxy structure is preferable, and a silyloxy structure having three alkyl groups having 1 to 3 carbon atoms is more preferable.

In addition, the above-described hydrocarbon group having a silyloxy structure preferably has 1 to 3 silyloxy structures.

Furthermore, the number of carbon atoms in the above-described hydrocarbon group having a silyloxy structure is preferably 10 or more, more preferably 10 to 100, and particularly preferably 16 to 50.

Preferred examples of the above-described hydrocarbon group having a silyloxy structure include a group represented by Formula (Si).

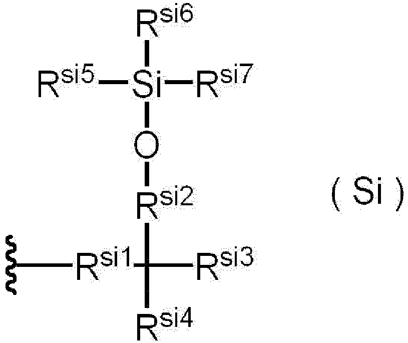

In Formula (Si), R^{sil} represents a single bond or an alkylene group having 1 to 3 carbon atoms, R^{si2} represents an alkylene group having 1 to 3 carbon atoms, R^{si3} and R^{si4} each independently represent a hydrogen atom, an alkyl group having 1 to 6 carbon atoms, or -OSiR^{si5}R^{si6}R^{si7}, and R^{si5} to R^{si7} each independently represent an alkyl group having 1 to 6 carbon atoms or an aryl group.

R^{si5} to R^{si7} in Formula (Si) are each independently preferably an alkyl group having 1 to 6 carbon atoms or a phenyl group, more preferably an alkyl group having 1 to 6 carbon atoms, and particularly preferably a linear or branched alkyl group having 1 to 4 carbon atoms.

As the perfluoroalkyl structure in the above-described organic group having a perfluoroalkyl structure, a perfluoroalkyl structure having 1 to 20 carbon atoms is preferable, a perfluoroalkyl structure having 5 to 20 carbon atoms is more preferable, and a perfluoroalkyl structure having 7 to 16 carbon atoms is particularly preferable. In addition, the above-described perfluoroalkyl structure may be linear, may have a branch, or may have a ring structure.

The above-described organic group having a perfluoroalkyl structure is preferably a perfluoroalkyl group, an alkyl group having a perfluoroalkyl structure, or an alkyl group having a perfluoroalkyl structure and an amide bond in the alkyl chain.

The number of carbon atoms in the above-described organic group having a perfluoroalkyl structure is preferably 5 or more, more preferably 10 or more, still more preferably 10 to 100, and particularly preferably 16 to 50.

Preferred examples of the above-described organic group having a perfluoroalkyl structure include groups shown below.

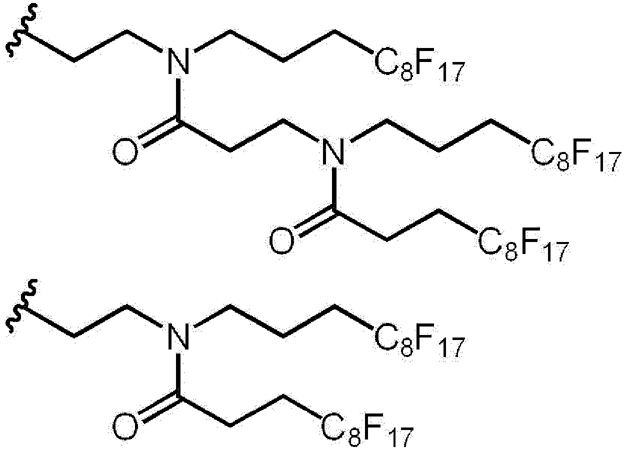

A moiety other than the aliphatic hydrocarbon group in the organic group having an aliphatic hydrocarbon group can be optionally set. For example, the "organic group having an aliphatic hydrocarbon group" may have a moiety such as -O-, -S-, -COO-, -OCONH-, -CONH-, and a hydrocarbon group (monovalent group or divalent group) other than the "aliphatic hydrocarbon group".

Examples of the hydrocarbon group other than the aliphatic hydrocarbon group include an aliphatic hydrocarbon group having 1 to 4 carbon atoms and an aromatic hydrocarbon group, and specifically, for example, a monovalent group such as an alkyl group having 1 to 4 carbon atoms and an aryl group or a divalent group derived from the monovalent group is used.

As the "aryl group", for example, an aryl group having 6 to 14 carbon atoms or the like is preferable, and examples thereof include a phenyl group, a 1-naphthyl group, a 2-naphthyl group, a biphenylyl group, and a 2-anthryl group. Among these, an aryl group having 6 to 10 carbon atoms is more preferable, and a phenyl group is particularly preferable.

In addition, the above-described aliphatic hydrocarbon group and the hydrocarbon group other than the above-described aliphatic hydrocarbon group may be substituted with a substituent selected from a halogen atom, an oxo group, and the like.

In R^{A}, the bond (substitution) of the organic group having an aliphatic hydrocarbon group to the ring A may be through the above-described aliphatic hydrocarbon group or the above-described hydrocarbon group existing in R^{A}, that is, may be directly bonded by a carbon-carbon bond, or may be through a moiety such as -O-, -S-, -COO-, -OCONH-, and -CONH-, which exists in RA. From the viewpoint of ease of synthesizing the compound, it is preferable to be through -O-, -S-, -COO-, or -CONH-, and it is particularly preferable to be through -O-.

In the compound represented by Formula (1) according to the present disclosure, from the viewpoint of solubility in a solvent, crystallization property, and yield, the total number of carbon atoms in all aliphatic hydrocarbon groups included in all R^{A}, R^{B}, and R^{C} is preferably 24 or more, more preferably 24 to 200, still more preferably 32 to 100, particularly preferably 34 to 80, and most preferably 36 to 80.

In addition, in the compound represented by Formula (1) according to the present disclosure, from the viewpoint of solubility in a solvent, crystallization property, and yield, the total number of carbon atoms in all aliphatic hydrocarbon groups included in all R^{A}'s is preferably 24 or more, more preferably 24 to 200, still more preferably 32 to 100, particularly preferably 34 to 80, and most preferably 36 to 80.

In addition, the compound represented by Formula (1) according to the present disclosure is a compound in which the number of carbon atoms in at least one aliphatic hydrocarbon group included in at least one of R^{A}, R^{B}, or R^{C} is 12 or more, and from the viewpoint of solubility in a solvent, crystallization property, and yield, it is preferable to be a compound having at least one aliphatic hydrocarbon group having 12 or more carbon atoms in at least one R^{A} or R^{C}, more preferable to be a compound having at least one aliphatic hydrocarbon group having 12 to 100 carbon atoms in at least one R^{A}, still more preferable to be a compound having at least one aliphatic hydrocarbon group having 18 to 40 carbon atoms in at least one R^{A}, and particularly preferable to be a compound having at least one aliphatic hydrocarbon group having 20 to 36 carbon atoms in at least one R^{A}.

In the compound represented by Formula (1) according to the present disclosure, the above-described effects are more excellent in a case where the number of carbon atoms in at least one aliphatic hydrocarbon group in R^{A}, R^{B}, or R^{C} is preferably 14 or more, more preferably 16 or more, still more preferably 18 or more, and particularly preferably 20 or more. The reason is considered that, as the number of carbon atoms increases, the contribution ratio of hydrophobicity in the entire molecule increases, which makes it easier to dissolve in a hydrophobic solvent, and with regard to a hydrophilic solvent, presumed that, as the number of carbon atoms increases, the cohesive force increases, which makes it easier to be crystallized.

Furthermore, from the viewpoint of crystallization property and yield, the above-described aliphatic hydrocarbon group is preferably an alkyl group and more preferably a linear alkyl group.

In addition, from the viewpoint of solubility in a solvent, crystallization property, and yield, the number of carbon atoms in one R^{A}, R^{B}, or R^{C} is preferably 12 to 200, more preferably 18 to 150, still more preferably 18 to 100, and particularly preferably 20 to 80, respectively.

Furthermore, the aliphatic hydrocarbon group having 12 or more carbon atoms included in the compound represented by Formula (1) according to the present disclosure is included in at least one of R^{A}, R^{B}, or R^{C}, and from the viewpoint of solubility in a solvent, crystallization property, and yield, it is preferable to be included in at least one of R^{A} or R^{C} and it is more preferable to be included in at least one of R^{A}'s.

From the viewpoint of yield and compound stability of peptide condensate, m in Formula (1) is preferably 1. In addition, from the viewpoint of deprotection property, m in Formula (1) is preferably 2.

From the viewpoint of solubility in a solvent, crystallization property, and yield, R^{B}'s in Formula (1) are each independently preferably a monovalent aliphatic hydrocarbon group or a (1+c)-valent aromatic group, more preferably a monovalent alkyl group having 1 to 30 carbon atoms or a (1+c)-valent aromatic group having 6 to 30 carbon atoms, still more preferably a monovalent alkyl group having 1 to 26 carbon atoms or a (1+c)-valent aromatic group having 6 to 20 carbon atoms, and particularly preferably a monovalent alkyl group having 1 to 22 carbon atoms or a (1+c)-valent aromatic group having 6 to 10 carbon atoms.

The monovalent alkyl group, (1+c)-valent aromatic group, and (1+c)-valent heteroaromatic group in R^{B} may have a substituent. The above-described substituent is not particularly limited, and examples thereof include an alkoxy group, an aryloxy group, a halogen atom, an alkyl group, an aryl group, an acyl group, an acyloxy group, an alkoxycarbonyl group, an aryloxycarbonyl group, an alkylthio group, an arylthio group, R^{st}-CO-NR^{st}-, -CON(R^{st})₂, a dialkylamino group, an alkylarylamino group, a diarylamino group, and a group obtained by combining two or more of these groups. R^{st} represents a hydrogen atom, an alkyl group, or an aryl group.

Examples of the (1+c)-valent aromatic group in R^{B} include a phenyl group, a fluorophenyl group, a difluorophenyl group, a chlorophenyl group, a dichlorophenyl group, a trichlorophenyl group, a methylphenyl group (tolyl group), a dimethylphenyl group (xylyl group), a methoxyphenyl group, a dimethoxyphenyl group, a trimethoxyphenyl group, a phenylene group, a benzenetriyl group, a 1-naphthyl group, a 2-naphthyl group, a naphthylene group, a naphthalenetriyl group.

Among these, in a case where m is 1 and Y^{A} is -OH, from the viewpoint of yield, a phenyl group, a methylphenyl group, a dimethylphenyl group, a methoxyphenyl group, a dimethoxyphenyl group, or a trimethoxyphenyl group is preferable, and a phenyl group, a methylphenyl group, or a methoxyphenyl group is more preferable.

In a case where m is 1 and Y^{A} is -NHR, from the viewpoint of deprotection property, a phenyl group, a methylphenyl group, a dimethylphenyl group, a methoxyphenyl group, a dimethoxyphenyl group, or a trimethoxyphenyl group is preferable, and a methylphenyl group, a dimethylphenyl group, a methoxyphenyl group, a dimethoxyphenyl group, or a trimethoxyphenyl group is more preferable.

In a case where m is 2 and Y^{A} is -OH, from the viewpoint of compound stability of peptide condensate, a phenyl group, a fluorophenyl group, a difluorophenyl group, a chlorophenyl group, a dichlorophenyl group, or a trichlorophenyl group is preferable, and a fluorophenyl group, a difluorophenyl group, a chlorophenyl group, a dichlorophenyl group, or a trichlorophenyl group is more preferable.

Examples of the (1+c)-valent heteroaromatic group in R^{B} include a monocyclic nitrogen-containing heteroaromatic group, a monocyclic oxygen-containing heteroaromatic group, a monocyclic sulfur-containing heteroaromatic group, a monocyclic nitrogen and oxygen-containing heteroaromatic group, a monocyclic nitrogen and sulfur-containing heteroaromatic group, a bicyclic nitrogen-containing heteroaromatic group, a bicyclic oxygen-containing heteroaromatic group, a bicyclic sulfur-containing heteroaromatic group, a bicyclic nitrogen and oxygen-containing heteroaromatic group, and a bicyclic nitrogen and sulfur-containing heteroaromatic group, which are (1+c)-valent. Specific examples thereof include a pyridyl group, a furanyl group, a thienyl group, a pyrrolyl group, an oxazolyl group, an imidazolyl group, a pyrazolyl group, a triazolyl group, a tetrazolyl group, a pyridinediyl group, a pyridinetriyl group, a furandiyl group, a thiophendiyl group, a pyrroldiyl group, a benzofrangyl group, a benzothiopheneyl group, and an indoldiyl group.

In Formula (1), from the viewpoint of solubility in a solvent, crystallization property, and yield, it is preferable that at least one R^{A} or R^{C} is a group represented by any of Formula (f1), Formula (a1), Formula (b1), or Formula (e1), it is more preferable to be a group represented by Formula (f1) or Formula (a1), and it is particularly preferable to be a group represented by Formula (f1).

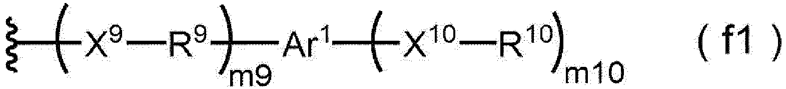

In Formula (f1), a wavy line portion represents a bonding position to other structures, m9 represents an integer of 1 to 3, X⁹'s each independently represent a single bond, -O-, -S-, -COO-, -OCO-, -OCONH-, -NHCONH-, -NHCO-, or -CONH-, R⁹'s each independently represent a divalent aliphatic hydrocarbon group, Ar¹ represents an (m10+1)-valent aromatic group or an (m10+1)-valent heteroaromatic group, m10 represents an integer of 1 to 3, X¹⁰'s each independently represent a single bond, -O-, -S-, -COO-, -OCO-, -OCONH-, -NHCONH-, -NHCO-, or -CONH-, R¹⁰'s each independently represent a monovalent aliphatic hydrocarbon group, and at least one of R¹⁰'s is a monovalent aliphatic hydrocarbon group having 5 or more carbon atoms.

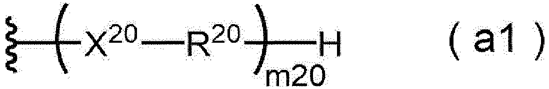

In Formula (a1), a wavy line portion represents a bonding position to other structures, m20 represents an integer of 1 to 10, X²⁰'s each independently represent a single bond, -O-, -S-, -COO-, -OCO-, -OCONH-, -NHCONH-, -NHCO-, or -CONH-, R²⁰'s each independently represent a divalent aliphatic hydrocarbon group, and at least one of R²⁰'s is a divalent aliphatic hydrocarbon group having 5 or more carbon atoms.

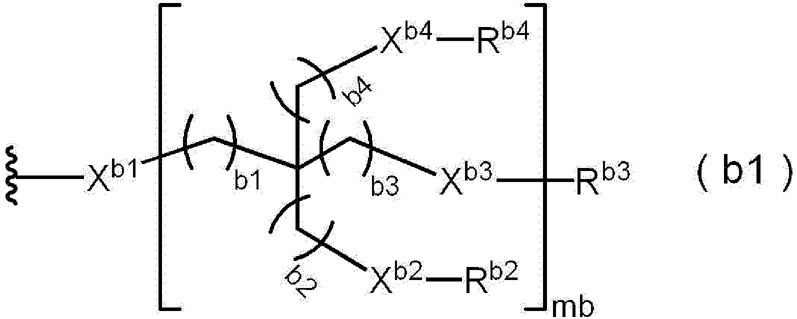

In Formula (b1), a wavy line portion represents a bonding position to other structures, mb represents 1 or 2, b1 to b4 each independently represent an integer of 0 to 2, X^{b1} to X^{b4} each independently represent a single bond, -O-, -S-, -COO-, -OCONH-, or -CONH-, R^{b2} and R^{b4} each independently represent a hydrogen atom, a methyl group, or an aliphatic hydrocarbon group having 5 or more carbon atoms, and R^{b3} represents an aliphatic hydrocarbon group having 5 or more carbon atoms.

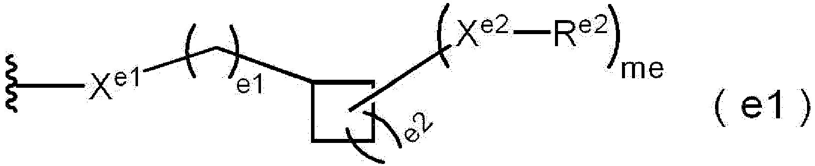

In Formula (e1), a wavy line portion represents a bonding position to other structures, X^{e1} represents a single bond, -O-, -S-, -NHCO-, or -CONH-, me represents an integer of 0 to 15, e1 represents an integer of 0 to 11, e2 represents an integer of 0 to 5, X^{e2}'s each independently represent a single bond, -O-, -S-, -COO-, -OCONH-, -NHCO-, or -CONH-, and R^{e2}'S each independently represent a hydrogen atom, a methyl group, or an organic group having an aliphatic hydrocarbon group having 5 or more carbon atoms.

m9 in Formula (f1) is preferably 1 or 2 and more preferably 1.
X⁹ and X¹⁰ in Formula (f1) are each independently preferably -O-, -S-, -COO-, -OCONH-, or -CONH-, and more preferably -O-.
R⁹'s in Formula (f1) are each independently preferably an alkylene group having 1 to 10 carbon atoms, more preferably an alkylene group having 1 to 4 carbon atoms, and particularly preferably a methylene group.
R¹⁰'s in Formula (f1) are each independently preferably a monovalent aliphatic hydrocarbon group having 5 to 60 carbon atoms, more preferably a monovalent aliphatic hydrocarbon group having 12 to 50 carbon atoms, still more preferably a monovalent aliphatic hydrocarbon group having 18 to 40 carbon atoms, and particularly preferably a monovalent aliphatic hydrocarbon group having 20 to 32 carbon atoms. In addition, R¹⁰'s are each independently preferably a linear alkyl group or a branched alkyl group and more preferably a linear alkyl group.
m10 in Formula (f1) is preferably 2 or 3 and more preferably 2.
Ar¹ in Formula (f1) is preferably an (m10+1)-valent aromatic group, more preferably a group obtained by removing (m10+1) pieces of hydrogen atoms from benzene or a group obtained by removing (m10+1) pieces of hydrogen atoms from naphthalene, and particularly preferably a group obtained by removing (m10+1) pieces of hydrogen atoms from benzene.

In addition, from the viewpoint of solubility in a solvent, crystallization property, and yield, the group represented by Formula (f1) is preferably a group represented by Formula (f2).

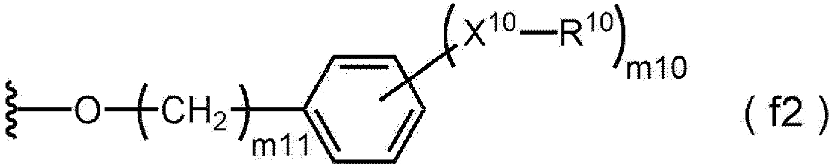

In Formula (f2), a wavy line portion represents a bonding position to other structures, m10 represents an integer of 1 to 3, m11 represents an integer of 1 to 3, X¹⁰'s each independently represent a single bond, -O-, -S-, -COO-, -OCO-, -OCONH-, -NHCONH-, -NHCO-, or -CONH-, and R¹⁰'s each independently represent a monovalent aliphatic hydrocarbon group having 5 or more carbon atoms.

m10, X¹⁰, and R¹⁰ in Formula (f2) have the same meaning as m10, X¹⁰, and R¹⁰ in Formula (f1), respectively, and the preferred aspects thereof are also the same.
m11 in Formula (f2) is preferably 1 or 2 and more preferably 1.

m20 in Formula (a1) is preferably 1 or 2.
X²⁰'s in Formula (a1) are each independently preferably -O-, -S-, -COO-, -OCONH-, or -CONH-, and more preferably -O-.
R²⁰ in Formula (a1) is preferably a divalent aliphatic hydrocarbon group having 5 or more carbon atoms, more preferably a divalent aliphatic hydrocarbon group having 5 to 60 carbon atoms, still more preferably a divalent aliphatic hydrocarbon group having 8 to 40 carbon atoms, and particularly preferably a divalent aliphatic hydrocarbon group having 12 to 32 carbon atoms. In addition, R²⁰ is preferably a linear alkylene group.

mb in Formula (b1) is preferably 1.
b1 to b4 in Formula (b1) are each independently preferably 1 or 2 and more preferably 1.
X^{b1} to X^{b4} in Formula (b1) are each independently preferably -O-, -S-, -COO-, -OCONH-, or -CONH-, and more preferably -O-.
R^{b2} and R^{b4} in Formula (b1) are each independently preferably a hydrogen atom, a methyl group, or an aliphatic hydrocarbon group having 5 to 60 carbon atoms, more preferably a hydrogen atom, a methyl group, or an alkyl group having 8 to 40 carbon atoms, and particularly preferably a hydrogen atom, a methyl group, or an alkyl group having 12 to 32 carbon atoms.
R^{b3} in Formula (b1) is preferably a monovalent aliphatic hydrocarbon group having 5 to 60 carbon atoms, more preferably a monovalent aliphatic hydrocarbon group having 8 to 40 carbon atoms, and particularly preferably a monovalent aliphatic hydrocarbon group having 12 to 32 carbon atoms. In addition, R^{b3} is preferably a linear alkyl group.

In addition, in the compound represented by Formula (1) according to the present disclosure, from the viewpoint of solubility in a solvent, preferred examples of the above-described aliphatic hydrocarbon group include an aliphatic hydrocarbon group having a branch, and more preferred examples thereof include groups shown below. A wavy line portion represents a bonding position to another structures, nt2 represents an integer of 3 or more, and nt3 represents an integer set such that the total number of carbon atoms in the following group is 14 to 300.

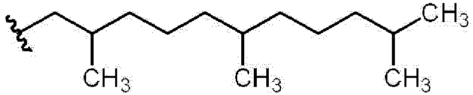

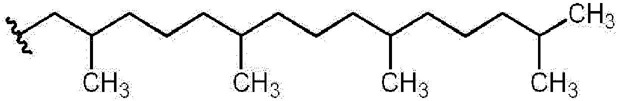

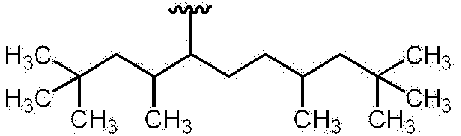

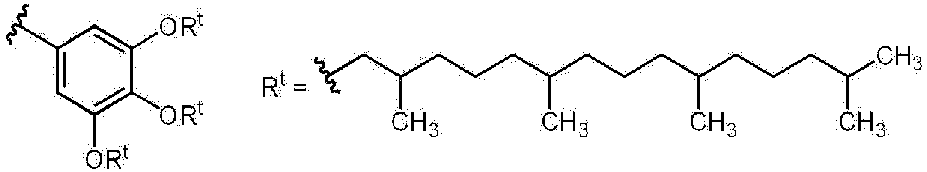

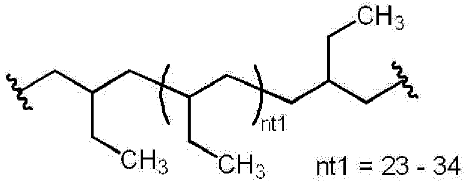

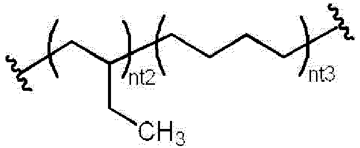

The substituent which may be included in the compound represented by Formula (1) on the ring A or R^{B} is not particularly limited, and examples thereof include an alkyl group having 1 to 4 carbon atoms, an alkoxy group, an aryloxy group, a halogen atom, a halogenated alkyl group, an aryl group, an acyl group, an acyloxy group, an alkoxycarbonyl group, an aryloxycarbonyl group, an alkylthio group, an arylthio group, R^{st}-CO-NR^{st}-, -CON(R^{st})₂, a dialkylamino group, an alkylarylamino group, a diarylamino group, and a group obtained by combining two or more of these groups. R^{st} represents a hydrogen atom, an alkyl group, or an aryl group.

From the viewpoint of deprotection rate, crystallization property, solubility in a solvent, and yield, the condensed polycyclic aromatic hydrocarbon compound represented by Formula (1) is preferably a compound represented by Formula (10) or Formula (20), and more preferably a compound represented by Formula (10).

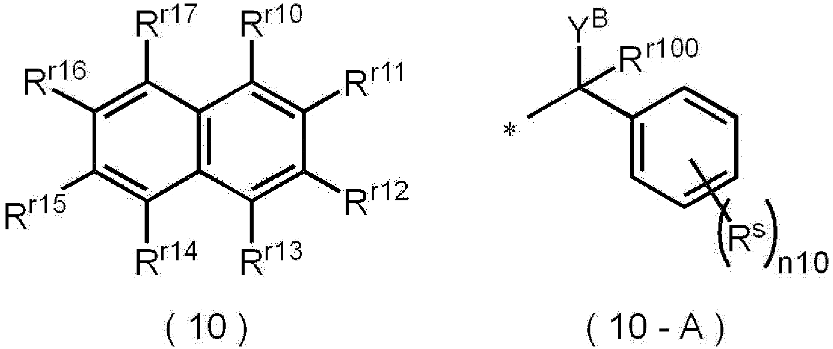

In Formula (10) and Formula (10-A),
Y^{B} represents -OH, -NHR, -SH, or -X⁰, where R represents a hydrogen atom, an alkyl group, an aromatic group-substituted alkyl group, a heteroaromatic group-substituted alkyl group, or an Fmoc group, and X⁰ represents Cl, Br, or I,
R^{r100} represents a hydrogen atom, an aryl group, or a heteroaryl group,
R^{s} represents a substituent or R^{A},
n10 represents an integer of 0 to 5,
adjacent R^{S}'s may be linked to each other through a substituent to form a ring,
R^{r10} and R^{r11} each independently represent a hydrogen atom, a substituent, a group represented by Formula (10-A), or R^{A},
any one of R^{r10} or R^{r11} is a group represented by Formula (10-A),
^{∗} represents a linking position with R^{r10} or R^{r11},
R^{r12} to R^{r17} each independently represent a hydrogen atom, a substituent, or R^{A},
at least one of R^{s}, R^{r1O} , ... , or R^{r17} is R^{A},
R^{A}'s each independently represent an aliphatic hydrocarbon group or an organic group having an aliphatic hydrocarbon group, and
the number of carbon atoms in at least one of the aliphatic hydrocarbon group included in at least one R^{A} is 12 or more.

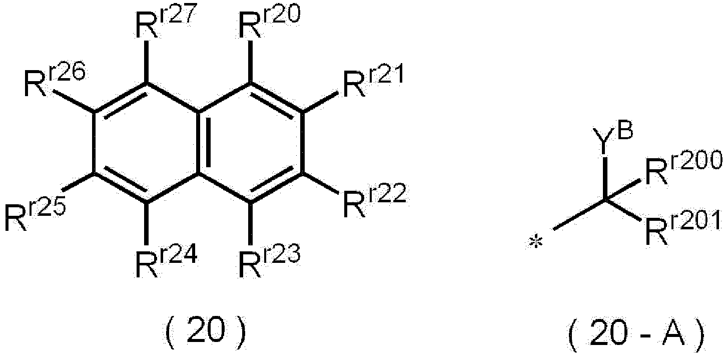

In Formula (20) and Formula (20-A),
Y^{B} represents -OH, -NHR, -SH, or -X⁰, where R represents a hydrogen atom, an alkyl group, an aromatic group-substituted alkyl group, a heteroaromatic group-substituted alkyl group, or a 9-fluorenylmethoxycarbonyl group (Fmoc group), and X⁰ represents Cl, Br, or I,
R^{r200} represents a hydrogen atom, an alkyl group, an aryl group, or a heteroaryl group,
R^{r201} represents an alkyl group,
R^{r20} and R^{r21} each independently represent a hydrogen atom, a substituent, a group represented by Formula (20-A), or R^{A},
any one of R^{r20} or R^{r21} is a group represented by Formula (20-A),
^{∗} represents a linking position with R^{r20} or R^{r21},
R^{r22} to R^{r27} each independently represent a hydrogen atom, a substituent, or R^{A},
the number of carbon atoms in R^{r201} is 12 or more, or at least one of R^{r20}, ... , or R^{r27} is R^{A},
R^{A}'s each independently represent an aliphatic hydrocarbon group or an organic group having an aliphatic hydrocarbon group, and
the number of carbon atoms in at least one of the aliphatic hydrocarbon group included in at least one R^{A} is 12 or more.

Y^{B}, R^{A}, R, and X⁰ in Formula (10) or Formula (20) have the same meaning as Y^{A}, R^{A}, R, and X⁰ in Formula (1), respectively, and the preferred aspects thereof are also the same.

From the viewpoint of deprotection rate, crystallization property, solubility in a solvent, and yield, R^{r100} in Formula (10) is preferably a hydrogen atom or an aryl group, and from the viewpoint of compound stability of peptide condensate, a hydrogen atom is particularly preferable.

The substituent in R^{s} of Formula (10) is not particularly limited, and examples thereof include an alkoxy group, an aryloxy group, a halogen atom, an alkyl group, a halogenated alkyl group, an aryl group, an acyl group, an acyloxy group, an alkoxycarbonyl group, an aryloxycarbonyl group, an alkylthio group, an arylthio group, R^{st}-CO-NR^{st}-, -CON(R^{st})₂, a dialkylamino group, an alkylarylamino group, a diarylamino group, a cyano group, and a group obtained by combining two or more of these groups. R^{st} represents a hydrogen atom, an alkyl group, or an aryl group. In addition, the above-described substituent in R^{s} is preferably a group having 0 to 4 carbon atoms.

Among these, as the above-described substituent in R^{s}, an alkoxy group, an aryloxy group, a halogen atom, an alkyl group, a halogenated alkyl group, or an aryl group is more preferable, an alkoxy group or an alkyl group is still more preferable, and an alkoxy group is particularly preferable.

From the viewpoint of deprotection rate, R^{s} in Formula (10) is preferably a substituent, and more preferably an alkyl group, an alkoxy group, an aryloxy group, a halogen atom, a halogenated alkyl group, or a cyano group. In particular, in a case where R^{r100} is a hydrogen atom, from the viewpoint of desorption rate, an alkyl group or an alkoxy group is still more preferable, and a methoxy group is particularly preferable. In addition, in a case where R^{r100} is a substituent other than a hydrogen atom, from the viewpoint of compound stability of peptide condensate, R^{s} is preferably a halogen atom, and more preferably a fluorine atom or a chlorine atom.
n10 in Formula (10) is preferably an integer of 0 to 3 and more preferably an integer of 0 to 2.

R^{r10} and R^{r11} in Formula (10) are each independently preferably a hydrogen atom, a group represented by Formula (10-A), or R^{A}, and more preferably a hydrogen atom or a group represented by Formula (10-A).

In addition, it is preferable that at least R^{r11} in Formula (10) is a group represented by Formula (10-A), and it is more preferable that R^{r10} is a hydrogen atom and R^{r11} is a group represented by Formula (10-A).

The substituent in R^{r10} to R^{r17} of Formula (10) has the same meaning as the substituent in Rs, and the preferred aspects thereof are also the same.

R^{r12} to R^{r17} in Formula (10) are preferably a hydrogen atom, an alkoxy group, an aryloxy group, a halogen atom, an alkyl group, a halogenated alkyl group, a cyano group, or R^{A}, more preferably a hydrogen atom, an alkoxy group, a halogen atom, an alkyl group, or R^{A}, and particularly preferably a hydrogen atom or R^{A}.

In addition, in Formula (10), the number of carbon atoms in at least one of the aliphatic hydrocarbon group included in at least one R^{A} is 12 or more, preferably 14 or more, more preferably 16 or more, still more preferably 18 or more, and particularly preferably 20 to 40.

Furthermore, in Formula (10), it is preferable that at least one of R^{r14}, R^{r15}, R^{r16}, or R^{r17} is R^{A}, and it is more preferable that R^{r15} is R^{A}.

In addition, the number of R^{A}'s in R^{r10} to R^{r17} is preferably 1 or 2, and more preferably 1.

From the viewpoint of deprotection rate, crystallization property, solubility in a solvent, and yield, R^{r200} in Formula (20) is preferably a hydrogen atom, or an alkyl group, and more preferably a hydrogen atom.

From the viewpoint of deprotection rate, crystallization property, solubility in a solvent, and yield, R^{r201} in Formula (20) is preferably an alkyl group having 1 to 30 carbon atoms, more preferably an alkyl group having 8 to 30 carbon atoms, and particularly preferably an alkyl group having 12 to 24 carbon atoms.

R^{r20} and R^{r21} in Formula (20) are each independently preferably a hydrogen atom, a group represented by Formula (20-A), or R^{A}, and more preferably a hydrogen atom or a group represented by Formula (20-A).

In addition, it is preferable that at least R^{r21} in Formula (20) is a group represented by Formula (20-A), and it is more preferable that R^{r20} is a hydrogen atom and R^{r21} is a group represented by Formula (20-A).

The substituent in R^{r20} to R^{r27} of Formula (20) has the same meaning as the substituent in Rs, and the preferred aspects thereof are also the same.

R^{r22} to R^{r27} in Formula (20) are preferably a hydrogen atom, an alkoxy group, an aryloxy group, a halogen atom, an alkyl group, a halogenated alkyl group, a cyano group, or R^{A}, more preferably a hydrogen atom, an alkoxy group, a halogen atom, an alkyl group, or R^{A}, and particularly preferably a hydrogen atom or R^{A}.

In addition, in Formula (20), the number of carbon atoms in R^{r201} is 12 or more, or at least one of R^{r20}, ... , or R^{r27} is R^{A}, and in a case of having R^{A}, the number of carbon atoms in at least one of the aliphatic hydrocarbon group included in at least one R^{A} is 12 or more. In addition, it is preferable that at least one of R^{r20}, ... , or R^{r27} is R^{A}, and the number of carbon atoms in at least one of the aliphatic hydrocarbon group included in at least one R^{A} is 12 or more.

The number of carbon atoms in the above-described aliphatic hydrocarbon group is preferably 14 or more, more preferably 16 or more, still more preferably 18 or more, and particularly preferably 20 to 40.

In Formula (20), it is preferable that at least one of R^{r24}, R^{r25}, R^{r26}, or R^{r27} is R^{A}, and it is more preferable that R^{r25} is R^{A}.

The number of R^{A}'s in R^{r20} to R^{r27} is preferably 1 or 2, and more preferably 1.

From the viewpoint of solubility in a solvent, crystallization property, and yield, it is preferable that R^{A} in Formula (10) of Formula (20) is a group represented by any of Formula (f1), Formula (a1), Formula (b1), or Formula (e1) described above, it is more preferable to be a group represented by any of Formula (f1) or Formula (a1) described above, it is still more preferable to be a group represented by Formula (f1) described above, and it is particularly preferable to be a group represented by Formula (f2).

In addition, from the viewpoint of deprotection rate, crystallization property, solubility in a solvent, and yield, the condensed polycyclic aromatic hydrocarbon compound represented by Formula (1) is still more preferably a compound represented by Formula (30) or Formula (40), and particularly preferably a compound represented by Formula (40).

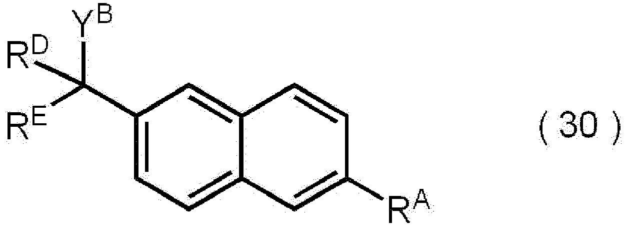

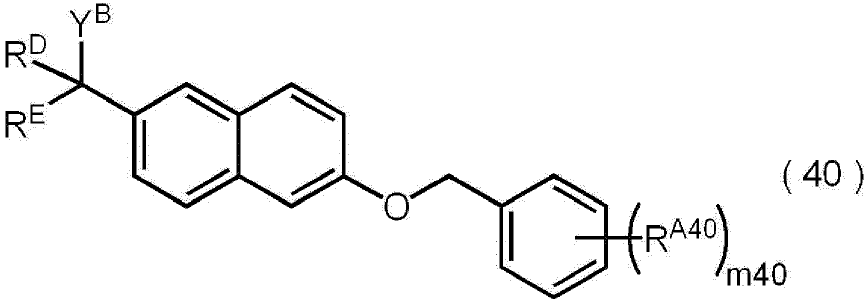

In Formula (30) and Formula (40),
Y^{B} represents -OH, -NHR, -SH, or -X⁰, where R represents a hydrogen atom, an alkyl group, an aromatic group-substituted alkyl group, a heteroaromatic group-substituted alkyl group, or an Fmoc group, and X⁰ represents Cl, Br, or I,
R^{D} represents an alkyl group, an aryl group, or a heteroaryl group,
R^{E} represents a hydrogen atom, an alkyl group, an aryl group, or a heteroaryl group,
R^{A} represents an aliphatic hydrocarbon group having 12 or more carbon atoms or an organic group having an aliphatic hydrocarbon group having 12 or more carbon atoms,
R^{A40}'S each independently represent an alkyl group having 12 or more carbon atoms or an alkoxy group having 12 or more carbon atoms, and
m40 represents an integer of 1 to 3.

Y^{B}, R^{A}, R, and X⁰ in Formula (30) or Formula (40) have the same meaning as Y^{A}, R^{A}, R, and X⁰ in Formula (1), respectively, and the preferred aspects thereof are also the same.

From the viewpoint of deprotection rate, crystallization property, solubility in a solvent, and yield, R^{D} in Formula (30) or Formula (40) is preferably an alkyl group or an aryl group, and more preferably an aryl group. In particular, in a case where R^{E} is a hydrogen atom and Y^{B} is -OH, a phenyl group, an alkylphenyl group, or an alkoxyphenyl group is preferable, and in a case where R^{E} is a hydrogen atom and Y^{B} is -NHR, an alkylphenyl group, an alkoxyphenyl group, a dialkylphenyl group, or a dialkoxyphenyl group is preferable, and a dialkylphenyl group or a dialkoxyphenyl group is more preferable.

In addition, the number of carbon atoms in R^{D} is preferably 1 to 30, more preferably 5 to 30, and particularly preferably 6 to 24.

From the viewpoint of deprotection rate, crystallization property, solubility in a solvent, and yield, R^{E} in Formula (30) or Formula (40) is preferably a hydrogen atom, an alkyl group, or an aryl group, and more preferably a hydrogen atom or an aryl group, and from the viewpoint of compound stability of peptide condensate, a hydrogen atom is particularly preferable.

R^{A40}'S in Formula (40) are each independently preferably an alkoxy group having 12 or more carbon atoms.

In addition, the number of carbon atoms in R^{A40} is preferably 14 or more, more preferably 16 or more, still more preferably 18 or more, and particularly preferably 20 to 40.

Furthermore, a substitution position of R^{A40} on the benzene ring is not particularly limited, but is preferably any of 3rd to 5th positions, in a case where the bonding position of the OCH₂ group on the benzene ring is the 1st position.
m40 in Formula (40) is preferably 1 or 2 and more preferably 2.

The molecular weight of the compound represented by Formula (1) is not particularly limited, but from the viewpoint of deprotection rate, crystallization property, solubility in a solvent, and yield, it is preferably 340 to 3,000, more preferably 400 to 2,000, still more preferably 500 to 1,500, and particularly preferably 800 to 1,300. In addition, in a case where the molecular weight is 3,000 or less, the proportion of Formula (1) in the target product is appropriate and the proportion of a compound obtained by deprotecting Formula (1) is not reduced, so that productivity is excellent.

Preferred specific examples of the compound represented by Formula (1) include compounds shown below, but needless to say, the compound represented by Formula (1) is not limited thereto. Me represents a methyl group, and Et represents an ethyl group.

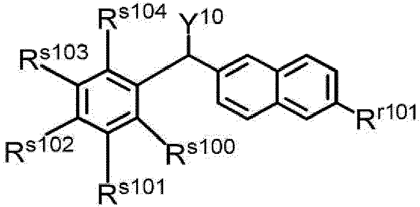

| Y¹⁰ | R^{s100} | R^{s101} | R^{s102} | R^{s103} | R^{s104} | R^{r101} |
|---|---|---|---|---|---|---|
| OH | H | H | H | H | H | 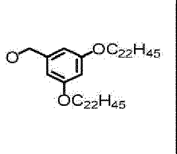 |
| OH | H | H | OMe | H | H | |
| OH | H | H | Me | H | H | |
| OH | OMe | H | H | H | H | |
| OH | Me | H | H | H | H | |
| OH | OMe | H | OMe | H | H | |
| OH | OMe | H | OMe | H | OMe | |
| OH | Me | H | Me | H | H | |
| OH | Me | H | Me | H | Me | |
| OH | H | H | C₆H₁₃ | H | H | |
| OH | H | H | OC₁₂H₂₅ | H | H | |
| OH | H | H | OC₁₈H₃₇ | H | H | |
| OH | OMe | H | OC₁₈H₃₇ | H | H | |
| OH | OC₁₈H₃₇ | H | OC₁₈H₃₇ | H | H | |
| NH₂ | H | H | H | H | H | |
| NH₂ | H | H | OMe | H | H | |
| NH₂ | OMe | H | OMe | H | H | |
| NH₂ | OMe | H | OMe | H | OMe | |
| NH₂ | Me | H | Me | H | H | |
| NH₂ | Me | H | Me | H | Me | |
| NHEt | H | H | H | H | H | |
| NHEt | H | H | OMe | H | H | |
| NHEt | H | H | Me | H | H | |
| NHEt | OMe | H | H | H | H | |
| NHEt | Me | H | H | H | H | |
| NHEt | OMe | H | OMe | H | H | |
| NHEt | OMe | H | OMe | H | OMe | |
| NHEt | Me | H | Me | H | H | |
| NHEt | Me | H | Me | H | Me | |
| SH | H | H | H | H | H | |
| Cl | H | H | H | H | H | |
| Br | H | H | H | H | H | |

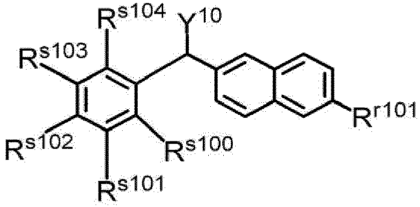

| Y¹⁰ | R^{s100} | R^{s101} | R^{s102} | R^{s103} | R^{s104} | R^{r101} |
|---|---|---|---|---|---|---|
| OH | H | H | H | H | H | 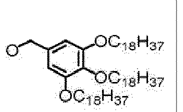 |
| OH | H | H | OMe | H | H | |
| OH | H | H | Me | H | H | |
| OH | OMe | H | H | H | H | |
| OH | Me | H | H | H | H | |
| OH | OMe | H | OMe | H | H | |
| OH | OMe | H | OMe | H | OMe | |
| OH | Me | H | Me | H | H | |
| OH | Me | H | Me | H | Me | |
| OH | H | H | C₆H₁₃ | H | H | |
| OH | H | H | OC₁₂H₂₅ | H | H | |
| OH | H | H | OC₁₈H₃₇ | H | H | |
| OH | OMe | H | OC₁₈H₃₇ | H | H | |
| OH | OC₁₈H₃₇ | H | OC₁₈H₃₇ | H | H | |
| NH₂ | H | H | H | H | H | |
| NH₂ | H | H | OMe | H | H | |
| NH₂ | OMe | H | OMe | H | H | |
| NH₂ | OMe | H | OMe | H | OMe | |
| NH₂ | Me | H | Me | H | H | |
| NH₂ | Me | H | Me | H | Me | |
| NHEt | H | H | H | H | H | |
| NHEt | H | H | OMe | H | H | |
| NHEt | H | H | Me | H | H | |
| NHEt | OMe | H | H | H | H | |
| NHEt | Me | H | H | H | H | |
| NHEt | OMe | H | OMe | H | H | |
| NHEt | OMe | H | OMe | H | OMe | |
| NHEt | Me | H | Me | H | H | |
| NHEt | Me | H | Me | H | Me | |
| SH | H | H | H | H | H | |
| Cl | H | H | H | H | H | |
| Br | H | H | H | H | H | |

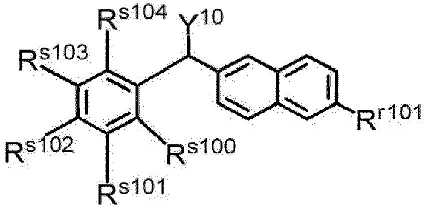

| Y¹⁰ | R^{s100} | R^{s101} | R^{s102} | R^{s103} | R^{s104} | R^{r101} |
|---|---|---|---|---|---|---|
| OH | H | H | H | H | H | |
| OH | H | H | OMe | H | H | |
| OH | H | H | Me | H | H | |
| OH | OMe | H | H | H | H | |
| OH | Me | H | H | H | H | |
| OH | OMe | H | OMe | H | H | |
| OH | OMe | H | OMe | H | OMe | |
| OH | Me | H | Me | H | H | |
| OH | Me | H | Me | H | Me | OC₂₂H₄₅ |
| OH | H | H | C₆H₁₃ | H | H | |
| OH | H | H | OC₁₂H₂₅ | H | H | |
| OH | H | H | OC₁₈H₃₇ | H | H | |
| OH | OMe | H | OC₁₈H₃₇ | H | H | |
| OH | OC₁₈H₃₇ | H | OC₁₈H₃₇ | H | H | |
| NH₂ | H | H | H | H | H | |
| NH₂ | H | H | OMe | H | H | |
| NH₂ | OMe | H | OMe | H | H | |
| NH₂ | OMe | H | OMe | H | OMe | |
| NH₂ | Me | H | Me | H | H | |
| NH₂ | Me | H | Me | H | Me | |
| NHEt | H | H | H | H | H | |
| NHEt | H | H | OMe | H | H | |
| NHEt | H | H | Me | H | H | |
| NHEt | OMe | H | H | H | H | |
| NHEt | Me | H | H | H | H | |
| NHEt | OMe | H | OMe | H | H | |
| NHEt | OMe | H | OMe | H | OMe | |
| NHEt | Me | H | Me | H | H | |
| NHEt | Me | H | Me | H | Me | |
| SH | H | H | H | H | H | |
| Cl | H | H | H | H | H | |
| Br | H | H | H | H | H | |

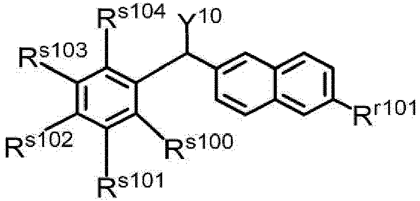

| Y¹⁰ | R^{s100} | R^{s101} | R^{s102} | R^{s103} | R^{s104} | R^{r101} |
|---|---|---|---|---|---|---|
| OH | H | H | H | H | H | |
| OH | H | H | OMe | H | H | |
| OH | H | H | Me | H | H | |
| OH | OMe | H | H | H | H | |
| OH | Me | H | H | H | H | |
| OH | OMe | H | OMe | H | H | |
| OH | OMe | H | OMe | H | OMe | |
| OH | Me | H | Me | H | H | |
| OH | Me | H | Me | H | Me | |
| OH | H | H | C₆H₁₃ | H | H | |
| OH | H | H | OC₁₂H₂₅ | H | H | |
| OH | H | H | OC₁₈H₃₇ | H | H | |
| OH | OMe | H | OC₁₈H₃₇ | H | H | |
| OH | OC₁₈H₃₇ | H | OC₁₈H₃₇ | H | H | |
| NH₂ | H | H | H | H | H | |
| NH₂ | H | H | OMe | H | H | OC₁₂H₂₄OC₂₂H ₄₅ |
| NH₂ | OMe | H | OMe | H | H | |
| NH₂ | OMe | H | OMe | H | OMe | |
| NH₂ | Me | H | Me | H | H | |
| NH₂ | Me | H | Me | H | Me | |
| NHEt | H | H | H | H | H | |
| NHEt | H | H | OMe | H | H | |
| NHEt | H | H | Me | H | H | |
| NHEt | OMe | H | H | H | H | |
| NHEt | Me | H | H | H | H | |
| NHEt | OMe | H | OMe | H | H | |
| NHEt | OMe | H | OMe | H | OMe | |
| NHEt | Me | H | Me | H | H | |
| NHEt | Me | H | Me | H | Me | |
| SH | H | H | H | H | H | |
| Cl | H | H | H | H | H | |
| Br | H | H | H | H | H | |

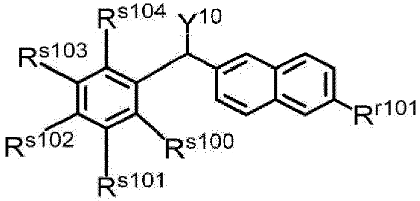

| Y¹⁰ | R^{s100} | R^{s101} | R^{s102} | R^{s103} | R^{s104} | R^{r101} |
|---|---|---|---|---|---|---|
| OH | H | OC₁₂H₂₅ | H | OC₁₂H₂₅ | H | OMe |
| OH | H | OC₁₈H₃₇ | H | OC₁₈H₃₇ | H | H |
| OH | H | OC₂₂H₄₅ | H | OC₂₂H₄₅ | H | OMe |
| OH | H | ^{OG}₁₂^{H}₂₅ | OC₁₂H₂₅ | OC₁₂H₂₅ | H | H |
| OH | H | OC₁₈H₃₇ | OC₁₈H₃₇ | OC₁₈H₃₇ | H | H |
| OH | H | OC₁₈H₃₇ | OC₁₈H₃₇ | OC₁₈H₃₇ | H | OMe |
| OH | H | OC₂₂H₄₅ | OC₂₂H₄₅ | OC₂₂H₄₅ | H | H |
| OH | H | OC₂₂H₄₅ | OC₂₂H₄₅ | OC₂₂H₄₅ | H | OMe |
| OH | OC₂₂H₄₅ | H | OC₂₂H₄₅ | H | H | H |
| OH | OC₂₂H₄₅ | H | OC₂₂H₄₅ | H | H | OMe |
| OH | OC₂₂H₄₅ | H | OC₂₂H₄₅ | H | H | OC₂₂H₄₅ |
| OH | OMe | H | OC₂₂H₄₅ | H | H | OC₂₂H₄₅ |
| OH | OMe | H | OC₂₂H₄₅ | H | OMe | H |
| OH | OMe | H | OC₂₂H₄₅ | H | OMe | OC₂₂H₄₅ |
| NH₂ | H | OC₁₂H₂₅ | H | OC₁₂H₂₅ | H | OMe |
| NH₂ | H | OC₁₈H₃₇ | H | OC₁₈H₃₇ | H | H |
| NH₂ | H | OC₁₈H₃₇ | OC₁₈H₃₇ | OC₁₈H₃₇ | H | OMe |
| NH₂ | H | OC₂₂H₄₅ | OC₂₂H₄₅ | OC₂₂H₄₅ | H | H |
| NH₂ | H | OC₂₂H₄₅ | OC₂₂H₄₅ | OC₂₂H₄₅ | H | OMe |
| NH₂ | OC₂₂H₄₅ | H | OC₂₂H₄₅ | H | H | H |
| NHEt | H | OC₁₂H₂₅ | H | OC₁₂H₂₅ | H | OMe |
| NHEt | H | OC₁₈H₃₇ | H | OC₁₈H₃₇ | H | H |
| NHEt | H | OC₂₂H₄₅ | H | OC₂₂H₄₅ | H | OMe |
| NHEt | H | OC₁₂H₂₅ | OC₁₂H₂₅ | OC₁₂H₂₅ | H | H |
| NHEt | H | OC₁₈H₃₇ | OC₁₈H₃₇ | OC₁₈H₃₇ | H | H |
| NHEt | H | OC₁₈H₃₇ | OC₁₈H₃₇ | OC₁₈H₃₇ | H | OMe |
| NHEt | H | OC₂₂H₄₅ | OC₂₂H₄₅ | OC₂₂H₄₅ | H | H |
| NHEt | H | OC₂₂H₄₅ | OC₂₂H₄₅ | OC₂₂H₄₅ | H | OMe |
| NHEt | OC₂₂H₄₅ | H | OC₂₂H₄₅ | H | H | H |
| SH | H | OC₁₂H₂₅ | H | OC₁₂H₂₅ | H | OMe |
| Cl | H | OC₁₂H₂₅ | H | OC₁₂H₂₅ | H | OMe |
| Br | H | OC₁₂H₂₅ | H | OC₁₂H₂₅ | H | OMe |

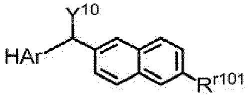

| HAr | Y¹⁰ | R^{r101} | | HAr | Y¹⁰ | R^{r101} |
|---|---|---|---|---|---|---|
| 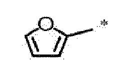 | OH | 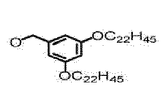 | | 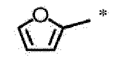 | NH₂ | 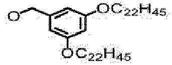 |
| 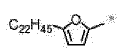 | OH | | | 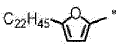 | NH₂ | |
| 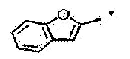 | OH | | | 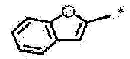 | NH₂ | |
| 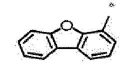 | OH | | | 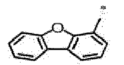 | NH₂ | |
| 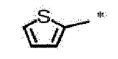 | OH | | | 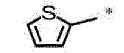 | NH₂ | |
| 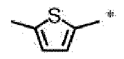 | OH | | | 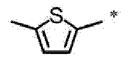 | NH₂ | |
| 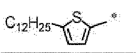 | OH | | | 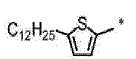 | NH₂ | |
| 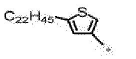 | OH | | | 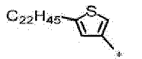 | NH_{z} | |
| 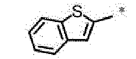 | OH | | | 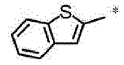 | NH₂ | |
| 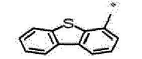 | OH | | | 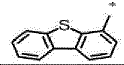 | NH₂ | |
| 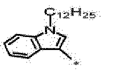 | OH | | | 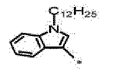 | NH₂ | |
| 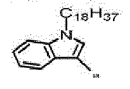 | OH | | | 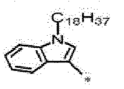 | NH₂ | |
| 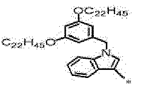 | OH | | | 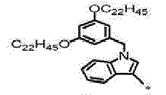 | NH₂ | |
| 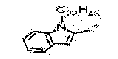 | OH | | | 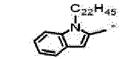 | NH₂ | |
| 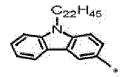 | OH | | | 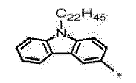 | NH₂ | |
| 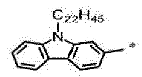 | OH | | | 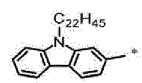 | NH₂ | |

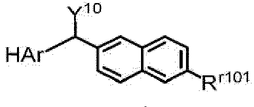

| HAr | Y¹⁰ | R^{r101} | | HAr | Y¹⁰ | R^{r101} |
|---|---|---|---|---|---|---|
| 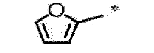 | NHEt | | | 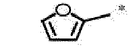 | SH | |
| 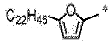 | NHEt | 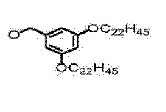 | | 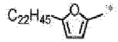 | SH | 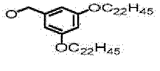 |
| 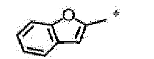 | NHEt | | | 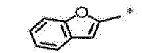 | SH | |
| 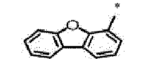 | NHEt | | | 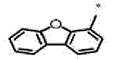 | SH | |
| 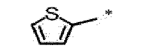 | NHEt | | | 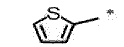 | SH | |
| 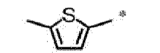 | NHEt | | | 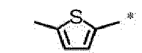 | SH | |
| 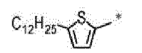 | NHEt | | | 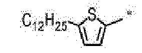 | SH | |
| 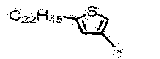 | NHEt | | | 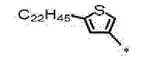 | SH | |
| 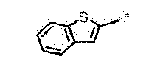 | NHEt | | | 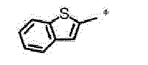 | SH | |
| 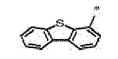 | NHEt | | | 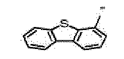 | SH | |
| 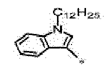 | NHEt | | | 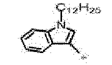 | SH | |
| 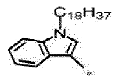 | NHEt | | | 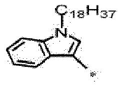 | SH | |
| 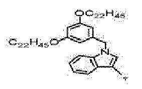 | NHEt | | | 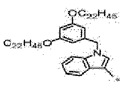 | SH | |
| 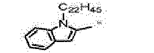 | NHEt | | | 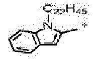 | SH | |
| 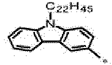 | NHEt | | | 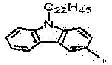 | SH | |
| 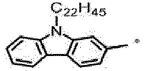 | NHEt | | | 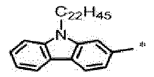 | SH | |

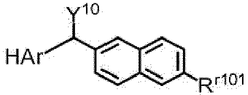

| HAr | Y¹⁰ | R^{r101} | | HAr | Y¹⁰ | R^{r101} |
|---|---|---|---|---|---|---|
| 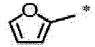 | Cl | | | 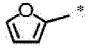 | Br | |
| 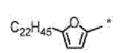 | Cl | 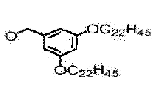 | | 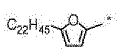 | Br | 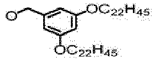 |
| 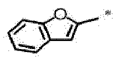 | Cl | | | 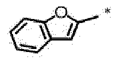 | Br | |
| 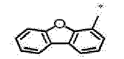 | Cl | | | 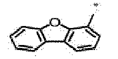 | Br | |
| 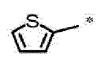 | Cl | | | 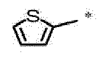 | Br | |
| 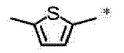 | Cl | | | 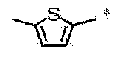 | Br | |
| 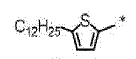 | Cl | | | 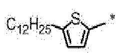 | Br | |
| 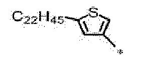 | Cl | | | 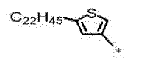 | Br | |
| 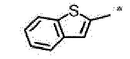 | Cl | | | 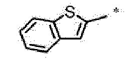 | Br | |
| 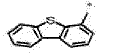 | Cl | | | 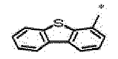 | Br | |
| 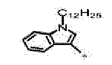 | Cl | | | 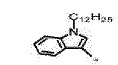 | Br | |
| 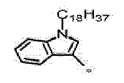 | Cl | | | 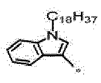 | Br | |
| 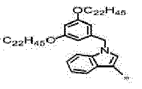 | Cl | | | 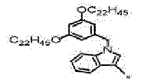 | Br | |
| 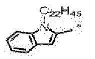 | Cl | | | 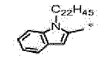 | Br | |
| 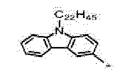 | Cl | | | 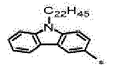 | Br | |
| 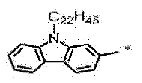 | Cl | | | 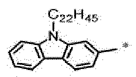 | Br | |

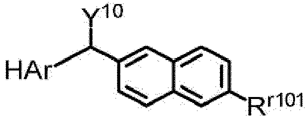

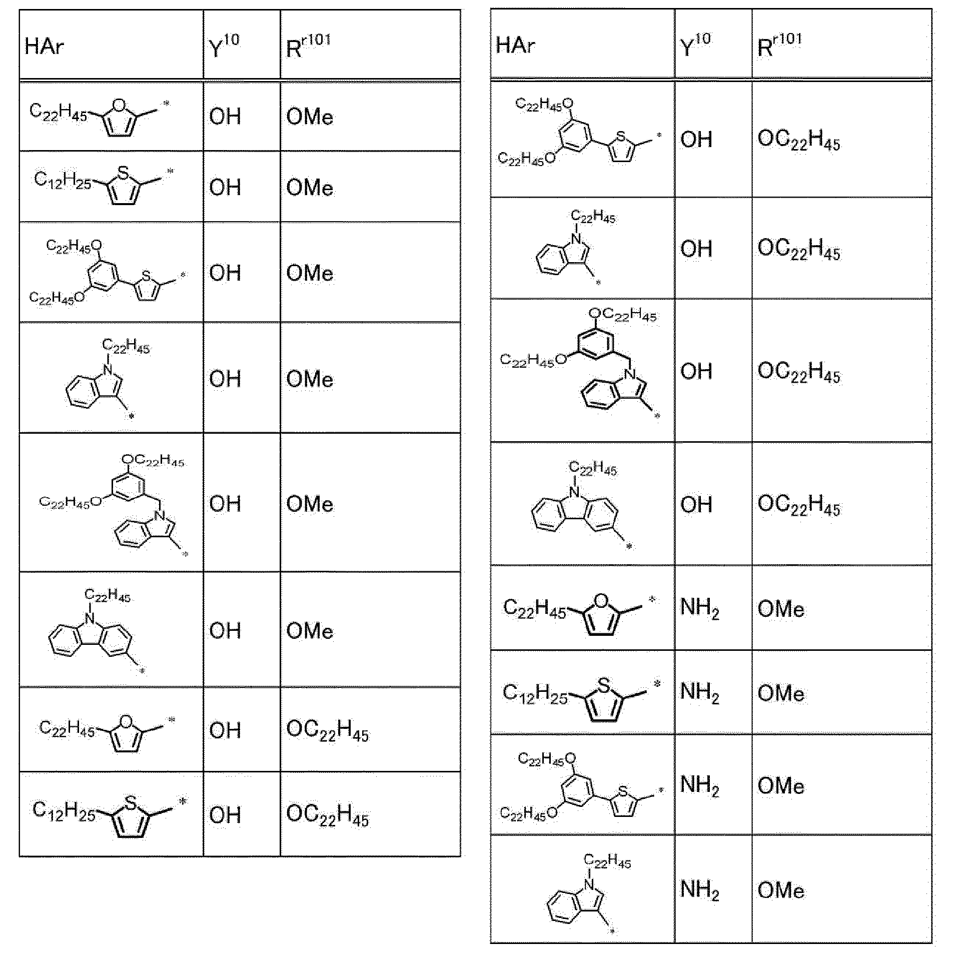

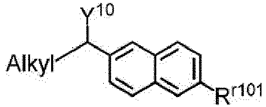

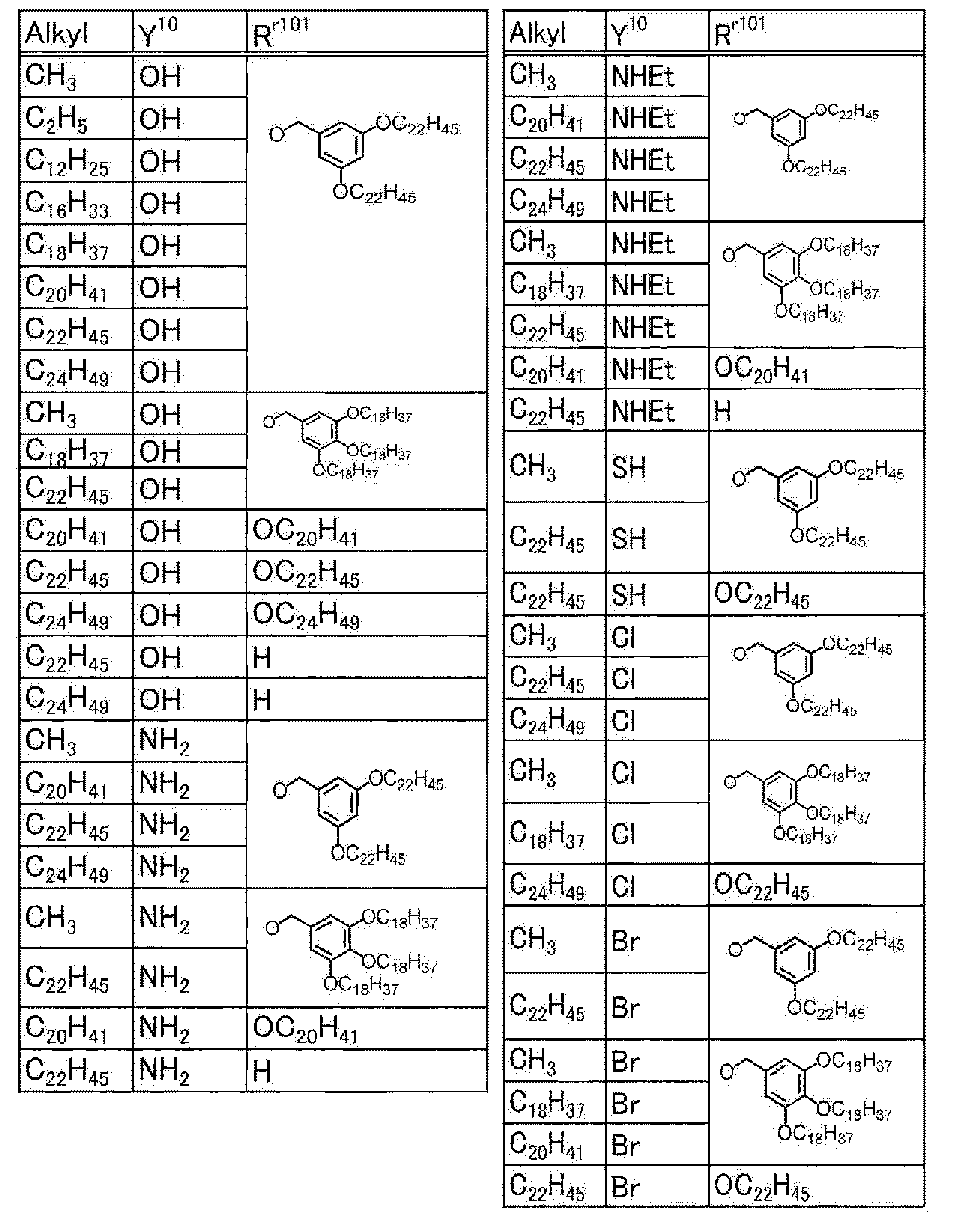

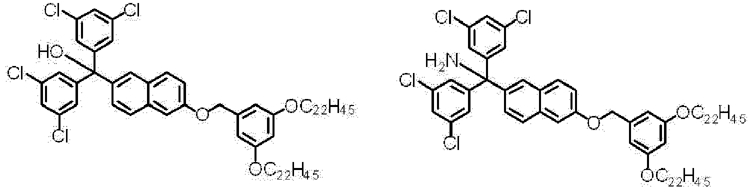

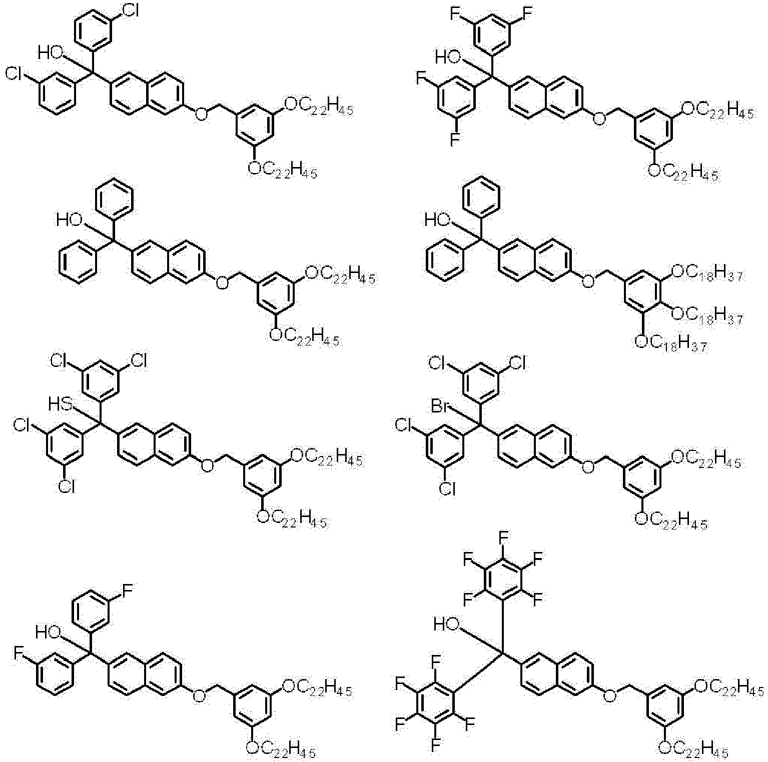

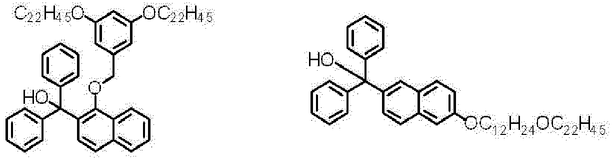

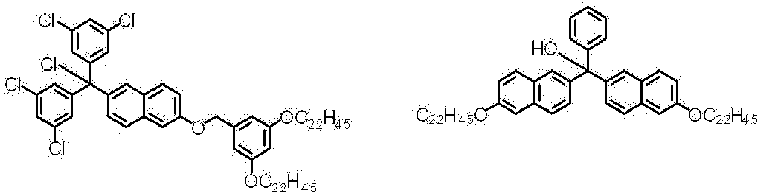

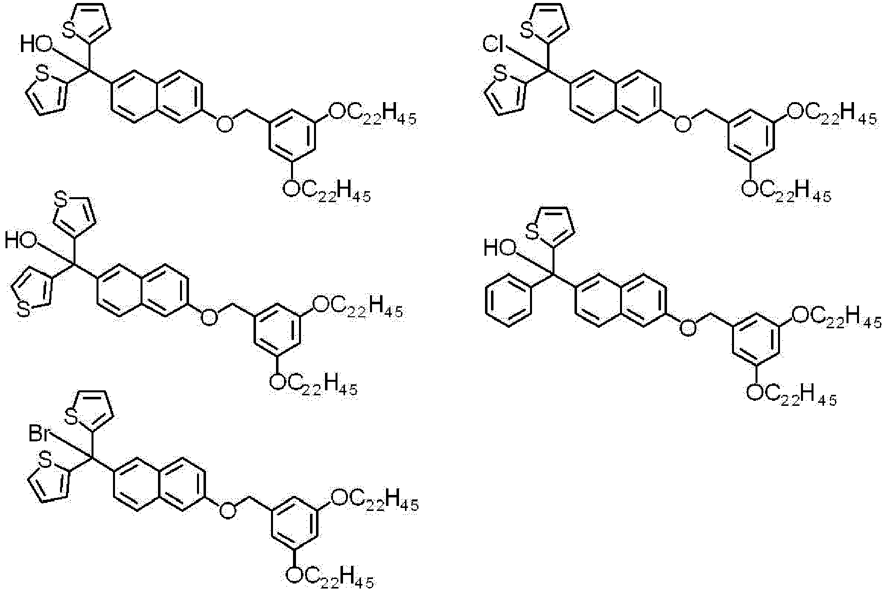

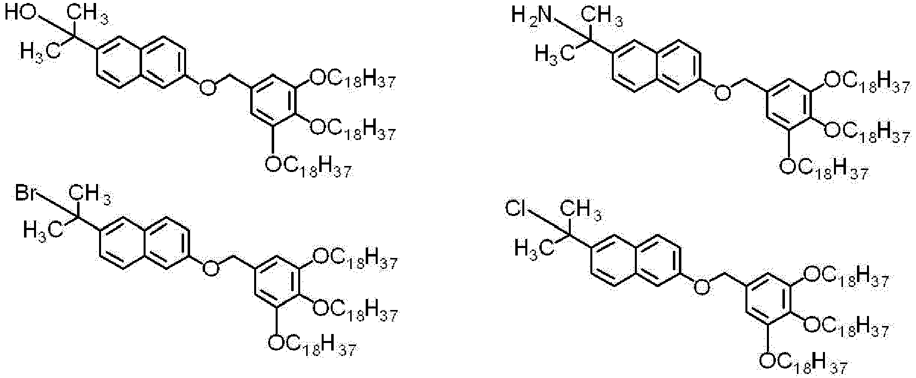

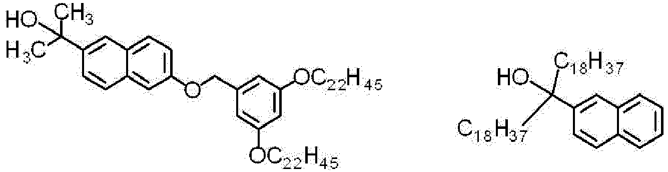

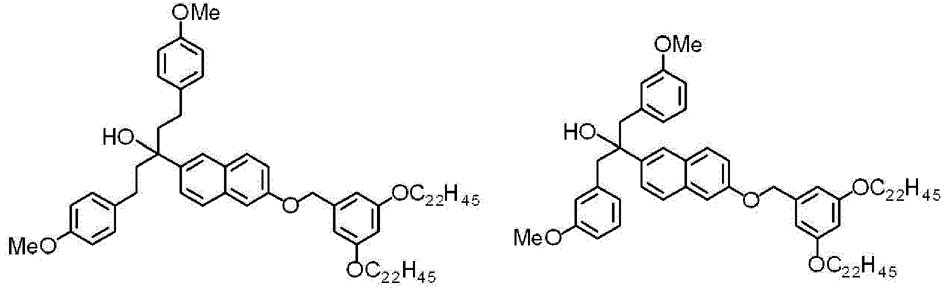

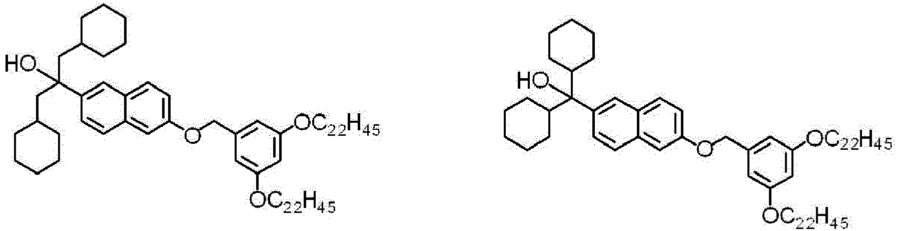

### <Method for producing condensed polycyclic aromatic hydrocarbon compound represented by Formula (1)>

A method for producing the condensed polycyclic aromatic hydrocarbon compound represented by Formula (1) is not particularly limited, and can be performed by referring to a known method.

Unless otherwise specified, a raw material compound used for producing the condensed polycyclic aromatic hydrocarbon compound represented by Formula (1) may be a commercially available compound, or may be produced by a known method or a method according to the known method.

In addition, the produced condensed polycyclic aromatic hydrocarbon compound represented by Formula (1) may be purified by a known purification method as necessary. For example, a method of isolating and purifying by recrystallization, column chromatography, or the like, a method of purifying by reprecipitation with a unit for changing the solution temperature, a unit for changing the solution composition, or the like, and the like can be performed.

A method for synthesizing the condensed polycyclic aromatic hydrocarbon compound represented by Formula (1) is not particularly limited, but for example, the method can be performed according to the following scheme. In addition, it is also possible to synthesize by referring to the synthesis method described in WO2010/113939A.

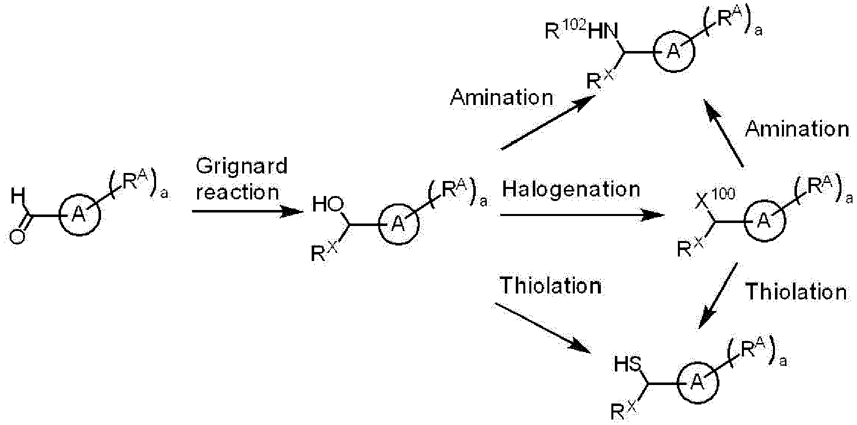

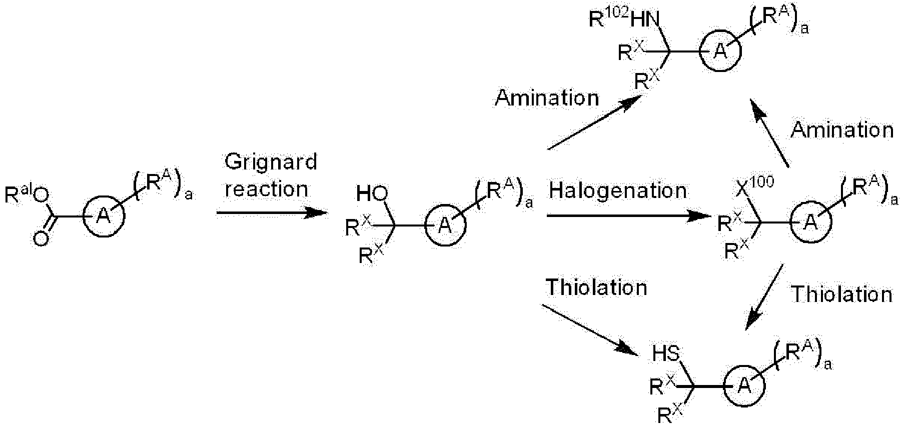

R^{al} represents an alkyl group, R^{X} represents an alkyl group, an aryl group, or a heteroaryl group, R¹⁰⁰ represents a hydrogen atom or OR¹⁰¹, R¹⁰¹ represents an alkyl group, X¹⁰⁰ represents Cl, Br, or I, and R¹⁰² represents a hydrogen atom, an alkyl group, or an Fmoc group.

### (Method for producing peptide compound)

In the method for producing a peptide compound according to the embodiment of the present disclosure, it is preferable that the step of using the condensed polycyclic aromatic hydrocarbon compound represented by Formula (1) is a C-terminal protecting step of protecting a carboxy group or an amide group of an amino acid compound or a peptide compound with the condensed polycyclic aromatic hydrocarbon compound represented by Formula (1).

In addition, from the viewpoint of ease of synthesizing the peptide compound and yield, it is more preferable that the method for producing a peptide compound according to the embodiment of the present disclosure further includes, in addition to the above-described C-terminal protecting step of protecting a carboxy group or an amide group of an amino acid compound or a peptide compound with the condensed polycyclic aromatic hydrocarbon compound represented by Formula (1), an N-terminal deprotecting step of deprotecting an N-terminal end of an N-terminal and C-terminal protected amino acid compound or an N-terminal and C-terminal protected peptide compound, which is obtained in the C-terminal protecting step, and a peptide chain extending step of condensing the N-terminal end of a C-terminal protected amino acid compound or a C-terminal protected peptide compound, which is obtained in the N-terminal deprotecting step, with an N-terminal protected amino acid compound or an N-terminal protected peptide compound; it is still more preferable that the method for producing a peptide compound according to the embodiment of the present disclosure further includes, in addition to the above steps, a precipitating step of precipitating an N-terminal and C-terminal protected peptide compound obtained in the peptide chain extending step; and it is particularly preferable that the method for producing a peptide compound according to the embodiment of the present disclosure further includes, one or more times in the following order after the precipitating step, a step of deprotecting the N-terminal end of the obtained N-terminal and C-terminal protected peptide compound, a step of condensing the N-terminal end of the obtained C-terminal protected peptide compound with an N-terminal protected amino acid compound or an N-terminal protected peptide compound, and a step of precipitating the obtained N-terminal and C-terminal protected peptide compound.

In addition, it is preferable that the method for producing a peptide compound according to the embodiment of the present disclosure further includes a C-terminal deprotecting step of deprotecting a C-terminal protective group.

Furthermore, it is preferable that the method for producing a peptide compound according to the embodiment of the present disclosure further includes, before the above-described C-terminal protecting step, a dissolving step of dissolving the condensed polycyclic aromatic hydrocarbon compound represented by Formula (1) in a solvent.

Hereinafter, each step and the like described above will be described in detail.

### <Dissolving step>

It is preferable that the method for producing a peptide compound according to the embodiment of the present disclosure includes, before the above-described C-terminal protecting step, a dissolving step of dissolving the condensed polycyclic aromatic hydrocarbon compound represented by Formula (1) in a solvent.

As the solvent, a general organic solvent can be used for the reaction, but since excellent reactivity can be expected as solubility in the above-described solvent is higher, it is preferable to select a solvent having a high solubility of the condensed polycyclic aromatic hydrocarbon compound represented by Formula (1). Specific examples thereof include halogenated hydrocarbons such as chloroform and dichloromethane; and nonpolar organic solvents such as 1,4-dioxane, tetrahydrofuran, and cyclopentyl methyl ether. Two or more of these solvents may be mixed and used in an appropriate ratio. In addition, as long as the condensed polycyclic aromatic hydrocarbon compound represented by Formula (1) can be dissolved, in the above-described halogenated hydrocarbons or nonpolar organic solvents, aromatic hydrocarbons such as benzene, toluene, and xylene; nitriles such as acetonitrile and propionitrile; ketones such as acetone and 2-butanone; amides such as N,N-dimethylformamide and N-methylpyrrolidone; and sulfoxides such as dimethyl sulfoxide may be mixed and used in an appropriate ratio.

In addition, a solvent described in Organic Process Research & Development, 2017, 21, 3, 365 to 369 may be used.

### <C-terminal protecting step>

It is preferable that the method for producing a peptide compound according to the embodiment of the present disclosure includes a C-terminal protecting step of protecting a carboxy group or an amide group of an amino acid compound or a peptide compound with the condensed polycyclic aromatic hydrocarbon compound represented by Formula (1).

The amino acid compound or peptide compound used in the above-described C-terminal protecting step is not particularly limited, and a known compound can be used. However, an N-terminal protected amino acid compound or an N-terminal protected peptide compound is preferable, and an Fmoc-protected amino acid compound or an Fmoc-protected peptide compound is more preferable.

In addition, it is preferable that a hydroxy group, an amino group, a carbonyl group, an amide group, an imidazole group, an indole group, a guanidyl group, a mercapto group, or the like, which is a moiety other than the C-terminal end in the amino acid compound or peptide compound used in the above-described C-terminal protecting step, is protected by a known protective group such as a protective group described later.

The amount of the amino acid compound or peptide compound, which is used as a reaction substrate, to be used is preferably 1 molar equivalent to 10 molar equivalent, more preferably 1 molar equivalent to 5 molar equivalent, still more preferably 1 molar equivalent to 2 molar equivalent, and particularly preferably 1 molar equivalent to 1.5 molar equivalent with respect to 1 molar equivalent of the condensed polycyclic aromatic hydrocarbon compound represented by Formula (1).

In a case where a condensed polycyclic aromatic hydrocarbon compound represented by Formula (1), in which Y^{A} in Formula (1) is -OH or -SH, is used, it is preferable to add a condensing agent in the presence of a condensation additive (condensation activating agent) in a solvent which does not affect the reaction, or to react in an acid catalyst.

In a case where a condensed polycyclic aromatic hydrocarbon compound represented by Formula (1), in which Y^{A} in Formula (1) is -NHR, is used, it is preferable to add a condensing agent in the presence of a condensation additive (condensation activating agent), or to react with a condensing agent and a base.

The amount of the condensation additive to be used is preferably 0.05 molar equivalent to 1.5 molar equivalent with respect to 1 molar equivalent of the condensed polycyclic aromatic hydrocarbon compound represented by Formula (1).

As the condensing agent, a condensing agent generally used in peptide synthesis can be used without limitation in the present disclosure. Examples thereof include 4-(4,6-dimethoxy-1,3,5-triazin-2-yl)-4-methylmorpholinium chloride (DMT-MM), O-(benzotriazol-1-yl)-1,1,3,3-tetramethyluronium hexafluorophosphate (HBTU), O-(7-azabenzotriazol-1-yl)-1,1,3,3-tetramethyluronium hexafluorophosphate (HATU), O-(6-chlorobenzotriazol-1-yl)-1,1,3,3 -tetramethyluronium hexafluorophosphate (HBTU(6-Cl)), O-(benzotriazol-1-yl)-1,1,3,3-tetramethyluronium tetrafluoroborate (TBTU), O-(6-chlorobenzotriazol-1-yl)-1,1,3,3 -tetramethyluronium tetrafluoroborate (TCTU), (1-cyano-2-ethoxy-2-oxoethylidenaminooxy)dimethylamino-morpholino-carbenium hexafluorophosphate (COMU), dicyclohexylcarbodiimide (DCC), diisopropylcarbodiimide (DIC), 1-ethyl-3-(3-dimethylaminopropyl) carbodiimide (EDC), a hydrochloride salt (EDC/HC1) of 1-ethyl-3-(3-dimethylaminopropyl) carbodiimide, and (benzotriazol-1-yloxy)tripyrrolidinophosphonium hexafluorophosphate (PyBoP), but the condensing agent is not limited thereto.

Among these, DIC, EDC, EDC/HCl, DMT-MM, HBTU, HATU, or COMU is preferable.

The amount of the condensing agent to be used is preferably 1 molar equivalent to 10 molar equivalent and more preferably 1 molar equivalent to 5 molar equivalent with respect to 1 molar equivalent of the condensed polycyclic aromatic hydrocarbon compound represented by Formula (1).

As the acid catalyst used in the condensation reaction, an acid catalyst generally used in peptide synthesis can be used without limitation, and examples thereof include methanesulfonic acid, trifluoromethanesulfonic acid, and p-toluenesulfonic acid.

Among these, methanesulfonic acid or p-toluenesulfonic acid is preferable.

The amount of the acid catalyst to be used is preferably more than 0 molar equivalent and 4.0 molar equivalent or less, more preferably 0.05 molar equivalent to 1.5 molar equivalent, and still more preferably 0.1 molar equivalent to 0.3 molar equivalent with respect to 1 molar equivalent of the condensed polycyclic aromatic hydrocarbon compound represented by Formula (1).

In the above-described C-terminal protecting step, it is preferable to add a condensation activating agent in order to promote the reaction and suppress side reactions such as racemization.

The condensation activating agent in the present disclosure is a reagent which, in a case of coexisting with the condensing agent, leads an amino acid to a corresponding active ester, symmetric acid anhydride, or the like to facilitate the formation of a peptide bond (amide bond).

As the condensation activating agent, a condensation activating agent generally used in peptide synthesis can be used without limitation, and examples thereof include 4-dimethylaminopyridine, N-methylimidazole, boronic acid derivative, 1-hydroxybenzotriazole (HOBt), ethyl 1-hydroxytriazole-4-carboxylate (HOCt), 1-hydroxy-7-azabenzotriazole (HOAt), 3-hydroxy-1,2,3-benzotriazin-4(3H)-one (HOOBt), N-hydroxysuccinimide (HOSu), N-hydroxyphthalimide (HOPht), N-hydroxy-5-norbornene-2,3-dicarboxyimide (HONb), pentafluorophenol, and ethyl(hydroxyimino)cyanoacetylate (Oxyma). Among these, 4-dimethylaminopyridine, HOBt, HOCt, HOAt, HOOBt, HOSu, HONb, or Oxyma is preferable.

The amount of the activating agent to be used is preferably more than 0 molar equivalent and 4.0 molar equivalent or less and more preferably 0.1 molar equivalent to 1.5 molar equivalent with respect to the amino acid compound or peptide compound.

As the base, a base generally used in peptide synthesis can be used without limitation, and examples thereof include a tertiary amine such as diisopropylethylamine.

As the solvent, the above-described solvent used in the above-described dissolving step can be suitably used.

The reaction temperature is not particularly limited, but is preferably -10°C to 80°C and more preferably 0°C to 40°C. The reaction time is not particularly limited, but is preferably 1 hour to 30 hours.

To confirm the progress of the reaction, a method same as that of a general liquid phase organic synthesis reaction can be applied. That is, the reaction can be traced using thin-layer silica gel chromatography, high performance liquid chromatography, NMR, or the like.

In addition, the N-terminal and C-terminal protected amino acid compound or N-terminal and C-terminal protected peptide compound obtained by the above-described C-terminal protecting step may be purified.

For example, in order to isolate the product obtained after dissolving the obtained N-terminal and C-terminal protected amino acid compound or N-terminal and C-terminal protected peptide compound in a solvent and performing a desired organic synthesis reaction, it is preferable to perform a method of changing the solvent to a solvent in which the N-terminal and C-terminal protected amino acid compound or N-terminal and C-terminal protected peptide compound is dissolved (for example, change of solvent composition or change of solvent type) and reprecipitating the resultant.

Specifically, for example, the reaction is performed under conditions such that the N-terminal and C-terminal protected amino acid compound or N-terminal and C-terminal protected peptide compound is dissolved, and after the reaction, the solvent is distilled off and then replaced, or after the reaction, by adding a polar solvent to the reaction system without distilling off the solvent, aggregates are precipitated and impurities are eliminated. As the solvent for replacement, polar organic solvents such as methanol, acetonitrile, and water are used alone or in combination. That is, the reaction is performed under conditions such that the N-terminal and C-terminal protected amino acid compound or N-terminal and C-terminal protected peptide compound is dissolved, and in the solvent replacement after the reaction, for example, a halogenated solvent, THF, or the like is used for dissolution, and a polar organic solvent such as methanol, acetonitrile, and water is used for precipitation.

### <N-terminal deprotecting step>

It is preferable that the method for producing a peptide compound according to the embodiment of the present disclosure includes an N-terminal deprotecting step of deprotecting the N-terminal end of the N-terminal and C-terminal protected amino acid compound or N-terminal and C-terminal protected peptide compound, which is obtained in the C-terminal protecting step.

As the N-terminal protective group, a protective group for an amino group described later, which is generally used in technical fields such as peptide chemistry, can be used, but in the present disclosure, a Boc group, a benzyloxycarbonyl group (hereinafter, also referred to as a Cbz group or a Z group), or an Fmoc group is suitably used.

The deprotection condition is appropriately selected depending on the type of the temporary protective group, but a group which can be deprotected under conditions different from the removal of the protective group derived from the condensed polycyclic aromatic hydrocarbon compound represented by Formula (1) is preferable. For example, in a case of the Fmoc group, the deprotection is performed by treating with a base, and in a case of the Boc group, the deprotection is performed by treating with an acid. The reaction is performed in a solvent which does not affect the reaction.

Examples of the base include secondary amines such as dimethylamine and diethylamine, and non-nucleophilic organic bases such as 1,8-diazabicyclo[5.4.0]-7-undecene (DBU), 1,4-diazabicyclo[2.2.2]octane (DABCO), and 1,5-diazabicyclo[4.3.0]-5-nonene (DBN).

As the solvent, the above-described solvent used in the above-described dissolving step can be suitably used.

### <Peptide chain extending step>

It is preferable that the method for producing a peptide compound according to the embodiment of the present disclosure includes a peptide chain extending step of condensing the N-terminal end of a C-terminal protected amino acid compound or a C-terminal protected peptide compound, which is obtained in the N-terminal deprotecting step, with an N-terminal protected amino acid compound or an N-terminal protected peptide compound.

The above-described peptide chain extending step is preferably performed under peptide synthesis conditions generally used in the field of peptide chemistry, in which the above-described condensing agent, condensation additive, and the like are used.

The N-terminal protected amino acid compound or N-terminal protected peptide compound is not particularly limited, and a desired compound can be used. However, an Fmoc-protected amino acid compound or an Fmoc-protected peptide compound can be suitably used.

In addition, it is preferable that a hydroxy group, an amino group, a carbonyl group, an amide group, an imidazole group, an indole group, a guanidyl group, a mercapto group, or the like, which is a moiety other than the C-terminal end in the N-terminal protected amino acid compound or N-terminal protected peptide compound, is protected by a known protective group such as a protective group described later.

### <Precipitating step>

It is preferable that the method for producing a peptide compound according to the embodiment of the present disclosure further includes a precipitating step of precipitating the N-terminal and C-terminal protected peptide compound obtained in the peptide chain extending step.

The above-described precipitating step can be performed in the same manner as the precipitation method in the purification which may be performed after the above-described C-terminal protecting step.

### <Chain extension>

It is preferable that the method for producing a peptide compound according to the embodiment of the present disclosure further includes, one or more times in the following order after the precipitating step, a step of deprotecting the N-terminal end of the obtained N-terminal and C-terminal protected peptide compound, a step of condensing the N-terminal end of the obtained C-terminal protected peptide compound with an N-terminal protected amino acid compound or an N-terminal protected peptide compound, and a step of precipitating the obtained N-terminal and C-terminal protected peptide compound.

By repeating the above-described three steps, the chain extension of the obtained peptide compound can be easily performed.

Each step in the above-described three steps can be performed in the same manner as each corresponding step described above.

### <C-terminal deprotecting step>

It is preferable that the method for producing a peptide compound according to the embodiment of the present disclosure further includes a C-terminal deprotecting step of deprotecting a C-terminal protective group.

In the above-described C-terminal deprotecting step, by removing the C-terminal protective group formed by the condensed polycyclic aromatic hydrocarbon compound represented by Formula (1) in the C-terminal protected peptide compound having a desired number of amino acid residues, a peptide compound, which is the final target product, can be obtained.

Preferred examples of a method of removing the C-terminal protective group include a deprotecting method using an acidic compound.

Examples thereof include a method of adding an acid catalyst and a hydrogenating method using a metal catalyst. Examples of the acid catalyst include trifluoroacetic acid (TFA), hydrochloric acid, trifluoroethanol (TFE), hexafluoroisopropanol (HFIP), and acetic acid, and TFA is preferable for peptides which do not decompose with strong acids, and TFE, HFIP, or acetic acid is preferable for peptides which decompose with strong acids. The concentration of the acid can be appropriately selected depending on the side chain protective group of the extending amino acid and the deprotection conditions, and examples thereof include 1% by mass to 100% by mass with respect to the total mass of the solvent used.

In addition, the concentration of TFA is preferably 70% by mass or less, more preferably 50% by mass or less, still more preferably 30% by mass or less, and particularly preferably 10% by mass or less.

The concentration of TFA is preferably 10% by volume or less, more preferably 5% by volume or less, and particularly preferably 1% by volume or less with respect to the total volume of the solvent used. The lower limit value is preferably 0.01% by volume, more preferably 0.1% by volume, and still more preferably 0.5% by volume.

The deprotection time is preferably 5 hours or less, more preferably 3 hours or less, and still more preferably 1 hour or less.

In the present disclosure, the C-terminal protective group can be deprotected even under weak acid conditions, and a side reaction of the obtained peptide can be suppressed.

The peptide compound, which is the final target product obtained by the method for producing a peptide compound according to the embodiment of the present disclosure, can be isolated and purified according to a method commonly used in peptide chemistry. For example, the peptide compound, which is the final target product, can be isolated and purified by extraction and washing the reaction mixture, crystallization, chromatography, and the like.

The type of peptide produced by the method for producing a peptide compound according to the embodiment of the present disclosure is not particularly limited, but it is preferable that the number of amino acid residues of the peptide compound is, for example, approximately several tens or less. Same as existing or unknown synthetic or native peptides, the peptide obtained by the method for producing a peptide compound according to the embodiment of the present disclosure can be used in various fields such as pharmaceuticals, foods, cosmetics, electronic materials, biosensors, and the like, but the use of the peptide is not limited thereto.

In the method for producing a peptide compound according to the embodiment of the present disclosure, the precipitating step can be appropriately omitted as long as it does not affect the reaction in the next step.

In a case where the amino acid compound or peptide compound used in the method for producing a peptide compound according to the embodiment of the present disclosure has a hydroxy group, an amino group, a carboxy group, a carbonyl group, a guanidyl group, a mercapto group, or the like, a protective group generally used in peptide chemistry or the like may be introduced into these groups, and the target compound can be obtained by removing the protective group as necessary after the reaction.

Examples of a protective group of the hydroxy group include an alkyl group having 1 to 6 carbon atoms, an aryl group, a trityl group, an aralkyl group having 7 to 10 carbon atoms, a formyl group, an acyl group having 1 to 6 carbon atoms, a benzoyl group, an aralkyl-carbonyl group having 7 to 10 carbon atoms, a 2-tetrahydropyranyl group, a 2-tetrahydrofuranyl group, a silyl group, and an alkenyl group having 2 to 6 carbon atoms. These groups may be substituted with one to three substituents selected from the group consisting of a halogen atom, an alkoxy group having 1 to 6 carbon atoms (for example, methoxy, ethoxy, and propoxy), and a nitro group.

Examples of a protective group of the amino group include a formyl group, an acyl group having 1 to 6 carbon atoms, an alkoxycarbonyl group having 1 to 6 carbon atoms, a benzoyl group, an aralkyl-carbonyl group having 7 to 10 carbon atoms, an aralkyloxycarbonyl group having 7 to 14 carbon atoms, a trityl group, a monomethoxytrityl group, a 1-(4,4-Dimethyl-2,6-dioxocyclohex-1-ylidene)-3-methylbutyl group, a phtaloyl group, an N,N-dimethylaminomethylene group, a silyl group, and an alkenyl group having 2 to 6 carbon atoms. These groups may be substituted with one to three substituents selected from the group consisting of a halogen atom, an alkoxy group having 1 to 6 carbon atoms, and a nitro group.

Examples of a protective group of the carboxy group include the above-described protective group of the hydroxy group and a trityl group.

Examples of a protective group of the carbonyl group include cyclic acetals (for example, 1,3-dioxane) and acyclic acetals (for example, di(alkyl having 1 to 6 carbon atoms) acetal).

Examples of a protective group of the guanidyl group include a 2,2,4,6,7-pentamethyldihydrobenzofuran-5-sulfonyl group, a 2,3,4,5,6-pentamethylbenzenesulfonyl group, a tosyl group, and a nitro group.

Examples of a protective group of the mercapto group (sulfhydryl group) include a trityl group, a 4-methylbenzyl group, an acetylaminomethyl group, a t-butyl group, and a t-butylthio group.

The method of removing these protective groups may be performed according to a known method described in, for example, Protective Groups in Organic Synthesis, John Wiley and Sons (1980). For example, a method using acid, base, ultraviolet light, hydrazine, phenylhydrazine, sodium N-methyldithiocarbamate, tetrabutylammonium fluoride, palladium acetate, trialkylsilyl halide, or the like, a reduction method, and the like are used.

### (Protective group-forming reagent)

The protective group-forming reagent according to the embodiment of the present disclosure includes a condensed polycyclic aromatic hydrocarbon compound represented by Formula (1).

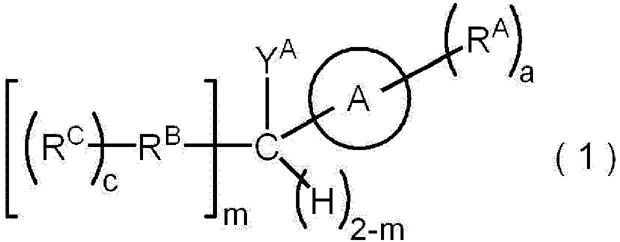

In Formula (1),
a ring A represents a condensed polycyclic aromatic hydrocarbon ring,
Y^{A} represents -OH, -NHR, SH, or -X⁰, where R represents a hydrogen atom, an alkyl group, an aromatic group-substituted alkyl group, a heteroaromatic group-substituted alkyl group, or a 9-fluorenylmethoxycarbonyl group (Fmoc group), and X⁰ represents Cl, Br, or I,
R^{A}'s each independently represent an aliphatic hydrocarbon group or an organic group having an aliphatic hydrocarbon group,
R^{B}'s each independently represent a monovalent aliphatic hydrocarbon group, a (1+c)-valent aromatic group, or a (1+c)-valent heteroaromatic group,
R^{C}'s each independently represent an aliphatic hydrocarbon group or an organic group having an aliphatic hydrocarbon group,
m represents 1 or 2, a represents an integer of 0 to 5, and c represents an integer of 0 to 5,
in a case where both a and c are 0, R^{B} is a monovalent aliphatic hydrocarbon group, and
the number of carbon atoms in at least one of the aliphatic hydrocarbon group (of R^{A}, R^{B}, and R^{C}) is 12 or more.

The protective group-forming reagent according to the embodiment of the present disclosure is preferably a protective group-forming reagent of a carboxy group or an amide group, and more preferably a C-terminal protective group-forming reagent of an amino acid compound or a peptide compound.

A preferred aspect of the condensed polycyclic aromatic hydrocarbon compound represented by Formula (1) in the protective group-forming reagent according to the embodiment of the present disclosure is the same as the above-described preferred aspect of the condensed polycyclic aromatic hydrocarbon compound represented by Formula (1) according to the present disclosure.

The protective group-forming reagent according to the embodiment of the present disclosure may be a solid reagent or a liquid reagent.

The content of the condensed polycyclic aromatic hydrocarbon compound represented by Formula (1) in the protective group-forming reagent according to the embodiment of the present disclosure is not particularly limited, but is preferably 0.1% by mass to 100% by mass, more preferably 1% by mass to 100% by mass, and still more preferably 3% by mass to 100% by mass with respect to the total mass of the protective group-forming reagent.

The protective group-forming reagent according to the embodiment of the present disclosure may include a component other than the condensed polycyclic aromatic hydrocarbon compound represented by Formula (1).

As the other components, a known component can be included. Examples thereof include water, an organic solvent, an antioxidant, and a pH adjuster.

### (Condensed polycyclic aromatic hydrocarbon compound represented by Formula (1a))

The compound according to the embodiment of the present disclosure is a condensed polycyclic aromatic hydrocarbon compound represented by Formula (1a).

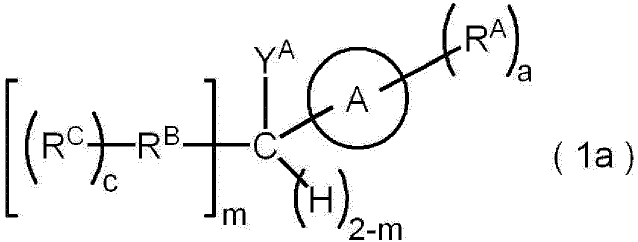

In Formula (1a),
a ring A represents a condensed polycyclic aromatic hydrocarbon ring,
Y^{A} represents -OH, -NHR, -SH, or -X⁰, where R represents a hydrogen atom, an alkyl group having 10 or less carbon atoms, an aromatic group-substituted alkyl group, a heteroaromatic group-substituted alkyl group, or a 9-fluorenylmethoxycarbonyl group (Fmoc group), and X⁰ represents Cl, Br, or I,
R^{A}'s each independently represent an aliphatic hydrocarbon group or an organic group having an aliphatic hydrocarbon group,
R^{B}'s each independently represent a monovalent aliphatic hydrocarbon group, a (1+c)-valent aromatic group, or a (1+c)-valent heteroaromatic group,
R^{C}'s each independently represent an aliphatic hydrocarbon group or an organic group having an aliphatic hydrocarbon group,
m represents 1 or 2, a represents an integer of 0 to 5, and c represents an integer of 0 to 5,
in a case where both a and c are 0, R^{B} is a monovalent aliphatic hydrocarbon group,
the number of carbon atoms in at least one of the aliphatic hydrocarbon group of R^{A}, R^{B}, and R^{C} is 18 or more, and
in a case where R^{B} is a monovalent aliphatic hydrocarbon group, R^{B} includes a linear saturated aliphatic hydrocarbon group having 18 or more carbon atoms.

The condensed polycyclic aromatic hydrocarbon compound represented by Formula (1a), which is the compound according to the embodiment of the present disclosure, is a novel compound and can be suitably used for producing a peptide compound. Among these, the compound according to the embodiment of the present disclosure can be suitably used as a protective group-forming reagent, more suitably used as a protective group-forming reagent of a carboxy group or an amide group, and particularly suitably used as a C-terminal protective group-forming reagent of an amino acid compound or a peptide compound.

The condensed polycyclic aromatic hydrocarbon compound represented by Formula (1a) in the compound according to the embodiment of the present disclosure is the same as the condensed polycyclic aromatic hydrocarbon compound represented by Formula (1) in the method for producing a peptide compound according to the embodiment of the present disclosure, except that, in a case where at least one of R^{A}, R^{C}, or R^{B} is an alkyl group, the number of carbon atoms in at least one aliphatic hydrocarbon group included in at least one R^{A} is 18 or more. In addition, the same applies to preferred aspects other than the preferred aspect described later.

In the condensed polycyclic aromatic hydrocarbon compound represented by Formula (1a), in a case where at least one of R^{A}, R^{C}, or R^{B} is an alkyl group, the number of carbon atoms in at least one aliphatic hydrocarbon group included in R^{B} or R^{C} is 18 or more. In addition, from the viewpoint of solubility in a solvent, crystallization property, and yield, in the at least one R^{A}, it is preferable that the number of carbon atoms in at least one aliphatic hydrocarbon group included in R^{B} or R^{C} is 18 to 100, it is more preferable that the number of carbon atoms in at least one aliphatic hydrocarbon group included in R^{B} or R^{C} is 18 to 40, and it is still more preferable that the number of carbon atoms in at least one aliphatic hydrocarbon group included in R^{B} or R^{C} is 20 to 36.

From the viewpoint of deprotection rate, crystallization property, solubility in a solvent, and yield, the condensed polycyclic aromatic hydrocarbon compound represented by Formula (1a) is preferably a compound represented by Formula (10a) or Formula (20a), and more preferably a compound represented by Formula (10a).

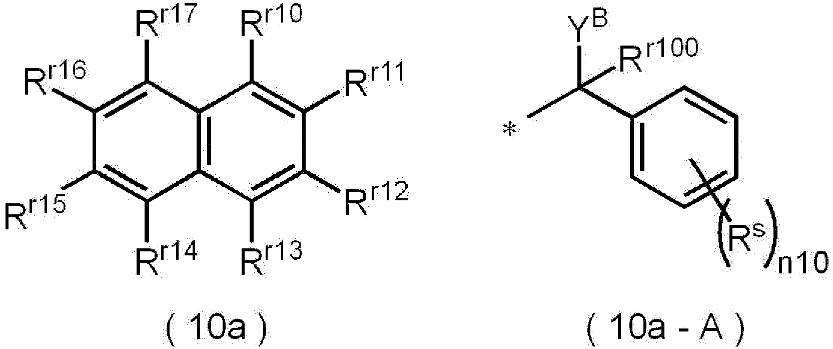

In Formula (10a) and Formula (10a-A),
Y^{B} represents -OH, -NHR, -SH, or -X⁰, where R represents a hydrogen atom, an alkyl group having 10 or less carbon atoms, an aromatic group-substituted alkyl group, a heteroaromatic group-substituted alkyl group, or a 9-fluorenylmethoxycarbonyl group (Fmoc group), and X⁰ represents Cl, Br, or I,
R^{r100} represents a hydrogen atom, an aryl group, or a heteroaryl group,
R^{s}'s each independently represent a substituent or R^{A},
n10 represents an integer of 0 to 5,
adjacent R^{s}'s may be linked to each other through a substituent to form a ring,
R^{r10} and R^{r11} each independently represent a hydrogen atom, a substituent, a group represented by Formula (10a-A), or R^{A},
any one of R^{r10} or R^{r11} is a group represented by Formula (10a-A),
^{∗} represents a linking position with R^{r10} or R^{r11},
R^{r12} to R^{r17} each independently represent a hydrogen atom, a substituent, or R^{A}, at least one of R^{s}, R^{r10}, ... , or R^{r17} is R^{A},
R^{A}'s each independently represent an aliphatic hydrocarbon group or an organic group having an aliphatic hydrocarbon group, and
the number of carbon atoms in at least one of the aliphatic hydrocarbon group included in at least one R^{A} is 18 or more.

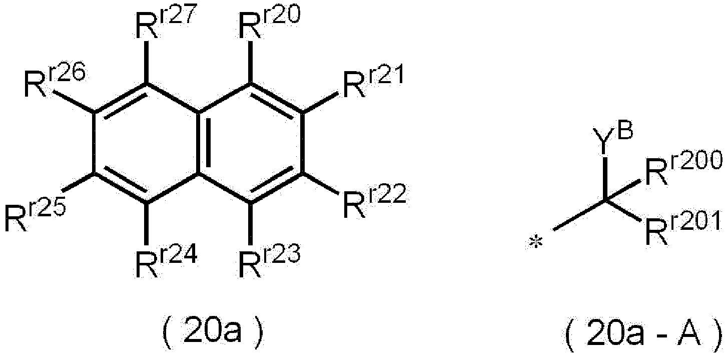

In Formula (20a) and Formula (20a-A),
Y^{B} represents -OH, -NHR, -SH, or -X⁰, where R represents a hydrogen atom, an alkyl group having 10 or less carbon atoms, an aromatic group-substituted alkyl group, a heteroaromatic group-substituted alkyl group, or a 9-fluorenylmethoxycarbonyl group (Fmoc group), and X⁰ represents Cl, Br, or I,
R^{r200} represents a hydrogen atom, an alkyl group, an aryl group, or a heteroaryl group,
R^{r201} represents an aliphatic hydrocarbon group,
R^{r20} and R^{r21} each independently represent a hydrogen atom, a substituent, a group represented by Formula (20a-A), or R^{A},
any one of R^{r20} or R^{r21} is a group represented by Formula (20a-A),
^{∗} represents a linking position with R^{r20} or R^{r21},
R^{r22} to R^{r27} each independently represent a hydrogen atom, a substituent, or R^{A}, and
in a case where R^{B} is an aliphatic hydrocarbon group, R^{B} includes a linear saturated aliphatic hydrocarbon group having 18 or more carbon atoms, or at least one of R^{r20}, ... , or R^{r27} is R^{A}, where R^{A}'s each independently represent an aliphatic hydrocarbon group or an organic group having an aliphatic hydrocarbon group, and the number of carbon atoms in at least one of the aliphatic hydrocarbon group included in at least one R^{A} is 18 or more.

The compound represented by Formula (10a) is the same as the compound represented by Formula (10) in the above-described method for producing a peptide compound according to the embodiment of the present disclosure, except that the number of carbon atoms in at least one aliphatic hydrocarbon group included in at least one R^{A} is 18 or more. In addition, the same applies to preferred aspects other than the preferred aspect described later.

The compound represented by Formula (20a) is the same as the compound represented by Formula (20) in the above-described method for producing a peptide compound according to the embodiment of the present disclosure, except that the number of carbon atoms in at least one aliphatic hydrocarbon group included in at least one R^{A} is 18 or more. In addition, the same applies to preferred aspects other than the preferred aspect described later.

R^{A} in the compound represented by any of Formula (10a) or Formula (20a) has the same meaning as R^{A} in the condensed polycyclic aromatic hydrocarbon compound represented by Formula (1a), and the preferred aspects thereof are also the same.

In addition, the condensed polycyclic aromatic hydrocarbon compound represented by Formula (1a) can be synthesized in the same manner as in the condensed polycyclic aromatic hydrocarbon compound represented by Formula (1).

### Examples

Hereinafter, the embodiments of the present invention will be more specifically described with reference to Examples. The materials, amounts to be used, proportions, treatment contents, treatment procedures, and the like shown in Examples can be appropriately modified as long as the modifications do not depart from the spirit of the embodiments of the present invention. Therefore, the scope of the embodiments of the present invention is not limited to the following specific examples. In addition, "parts" and "%" are on a mass basis unless otherwise specified.

Unless otherwise specified, purification by column chromatography was performed using an automatic purification device ISOLERA (manufactured by Biotage Ltd.) or a medium pressure liquid chromatograph YFLC-Wprep 2XY.N (manufactured by YAMAZEN).

Unless otherwise specified, SNAPKP-SI1 Cartridge (manufactured by Biotage Ltd.) or a high flash column W001, W002, W003, W004, or W005 (manufactured by YAMAZEN) was used as a carrier in silica gel column chromatography.

The mixing ratio in an eluent used for column chromatography is the volume ratio. For example, "gradient elution of hexane:ethyl acetate = 50:50 to 0:100" means that an eluent of 50% hexane and 50% ethyl acetate is finally changed to an eluent of 0% hexane and 100% ethyl acetate.

In addition, "gradient elution of hexane:ethyl acetate = 50:50 to 0:100 and gradient elution of methanol:ethyl acetate = 0:100 to 20:80" means that an eluent of 50% hexane and 50% ethyl acetate is changed to an eluent of 0% hexane and 100% ethyl acetate, and then the eluent of 0% hexane and 100% ethyl acetate is finally changed to an eluent of 20% methanol and 80% ethyl acetate.

MS spectrum was measured using ACQUITY SQD LC/MS System (manufactured by Waters Corporation; ionization method; electrospray ionization (ESI) method).

NMR spectrum was measured using Bruker AV300 (manufactured by Bruker, 300 MHz) or Bruker AV400 (manufactured by Bruker, 400 MHz), using tetramethylsilane as an internal reference, and all δ values were shown in ppm.

<Synthesis of protective group-forming reagent (compound (1-1))>

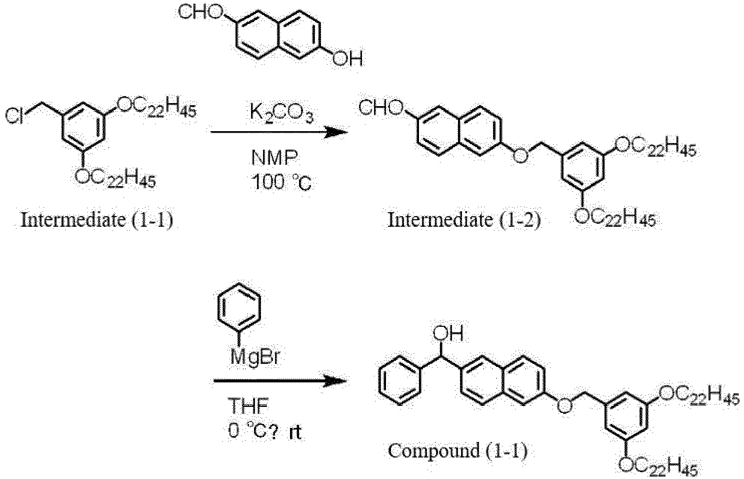

An intermediate (1-1) was synthesized according to the method described in EP2518041A.

The intermediate (1-1) (3.00 g, 3.87 mmol), 6-hydroxy-2-naphthaldehyde (1.33 g, 7.72 mmol), potassium carbonate (2.14 g, 15.5 mmol), and N-methylpyrrolidone (NMP, 40 mL) were mixed, and the mixture was stirred at 100°C for 6 hours under a nitrogen atmosphere. The reaction solution was cooled to room temperature (25°C, the same applies hereinafter) and extracted with cyclopentyl methyl ether and water. Methanol was added to the obtained organic layer to precipitate solid, and the solid was filtered and dried under reduced pressure to obtain an intermediate (1-2) (3.06 g, yield: 86.8%).

Under a nitrogen atmosphere, the intermediate (1-2) (912 mg, 1.00 mmol) was mixed with tetrahydrofuran (THF, 10 mL), the mixture was cooled to 0°C, and a phenylmagnesium bromide tetrahydrofuran solution(1.0 mol/L) (2.0 mL, 2.00 mmol) was added dropwise thereto. The reaction solution was heated to room temperature (rt) and stirred for 2 hours. After the mixture was further cooled to 0°C, water (0.5 mL) was gently added dropwise thereto, and then the mixture was extracted with cyclopentyl methyl ether and a saturated ammonium chloride aqueous solution. After separating, methanol was added to the obtained organic layer to precipitate solid, and the solid was filtered and dried to obtain a compound (1-1) (917 mg, yield: 92.6%).

¹H-NMR (CDCl₃, 400 MHz): δ = 0.88 (6H, t), 1.19 to 1.80 (80H, m), 2.27 (1H, d), 3.94 (4H, t), 5.09 (2H, s), 5.98 (1H, d), 6.41 (1H, t), 6.60 (2H, d), 7.17 (1H, d), 7.22 (1H, dd), 7.23 (1H, dd), 7.28 (1H, dd), 7.34 (2H, t), 7.39 (1H, d), 7.42 (2H, d), 7.67 (1H, d), 7.74 (1H, d), 7.81 (1H, s)

### <Synthesis of protective group-forming reagent (compound (1-2))>

The following compound (1-2) was obtained by synthesizing in the same manner as in the above-described compound (1-1).

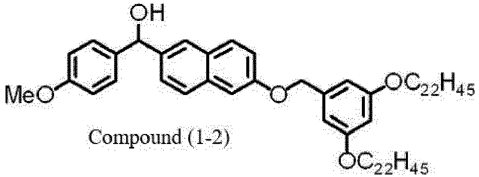

¹H-NMR (CDCl₃, 400 MHz): δ = 0.88 (6H, t), 1.19 to 1.80 (80H, m), 2.21 (1H, d), 3.79 (3H, s), 3.94 (4H, t), 5.08 (2H, s), 5.95 (1H, d), 6.41 (1H, t), 6.60 (2H, d), 6.87 (2H, d), 7.17 (1H, d), 7.22 (1H, dd), 7.32 (2H, d), 7.37 (1H, dd), 7.66 (1H, d), 7.73 (1H, d), 7.80 (1H, s)

The following compound (1-3) and compound (1-4) could be synthesized in the same manner as in the above-described compound (1-2).

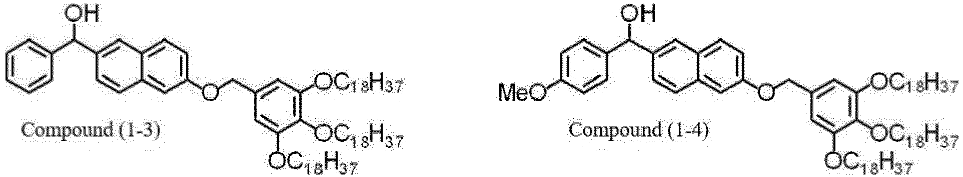

<Synthesis of protective group-forming reagent (compound (1-N-1))>

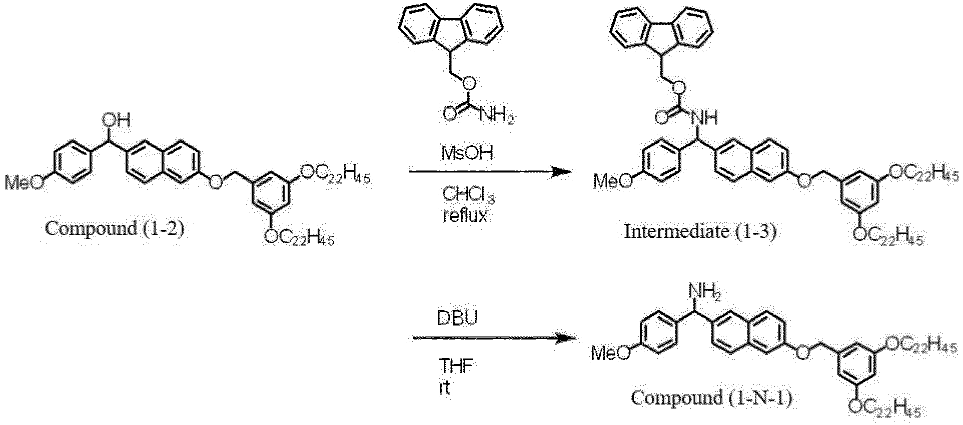

The compound (1-2) (1.02 g, 1.00 mmol), (9H-fluoren-9-ylmethoxy)carboxamide (1.20 g, 5.00 mmol), and chloroform (15 mL) were mixed, the mixture was heated to 50°C, and a solution prepared by diluting methanesulfonic acid (MsOH, 10.3 µL, 0.15 mmol) with chloroform (5 mL) was added dropwise to the reaction solution. The reaction solution was heated to reflux and stirred for 2 hours. The temperature was lowered to room temperature, the mixture was extracted with chloroform and a saturated sodium hydrogen carbonate aqueous solution, the obtained organic layer was dried over sodium sulfate and filtered, and the filtrate was concentrated under reduced pressure. Methanol was added to the obtained crude product to precipitate solid, and the solid was filtered and dried under reduced pressure to obtain an intermediate (1-3) (770 mg, yield: 62.0%).

The intermediate (1-3) (1.09 g, 0.806 mmol) was mixed with tetrahydrofuran (8 mL), diazabicycloundecene (DBU, 245 µL, 1.61 mmol) was added thereto, and the mixture was stirred at room temperature for 2 hours. After the reaction was completed, acetonitrile (80 mL) was added thereto and stirred, and then the precipitate was filtered and dried under reduced pressure to obtain a compound (1-N-1) (810 mg, yield: 98.7%).

¹H-NMR (CDCl₃, 400 MHz): δ = 0.88 (6H, t), 1.19 to 1.80 (80H, m), 3.78 (3H, s), 3.94 (4H, t), 5.08 (2H, s), 5.30 (1H, s), 6.40 (1H, t), 6.60 (2H, d), 6.85 (2H, d), 7.16 (1H, d), 7.22 (1H, dd), 7.31 (2H, d), 7.36 (1H, dd), 7.64 (1H, d), 7.73 (1H, d), 7.80 (1H, s)

The following compound (1-N-2) could be synthesized in the same manner as in the above-described compound (1-N-1).

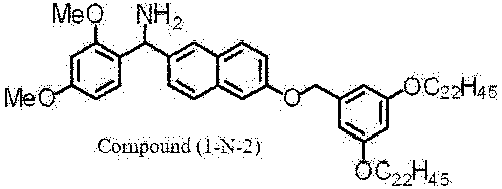

<Synthesis of protective group-forming reagents (compounds (2-1) and (2-2))>

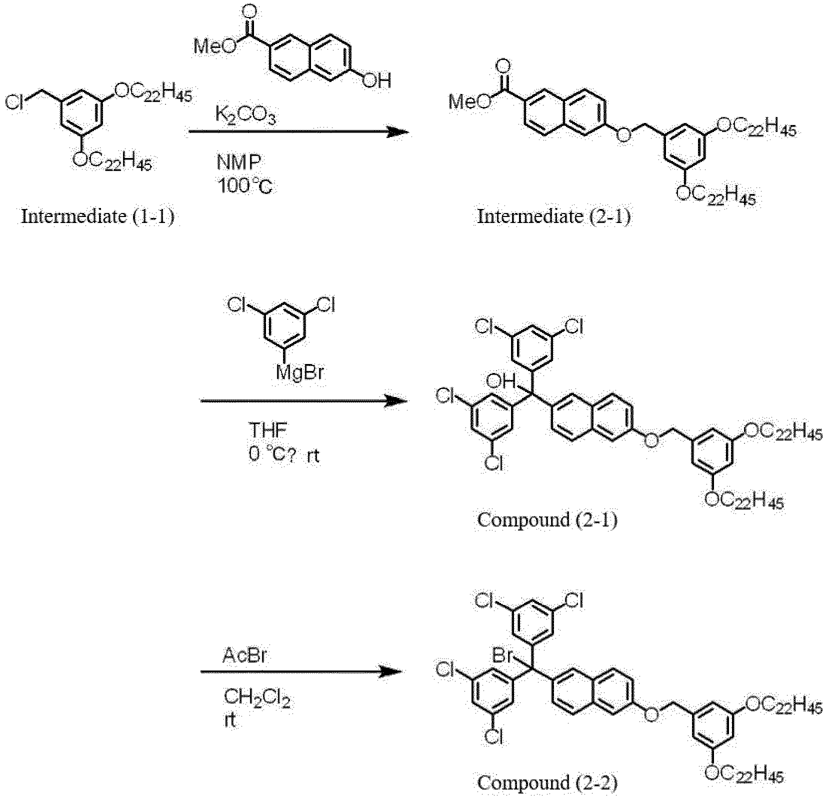

The intermediate (1-1) (12.0 g, 15.5 mmol), methyl 6-hydroxy-2-naphthoate (6.26 g, 30.9 mmol), potassium carbonate (8.55 g, 61.9 mmol), and N-methylpyrrolidone (NMP, 155 mL) were mixed, and the mixture was stirred at 100°C for 4 hours under a nitrogen atmosphere. The reaction solution was cooled to room temperature and extracted with cyclopentyl methyl ether and water. Methanol was added to the obtained organic layer to precipitate solid, and the solid was filtered and dried under reduced pressure to obtain an intermediate (1-2) (13.8 g, yield: 94.6%).

Under a nitrogen atmosphere, the intermediate (1-2) (2.50 g, 2.66 mmol) was mixed with tetrahydrofuran (5 mL), the mixture was cooled to 0°C, and a 3,5-dichlorophenylmagnesium bromide tetrahydrofuran solution(0.5 mol/L) (21.2 mL, 10.6 mmol) was added dropwise thereto. The reaction solution was heated to room temperature and stirred for 2 hours. After the mixture was further cooled to 0°C, water (0.5 mL) was gently added dropwise thereto, and then the mixture was extracted with cyclopentyl methyl ether and a saturated ammonium chloride aqueous solution. The obtained organic layer was dried over sodium sulfate and filtered, and the filtrate was concentrated under reduced pressure. The obtained crude product was purified by column chromatography (hexane:ethyl acetate = 19:1 to 9:1) to obtain a compound (2-1) (2.78 g, yield: 87.0%).

¹H-NMR (CDCl₃, 400 MHz): δ = 0.88 (6H, t), 1.21 to 1.80 (80H, m), 2.86 (1H, s), 3.94 (4H, t), 5.11 (2H, s), 6.42 (1H, t), 6.61 (2H, s), 7.20 (1H, d), 7.23 (4H, d), 7.25 to 7.28 (2H, m), 7.33 (2H, t), 7.47 (1H, d), 7.69 (1H, d), 7.73 (1H, d)

The compound (2-1) (1.50 g, 1.25 mmol) was mixed with dichloromethane (12.5 mL), bromide acetate (279 µL, 3.74 mmol) was added dropwise thereto, and the mixture was stirred at 30°C for 3 hours. After the reaction solution was concentrated under reduced pressure, acetonitrile was added to the residue, and the resulting precipitate was filtered and dried under reduced pressure to obtain a compound (2-2) (1.58 g, yield: 100%).

¹H-NMR (CDCl₃, 400 MHz): δ = 0.88 (6H, t), 1.21 to 1.80 (80H, m), 3.94 (4H, t), 5.12 (2H, s), 6.42 (1H, t), 6.61 (2H, d), 7.18 (1H, d), 7.20 to 7.28 (6H, m), 7.36 (2H, t), 7.54 (1H, dd), 7.63 (1H, d), 7.73 (1H, d)

<Synthesis of comparative protective group-forming reagent (comparative compound (1-1))>

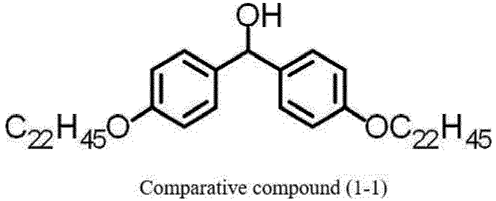

A comparative compound (1-1) was synthesized by the method described in WO2010/113939A.

<Synthesis of comparative protective group-forming reagent (comparative compound (1-N-1))>

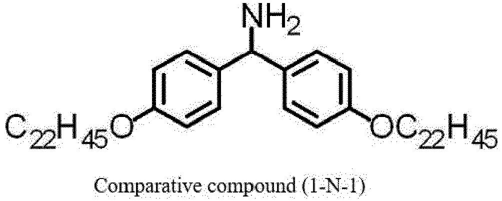

A comparative compound (1-N-1) was also synthesized by the method described in WO2010/113939A.

<Synthesis of comparative protective group-forming reagent (comparative compound (2-1))>

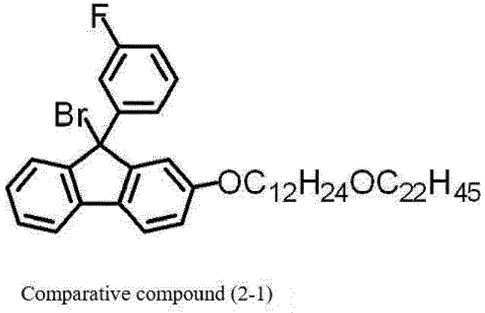

A comparative compound (2-1) was synthesized by the method described in WO2010/104169A.

### (Example 1)

### <Synthesis of protected amino acid compound (N-terminal and C-terminal protected amino acid (1-1))>

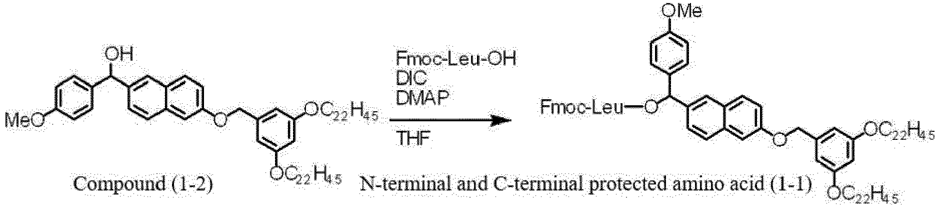

The compound (1-2) (306 mg, 0.30 mmol), N-[(9H-fluoren-9-ylmethoxy)carbonyl]-L-leucine (Fmoc-Leu-OH, 159 mg, 0.45 mmol), and tetrahydrofuran (1.5 mL) were mixed at room temperature, and 4-dimethylaminopyridine (DMAP, 7.0 mg, 0.06 mmol) and diisopropylcarbodiimide (69.7 µL, 0.45 mmol) were added thereto. After stirring the reaction solution for 1 hour, methanol (15 mL) was added thereto to precipitate solid, and the solid was filtered and dried under reduced pressure to obtain an N-protected and C-protected amino acid (1-1) (380 mg, 95.5%).

Fmoc represents a 9-fluorenylmethoxycarbonyl group, and Leu represents a leucine residue.

### (Example 2 and Comparative Example 1)

Same as the method for obtaining the N-protected and C-protected amino acid (1-1), the corresponding N-protected and C-protected amino acid was synthesized by condensing the compound (1-1) or comparative compound (1-1) with N-[(9H-fluoren-9-ylmethoxy)carbonyl]-L-leucine.

### [Evaluation 1]

### <Solution stability>

The obtained N-protected and C-protected amino acid (10 mg) and 2,6-di-tert-butyl-4-methylphenol (internal standard: 8 mg) were dissolved in a tetrahydrofuran solution (20 mL) and acetic acid (2 mL), respectively, and after allowing time to pass at room temperature for three days, the amount of remaining N-protected and C-protected amino acid was determined and evaluated based on the following standard. The results are shown in Table 1.

Regarding the evaluation of the solution stability, a case of "B" or higher was regarded as acceptable. The results are shown in Table 1.

It can be said that, as the solution stability is higher, peptide stability is high, side reactions can be suppressed, and yield is excellent.

-Evaluation standard-
"A": survival rate was 99% or more.
"B": survival rate was 97% or more and less than 99%.
"C": survival rate was 95% or more and less than 97%.
"D": survival rate was less than 95%.

**[Table 1]**

| | Compound | Solution stability |
|---|---|---|
| Example 1 | Compound (1-2) | A |
| Example 2 | Compound (1-1) | A |
| Comparative example 1 | Comparative compound (1-1) | C |

From Table 1, since the condensed polycyclic aromatic hydrocarbon compound represented by Formula (1) used in Example 1 or Example 2 was superior in solution stability as compared with the compound shown in Comparative Example 1, the yield of the peptide compound was excellent.

### [Evaluation 2]

### <Deprotection rate>

With regard to the N-protected and C-protected amino acid (1-1) synthesized above, the protected carboxylic acid moiety was deprotected by the following procedure.

100 mg of Fmoc-Leu-OTag (N-terminal and C-terminal protected amino acid using the N-terminal and C-terminal protected amino acid (1-1)) and Fmoc-Gly-OH (internal standard) in an equimolar amount of Fmoc-Leu-OTag were mixed, and then dichloromethane/trifluoroethanol/trifluoroacetic acid (100/10/1: vol%) was added thereto so that the substrate concentration was 0.026 M based on Fmoc-Leu-OTag, and the mixture was stirred at 30°C.

5 µL of the reaction solution was dissolved in 400 µL of methanol (MeOH), and using Ultra Performance LC (ultra-performance liquid chromatography, manufactured by Waters Corporation, model number: ACQUITY), the deprotection ratio (%) was determined by quantifying the ratio of Fmoc-Leu-OH and Fmoc-Gly-OH produced by deprotecting Fmoc-Leu-OTag, and the time required for deprotection to be completed was obtained.

With the N-protected and C-protected amino acid (1-1), it was found that the deprotection was completed within 10 minutes.

The columns and measurement conditions used for the ultra-performance liquid chromatography are shown below.
Column: manufactured by Waters Corporation, model number: BEH C18 1.7 µm, 2.1 mm x 30 mm
Flow rate: 0.5 mL/min
Solvent: solution A: 0.1% formic acid-water, solution B: 0.1% formic acid-acetonitrile
Gradient cycle: 0.00 min (solution A/solution B = 95/5), 2.00 min (solution A/solution B = 5/95), 3.00 min (solution A/solution B = 95/5)
Detection wavelength: 254 nm

From the above, it was found that the condensed polycyclic aromatic hydrocarbon compound represented by Formula (1) used in Example 1 had a high deprotection rate and had no problem in practical use. Combined with the result of solution stability, both solution stability and deprotection rate (deprotection property) could be achieved.

### (Example 3)

### <Synthesis of protected amino acid compound (N-terminal and C-terminal protected amino acid (1-1))>

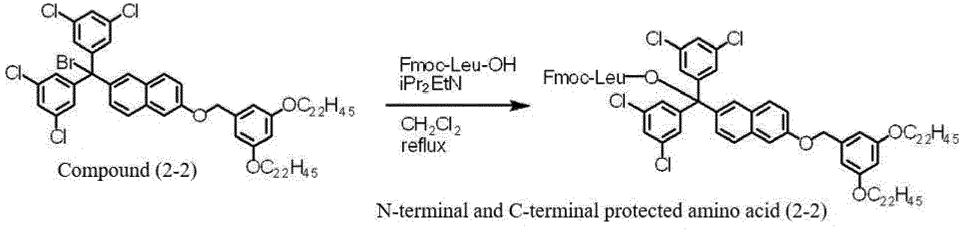

The compound (2-2) (500 mg, 0.39 mmol), N-[(9H-fluoren-9-ylmethoxy)carbonyl]-L-leucine (419 mg, 1.18 mmol), and dichloromethane (4 mL) were mixed at room temperature, and diisopropylethylamine (iPr₂EtN, 207 µL, 1.18 mmol) was added thereto. After heating to reflux the reaction solution and stirring for 20 hours, acetonitrile (20 mL) was added thereto to precipitate solid, and the solid was filtered and dried under reduced pressure to obtain an N-protected and C-protected amino acid (2-2) (550 mg, 82.3%).

### (Comparative Example 2)

Same as the method for obtaining the N-protected and C-protected amino acid (2-2), the comparative compound (2-1) was condensed with N-[(9H-fluoren-9-ylmethoxy)carbonyl]-L-leucine, and as a result, the recovery rate was 62%. In addition, the content of the target product in the recovery rate of 62% was approximately 80% by mole in terms of NMR, and approximately 20% by mole of the raw material remained.

From the above, since the condensed polycyclic aromatic hydrocarbon compound represented by Formula (1) used in Example 3 could load amino acid in a high yield, the yield of the peptide compound was excellent.

### (Example 4)

### <Synthesis of protected amino acid compound (N-terminal and C-terminal protected amino acid (1-N-1))>

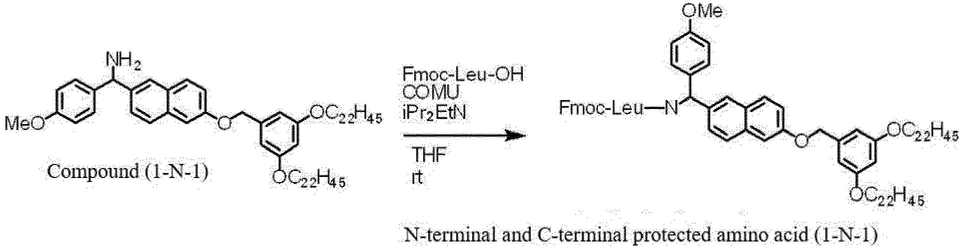

The compound (1-N-1) (500 mg, 0.49 mmol), N-[(9H-fluoren-9-ylmethoxy)carbonyl]-L-leucine (260 mg, 0.49 mmol), and tetrahydrofuran (2.5 mL) were mixed at room temperature, and diisopropylethylamine (337 µL, 0.74 mmol) and (1-cyano-2-ethoxy-2-oxoethylidenaminooxy)dimethylamino-morpholino-carbenium hexafluorophosphate (COMU) (315 mg, 0.74 mmol) were added thereto. After stirring the reaction solution for 1 hour, acetonitrile (25 mL) was added thereto to precipitate solid, and the solid was filtered and dried under reduced pressure to obtain an N-protected and C-protected amino acid (1-N-1) (637 mg, 95.9%).

### (Comparative Example 3)

Same as the method for obtaining the N-protected and C-protected amino acid (1-N-1), the comparative compound (1-N-1) was condensed with N-[(9H-fluoren-9-ylmethoxy)carbonyl]-L-leucine, and as a result, the yield was 85%.

From the above, since the condensed polycyclic aromatic hydrocarbon compound represented by Formula (1) used in Example 4 could load amino acid in a high yield, the yield of the peptide compound was excellent.

### <Synthesis of protected peptide (5-residue peptide: Fmoc-MeNle-MeNle-Arg(Pbf)-Cys(Trt)-Gly-NH-protective group)>

Details of each abbreviation other than the above are shown below.
MeNle: N-methylnorleucine residue
Arg(Pbf): Pbf-protected arginine residue
Pbf: 2,2,4,6,7-pentamethyldihydrobenzofuran-5-sulfonyl group
Cys(Trt): Trt-protected cysteine residue
Trt: triphenylmethyl group
Gly: glycine residue

### (Example 5: synthesis of Fmoc-Gly-O-2NaphTAG (1))

The compound (1-2) (also referred to as "2NaphTAG (1)") (2.00 g, 1.96 mmol) and Fmoc-Gly-OH (1.5 molar equivalent), tetrahydrofuran (9.8 mL), 4-dimethylaminopyridine (0.2 molar equivalent), and diisopropylcarbodiimide (1.5 molar equivalent) were mixed and stirred. After the condensation reaction was completed, acetonitrile (98 mL) was added thereto and stirred, and then the precipitate was filtered, washed with acetonitrile, and dried under reduced pressure to obtain Fmoc-Gly-O-2NaphTAG (1) (2.54 g, yield: 99.7%).

### (Example 6: synthesis of Fmoc-Cys(Trt)-Gly-O-2NaphTAG (1))

Fmoc-Gly-O-2NaphTAG (1) (2.30 g, 1.77 mmol) was dissolved in tetrahydrofuran (8.9 mL), and diazabicycloundecene (DBU, 2.0 molar equivalent) was added thereto and stirred. After the deprotection reaction was completed, N-methylmorpholine (2.05 molar equivalent) and methanesulfonic acid (2.0 molar equivalent) were added thereto in sequence, and Fmoc-Cys(Trt)-OH (1.25 molar equivalent) and (1-cyano-2-ethoxy-2-oxoethylidenaminooxy)dimethylamino-morpholino-carbenium hexafluorophosphate (COMU, 1.25 molar equivalent) were added thereto and stirred. After the condensation reaction was completed, acetonitrile (89 mL) was added thereto and stirred, and then the precipitate was filtered, washed with acetonitrile, and dried under reduced pressure to obtain Fmoc-Cys(Trt)-Gly-O-2NaphTAG (1) (2.87 g, yield: 98.6%).

### (Example 7: synthesis of Fmoc-Arg(Pbf)-Cys(Trt)-Gly-O-2NaphTAG (1))

Fmoc-Cys(Trt)-Gly-NH-2NaphTAG (1) (2.00 g, 1.22 mmol) was dissolved in tetrahydrofuran (6.1 mL), and diazabicycloundecene (DBU, 2.0 molar equivalent) was added thereto and stirred. After the deprotection reaction was completed, N-methylmorpholine (2.05 molar equivalent) and methanesulfonic acid (2.0 molar equivalent) were added thereto in sequence, and Fmoc-Arg(Pbf)-OH (1.25 molar equivalent) and (1-cyano-2-ethoxy-2-oxoethylidenaminooxy)dimethylamino-morpholino-carbenium hexafluorophosphate (COMU, 1.25 molar equivalent) were added thereto and stirred. After the condensation reaction was completed, acetonitrile (61 mL) was added thereto and stirred, and then the precipitate was filtered, washed with acetonitrile, and dried under reduced pressure to obtain Fmoc-Arg(Pbf)-Cys(Trt)-Gly-O-2NaphTAG (1) (2.22 g, yield: 88.9%).

### (Example 8: synthesis of Fmoc-MeNle-Arg(Pbf)-Cys(Trt)-Gly-NH-O-2NaphTAG (1))

Fmoc-Arg(Pbf)-Cys(Trt)-Gly-O-2NaphTAG (1) (1.60 g, 0.78 mmol) was dissolved in tetrahydrofuran (3.9 mL), and diazabicycloundecene (DBU, 2.0 molar equivalent) was added thereto and stirred. After the deprotection reaction was completed, N-methylmorpholine (2.05 molar equivalent) and methanesulfonic acid (2.0 molar equivalent) were added thereto in sequence, and Fmoc-MeNle-OH (1.25 molar equivalent) and (1-cyano-2-ethoxy-2-oxoethylidenaminooxy)dimethylamino-morpholino-carbenium hexafluorophosphate (COMU, 1.25 molar equivalent) were added thereto and stirred. After the condensation reaction was completed, acetonitrile (39 mL) was added thereto and stirred, and then the precipitate was filtered, washed with acetonitrile, and dried under reduced pressure to obtain Fmoc-MeNle-Arg(Pbf)-Cys(Trt)-Gly-O-2NaphTAG (1) (1.64 g, yield: 96.5%).

### (Example 9: synthesis of Fmoc-MeNle-MeNle-Arg(Pbf)-Cys(Trt)-Gly-O-2NaphTAG (1))

Fmoc-MeNle-Arg(Pbf)-Cys(Trt)-Gly-O-2NaphTAG (1) (1.50 g, 0.69 mmol) was dissolved in tetrahydrofuran (3.4 mL), and diazabicycloundecene (DBU, 2.0 molar equivalent) was added thereto and stirred. After the deprotection reaction was completed, N,N-diisopropylethylamine (6.05 molar equivalent) and methanesulfonic acid (2.0 molar equivalent) were added thereto in sequence, and Fmoc-MeNle-OH (2.0 molar equivalent) and (7-azabenzotriazole-1-yloxy)tripyrolidinophosphonium hexafluorophosphate (PyAOP, 2.0 molar equivalent) were added thereto and stirred. After the condensation reaction was completed, acetonitrile (34 mL) was added thereto and stirred, and then the precipitate was filtered, washed with acetonitrile, and dried under reduced pressure to obtain Fmoc-MeNle-MeNle-Arg(Pbf)-Cys(Trt)-Gly-O-2NaphTAG (1) (1.58 g, yield: 99.4%).

As shown in Examples 5 to 9, the condensed polycyclic aromatic hydrocarbon compound represented by Formula (1) is excellent in the yield of the obtained peptide compound.

In addition, as shown in Examples 5 to 9, it can be seen that the condensed polycyclic aromatic hydrocarbon compound represented by Formula (1) has a high yield in all cases, and the total yield is also excellent. The method for producing a peptide compound according to the embodiment of the present disclosure can also be applied to a production of a cyclic peptide compound having an N-alkylamide structure. The C-terminal protective group can be deprotected even under weak acid conditions, a side reaction of the obtained peptide can be suppressed, the purity is high, and the yield is high.

The disclosure of Japanese Patent Application No. 2019-122431 filed on June 28, 2019 is incorporated in the present specification by reference.

All documents, patent applications, and technical standards described in the present specification are incorporated herein by reference to the same extent as in a case of being specifically and individually noted that individual documents, patent applications, and technical standards are incorporated by reference.

## Claims

1. A method for producing a peptide compound, comprising:
a step of using a condensed polycyclic aromatic hydrocarbon compound represented by Formula (1), 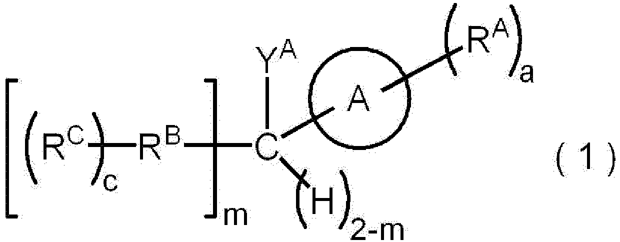
in Formula (1),
a ring A represents a condensed polycyclic aromatic hydrocarbon ring,
Y^{A} represents -OH, -NHR, SH, or -X⁰, where R represents a hydrogen atom, an alkyl group, an aromatic group-substituted alkyl group, a heteroaromatic group-substituted alkyl group, or a 9-fluorenylmethoxycarbonyl group, and X⁰ represents Cl, Br, or I,
R^{A}'s each independently represent an aliphatic hydrocarbon group or an organic group having an aliphatic hydrocarbon group,
R^{B}'s each independently represent a monovalent aliphatic hydrocarbon group, a (1+c)-valent aromatic group, or a (1+c)-valent heteroaromatic group,
R^{C}'s each independently represent an aliphatic hydrocarbon group or an organic group having an aliphatic hydrocarbon group,
m represents 1 or 2, a represents an integer of 0 to 5, and c represents an integer of 0 to 5,
in a case where both a and c are 0, R^{B} is a monovalent aliphatic hydrocarbon group, and
the number of carbon atoms in at least one aliphatic hydrocarbon group is 12 or more.

2. The method for producing a peptide compound according to claim 1,
wherein the step of using the condensed polycyclic aromatic hydrocarbon compound represented by Formula (1) is a C-terminal protecting step of protecting a carboxy group or an amide group of an amino acid compound or a peptide compound with the condensed polycyclic aromatic hydrocarbon compound represented by Formula (1).

3. The method for producing a peptide compound according to claim 2,
wherein the amino acid compound or the peptide compound in the C-terminal protecting step is an N-terminal protected amino acid compound or an N-terminal protected peptide compound, and
the method for producing a peptide compound further comprising:
an N-terminal deprotecting step of deprotecting an N-terminal end of an N-terminal and C-terminal protected amino acid compound or an N-terminal and C-terminal protected peptide compound, which is obtained in the C-terminal protecting step,
a peptide chain extending step of condensing the N-terminal end of a C-terminal protected amino acid compound or a C-terminal protected peptide compound, which is obtained in the N-terminal deprotecting step, with an N-terminal protected amino acid compound or an N-terminal protected peptide compound; and
a precipitating step of precipitating the N-terminal and C-terminal protected peptide compound obtained in the peptide chain extending step.

4. The method for producing a peptide compound according to claim 3, further comprising, one or more times in the following order after the precipitating step:
a step of deprotecting the N-terminal end of the obtained N-terminal and C-terminal protected peptide compound;
a step of condensing the N-terminal end of the obtained C-terminal protected peptide compound with an N-terminal protected amino acid compound or an N-terminal protected peptide compound; and
a step of precipitating the obtained N-terminal and C-terminal protected peptide compound.

5. The method for producing a peptide compound according to any one of claims 1 to 4, further comprising:
a C-terminal deprotecting step of deprotecting a C-terminal protective group.

6. The method for producing a peptide compound according to any one of claims 1 to 5,
wherein the ring A is a naphthalene ring.

7. The method for producing a peptide compound according to any one of claims 1 to 6,
wherein a total number of carbon atoms in the aliphatic hydrocarbon group is 36 to 80.

8. The method for producing a peptide compound according to any one of claims 1 to 7,
wherein the condensed polycyclic aromatic hydrocarbon compound represented by Formula (1) is a compound represented by any of Formula (10) or Formula (20), 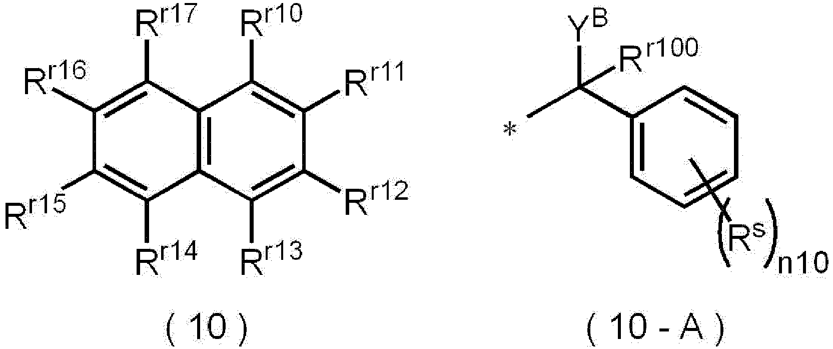
in Formula (10) and Formula (10-A),
Y^{B} represents -OH, -NHR, -SH, or -X⁰, where R represents a hydrogen atom, an alkyl group, an aromatic group-substituted alkyl group, a heteroaromatic group-substituted alkyl group, or a 9-fluorenylmethoxycarbonyl group, and X⁰ represents Cl, Br, or I,
R^{r100} represents a hydrogen atom, an aryl group, or a heteroaryl group,
R^{s}'s each independently represent a substituent or R^{A},
n10 represents an integer of 0 to 5,
adjacent R^{s}'s may be linked to each other through a substituent to form a ring,
R^{r10} and R^{r11} each independently represent a hydrogen atom, a substituent, a group represented by Formula (10-A), or R^{A},
any one of R^{r10} or R^{r11} is a group represented by Formula (10-A),
^{∗} represents a linking position with R^{r10} or R^{r11},
R^{r12} to R^{r17} each independently represent a hydrogen atom, a substituent, or R^{A},
at least one of R^{s}, R^{r10}, ... , or R^{r17} is R^{A},
R^{A}'s each independently represent an aliphatic hydrocarbon group or an organic group having an aliphatic hydrocarbon group, and
the number of carbon atoms in at least one aliphatic hydrocarbon group is 12 or more, 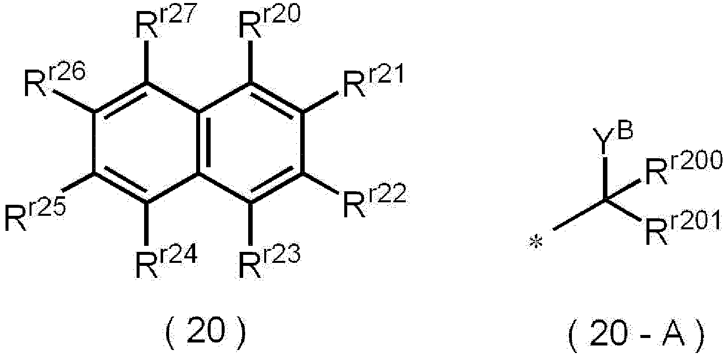
in Formula (20) and Formula (20-A),
Y^{B} represents -OH, -NHR, -SH, or -X⁰, where R represents a hydrogen atom, an alkyl group, an aromatic group-substituted alkyl group, a heteroaromatic group-substituted alkyl group, or a 9-fluorenylmethoxycarbonyl group, and X⁰ represents Cl, Br, or I,
R^{r200} represents a hydrogen atom, an alkyl group, an aryl group, or a heteroaryl group,
R^{r201} represents an alkyl group,
R^{r20} and R^{r21} each independently represent a hydrogen atom, a substituent, a group represented by Formula (20-A), or R^{A},
any one of R^{r20} or R^{r21} is a group represented by Formula (20-A),
^{∗} represents a linking position with R^{r20} or R^{r21},
R^{r22} to R^{r27} each independently represent a hydrogen atom, a substituent, or R^{A},
the number of carbon atoms in R^{r201} is 12 or more, or at least one of R^{r20}, ... , or R^{r27} is R^{A},
R^{A}'s each independently represent an aliphatic hydrocarbon group or an organic group having an aliphatic hydrocarbon group, and
the number of carbon atoms in at least one aliphatic hydrocarbon group is 12 or more.

9. The method for producing a peptide compound according to claim 8,
wherein R^{A}'s in Formula (10) or Formula (20) are each independently a group represented by Formula (f1) or Formula (a1), 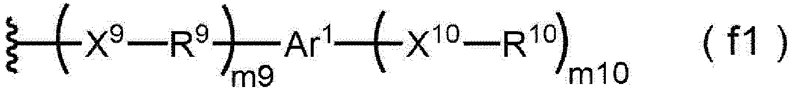
in Formula (f1), a wavy line portion represents a bonding position to other structures, m9 represents an integer of 1 to 3, X⁹'s each independently represent a single bond, -O-, -S-, -COO-, -OCO-, -OCONH-, -NHCONH-, -NHCO-, or -CONH-, R⁹'s each independently represent a divalent aliphatic hydrocarbon group, Ar¹ represents an (m10+1)-valent aromatic group or an (m10+1)-valent heteroaromatic group, m10 represents an integer of 1 to 3, X¹⁰'s each independently represent a single bond, -O-, -S-, -COO-, -OCO-, -OCONH-, -NHCONH-, -NHCO-, or -CONH-, R¹⁰'s each independently represent a monovalent aliphatic hydrocarbon group, and at least one of R¹⁰'s is a monovalent aliphatic hydrocarbon group having 5 or more carbon atoms, 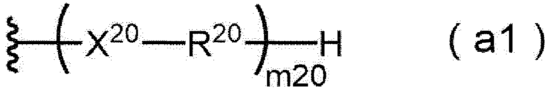
in Formula (a1), a wavy line portion represents a bonding position to other structures, m20 represents an integer of 1 to 10, X²⁰'s each independently represent a single bond, -O-, -S-, -COO-, -OCO-, -OCONH-, -NHCONH-, -NHCO-, or -CONH-, R²⁰'s each independently represent a divalent aliphatic hydrocarbon group, and at least one of R²⁰'s is a divalent aliphatic hydrocarbon group having 5 or more carbon atoms.

10. The method for producing a peptide compound according to claim 9, (f2),
wherein the group represented by Formula (f1) is a group represented by Formula 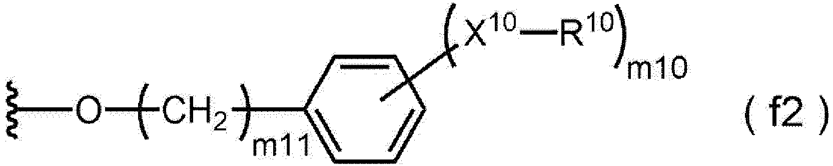
in Formula (f2), a wavy line portion represents a bonding position to other structures, m10 represents an integer of 1 to 3, m11 represents an integer of 1 to 3, X¹⁰'s each independently represent a single bond, -O-, -S-, -COO-, -OCO-, -OCONH-, -NHCONH-, -NHCO-, or -CONH-, and R¹⁰'s each independently represent a monovalent aliphatic hydrocarbon group having 5 or more carbon atoms.

11. A protective group-forming reagent comprising:
a condensed polycyclic aromatic hydrocarbon compound represented by Formula (1), 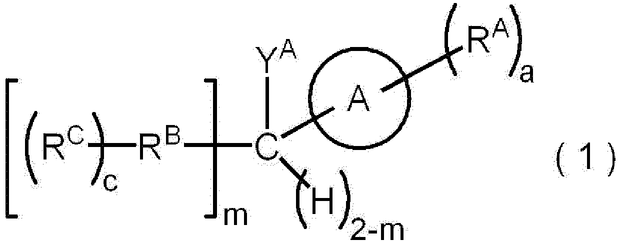
in Formula (1),
a ring A represents a condensed polycyclic aromatic hydrocarbon ring,
Y^{A} represents -OH, -NHR, SH, or -X⁰, where R represents a hydrogen atom, an alkyl group, an aromatic group-substituted alkyl group, a heteroaromatic group-substituted alkyl group, or a 9-fluorenylmethoxycarbonyl group, and X⁰ represents Cl, Br, or I,
R^{A}'s each independently represent an aliphatic hydrocarbon group or an organic group having an aliphatic hydrocarbon group,
R^{B}'s each independently represent a monovalent aliphatic hydrocarbon group, a (1+c)-valent aromatic group, or a (1+c)-valent heteroaromatic group,
R^{C}'s each independently represent an aliphatic hydrocarbon group or an organic group having an aliphatic hydrocarbon group,
m represents 1 or 2, a represents an integer of 0 to 5, and c represents an integer of 0 to 5,
in a case where both a and c are 0, R^{B} is a monovalent aliphatic hydrocarbon group, and
the number of carbon atoms in at least one aliphatic hydrocarbon group is 12 or more.

12. The protective group-forming reagent according to claim 11,
wherein the protective group-forming reagent is a protective group-forming reagent of a carboxy group or an amide group.

13. The protective group-forming reagent according to claim 11 or 12,
wherein the protective group-forming reagent is a C-terminal protective group-forming reagent of an amino acid compound or a peptide compound.

14. A condensed polycyclic aromatic hydrocarbon compound represented by Formula (1a), 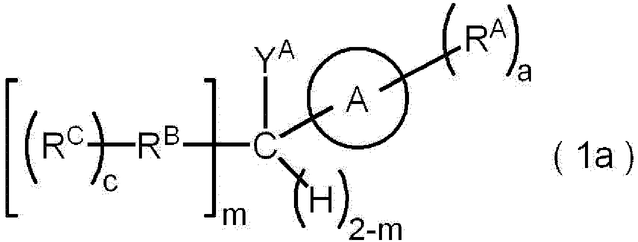
in Formula (1a),
a ring A represents a condensed polycyclic aromatic hydrocarbon ring,
Y^{A} represents -OH, -NHR, -SH, or -X⁰, where R represents a hydrogen atom, an alkyl group having 10 or less carbon atoms, an aromatic group-substituted alkyl group, a heteroaromatic group-substituted alkyl group, or a 9-fluorenylmethoxycarbonyl group, and X⁰ represents Cl, Br, or I,
R^{A}'s each independently represent an aliphatic hydrocarbon group or an organic group having an aliphatic hydrocarbon group,
R^{B}'s each independently represent a monovalent aliphatic hydrocarbon group, a (1+c)-valent aromatic group, or a (1+c)-valent heteroaromatic group,
R^{C}'s each independently represent an aliphatic hydrocarbon group or an organic group having an aliphatic hydrocarbon group,
m represents 1 or 2, a represents an integer of 0 to 5, and c represents an integer of 0 to 5,
in a case where both a and c are 0, R^{B} is a monovalent aliphatic hydrocarbon group,
the number of carbon atoms in at least one aliphatic hydrocarbon group is 18 or more, and
in a case where R^{B} is a monovalent aliphatic hydrocarbon group, R^{B} includes a linear saturated aliphatic hydrocarbon group having 18 or more carbon atoms.

15. The condensed polycyclic aromatic hydrocarbon compound according to claim 14,
wherein the ring A is a naphthalene ring.

16. The condensed polycyclic aromatic hydrocarbon compound according to claim 14 or 15,
wherein the condensed polycyclic aromatic hydrocarbon compound represented by Formula (1a) is a compound represented by any of Formula (10a) or Formula (20a), 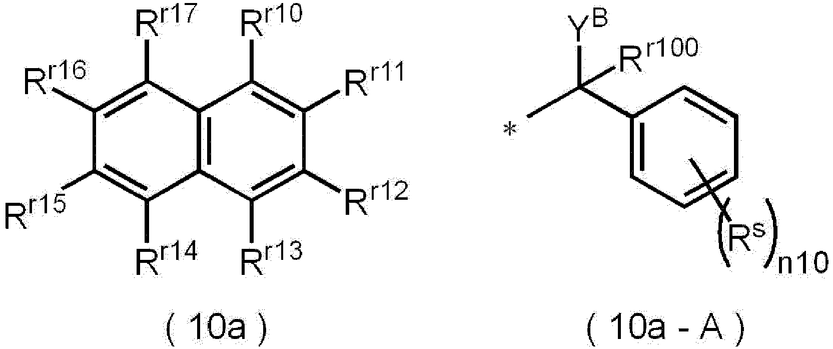
in Formula (10a) and Formula (10a-A),
Y^{B} represents -OH, -NHR, -SH, or -X⁰, where R represents a hydrogen atom, an alkyl group having 10 or less carbon atoms, an aromatic group-substituted alkyl group, a heteroaromatic group-substituted alkyl group, or a 9-fluorenylmethoxycarbonyl group, and X⁰ represents Cl, Br, or I,
R^{r100} represents a hydrogen atom, an aryl group, or a heteroaryl group,
R^{s}'s each independently represent a substituent or R^{A},
n10 represents an integer of 0 to 5,
adjacent R^{s}'s may be linked to each other through a substituent to form a ring,
R^{r10} and R^{r11} each independently represent a hydrogen atom, a substituent, a group represented by Formula (10a-A), or R^{A},
any one of R^{r10} or R^{r11} is a group represented by Formula (10a-A),
^{∗} represents a linking position with R^{r10} or R^{r11},
R^{r12} to R^{r17} each independently represent a hydrogen atom, a substituent, or R^{A},
at least one of R^{s}, R^{r10}, ... , or R^{r17} is R^{A},
R^{A}'s each independently represent an aliphatic hydrocarbon group or an organic group having an aliphatic hydrocarbon group, and
the number of carbon atoms in at least one aliphatic hydrocarbon group is 18 or more, 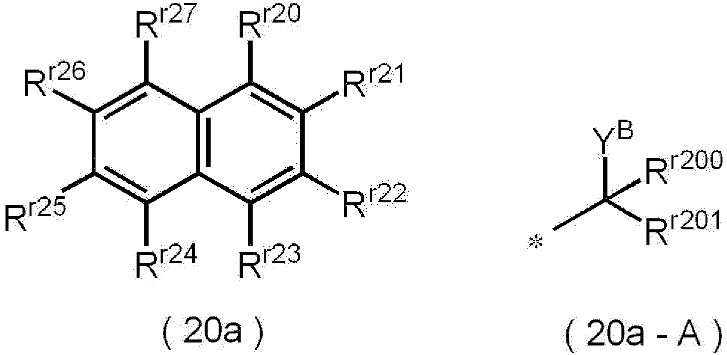
in Formula (20a) and Formula (20a-A),
Y^{B} represents -OH, -NHR, -SH, or -X⁰, where R represents a hydrogen atom, an alkyl group having 10 or less carbon atoms, an aromatic group-substituted alkyl group, a heteroaromatic group-substituted alkyl group, or a 9-fluorenylmethoxycarbonyl group, and X⁰ represents Cl, Br, or I,
R^{r200} represents a hydrogen atom, an alkyl group, an aryl group, or a heteroaryl group,
R^{r201} represents an aliphatic hydrocarbon group,
R^{r20} and R^{r21} each independently represent a hydrogen atom, a substituent, a group represented by Formula (20a-A), or R^{A},
any one of R^{r20} or R^{r21} is a group represented by Formula (20a-A),
^{∗} represents a linking position with R^{r20} or R^{r21},
R^{r22} to R^{r27} each independently represent a hydrogen atom, a substituent, or R^{A}, and
in a case where R^{B} is an aliphatic hydrocarbon group, R^{B} includes a linear saturated aliphatic hydrocarbon group having 18 or more carbon atoms, or at least one of R^{r20}, ... , or R^{r27} is R^{A}, where R^{A}'s each independently represent an aliphatic hydrocarbon group or an organic group having an aliphatic hydrocarbon group, and the number of carbon atoms in at least one aliphatic hydrocarbon group is 18 or more.

17. The condensed polycyclic aromatic hydrocarbon compound according to claim 16,
wherein R^{A}'s in Formula (10a) or Formula (20a) are each independently a group represented by Formula (f1) or Formula (a1), 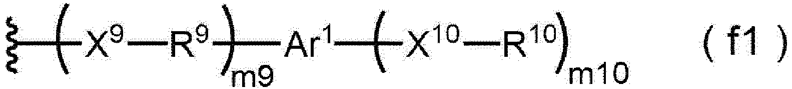
in Formula (f1), a wavy line portion represents a bonding position to other structures, m9 represents an integer of 1 to 3, X⁹'s each independently represent a single bond, -O-, -S-, -COO-, -OCO-, -OCONH-, -NHCONH-, -NHCO-, or -CONH-, R⁹'s each independently represent a divalent aliphatic hydrocarbon group, Ar¹ represents an (m10+1)-valent aromatic group or an (m10+1)-valent heteroaromatic group, m10 represents an integer of 1 to 3, X¹⁰'s each independently represent a single bond, -O-, -S-, -COO-, -OCO-, -OCONH-, -NHCONH-, -NHCO-, or -CONH-, R¹⁰'s each independently represent a monovalent aliphatic hydrocarbon group, and at least one of R¹⁰'s is a monovalent aliphatic hydrocarbon group having 5 or more carbon atoms, 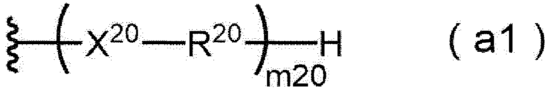
in Formula (a1), a wavy line portion represents a bonding position to other structures, m20 represents an integer of 1 to 10, X²⁰'s each independently represent a single bond, -O-, -S-, -COO-, -OCO-, -OCONH-, -NHCONH-, -NHCO-, or -CONH-, R²⁰'s each independently represent a divalent aliphatic hydrocarbon group, and at least one of R²⁰'s is a divalent aliphatic hydrocarbon group having 5 or more carbon atoms.

18. The condensed polycyclic aromatic hydrocarbon compound according to claim 17, (f2),
wherein the group represented by Formula (f1) is a group represented by Formula 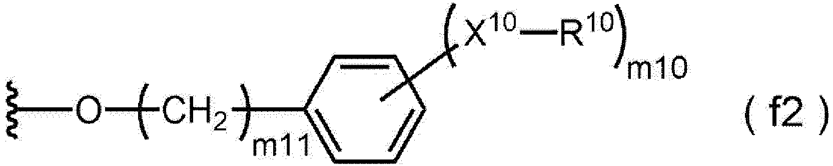
in Formula (f2), a wavy line portion represents a bonding position to other structures, m10 represents an integer of 1 to 3, m11 represents an integer of 1 to 3, X¹⁰'s each independently represent a single bond, -O-, -S-, -COO-, -OCO-, -OCONH-, -NHCONH-, -NHCO-, or -CONH-, and R¹⁰'s each independently represent a monovalent aliphatic hydrocarbon group having 5 or more carbon atoms.
